# EUROPEAN PATENT APPLICATION

(11) **EP 4 710 934 A1**
(43) Date of publication of application: **18.03.2026**
(21) Application number: 24200318.4
(22) Date of filing: 13.09.2024
(51) Int. Cl.: A61K 38/19, A61P 25/28

(54) **TARGETING OF MICROGLIA IN NEURODEGENERATIVE DISEASES**

(71) Applicant: Albert-Ludwigs-Universität Freiburg, 79098 Freiburg (DE)
(72) Inventor: Prinz, Marco, 79249 Merzhausen (DE); Kierdorf, Katrin, 79106 Freiburg (DE); Ardura Fabregat, Alberto, Massachussetts, 02138 (US); Bosch, Lance Fredrick Pahutan, 70180 Stuttgart (DE)
(74) Representative: Hertin und Partner Rechts- und Patentanwälte PartG mbB

(57) **Abstract**

Microglial spatial heterogeneity remains a crucial yet poorly studied question in light of potential cell-directed therapies for Alzheimer's disease (AD). Little is known about the dynamics of spatially distinct microglia states, which are either adjacent or non-associated with the plaque site, and their selective contributions to neurodegeneration in vivo. So far, research has essentially focused on pathology-associated microglia. Here, we combined novel multicolor fluorescence fate mapping, single-cell transcriptional analysis, epigenetic profiling, advanced immunohistochemistry and computational modelling to comprehensively characterize the relation of plaque-associated and non-plaque-associated microglia during neurodegeneration. This approach enabled us to identify and characterize non-plaque-associated microglia as a unique and highly dynamic microglial state in a mouse model of AD. Non-plaque-associated microglia modulate network expansion, quickly adapt to environmental cues and their transition to plaque-associated microglia can be specifically modulated during disease, contrary to their reputation as a passive bystander subpopulation. This description of the dynamics of spatially segregated microglial states and their distinct molecular features may therefore open promising new avenues for state-specific therapeutic interventions during neurodegeneration.

## Description

The invention relates to the field of neurodegenerative diseases, more specifically microglia-mediated therapies.

The invention relates to a colony stimulating factor 1 receptor (CSF1 R) ligand for use in the treatment or prevention of a neurodegenerative disease in a subject.

The invention further relates to a pharmaceutical composition comprising the CSF1R ligand according to the invention for use in the treatment or prevention of a neurodegenerative disease, additionally comprising a pharmaceutically acceptable carrier.

Moreover, the invention relates to a colony stimulating factor 1 receptor (CSF1R) ligand for use in the modulation of the differentiation of non-plaque associated microglia (nonPAM) to plaque-associated microglia (PAM).

### BACKGROUND OF THE INVENTION

As versatile tissue-resident macrophages of the central nervous system (CNS) parenchyma, microglia are not only involved in tissue development and homoeostasis but also in virtually all neuroinflammatory, neurodegenerative, neuropsychiatric and neurooncological disorders¹⁻⁶. This functional engagement is typically accompanied by strong microglial reactivity and a robust numeric increase, called microgliosis, potentially induced by proliferative expansion of differentiated preexisting microglia⁷⁻⁹.

Microglia are involved in tissue development and homeostasis and play roles in various neurological disorders. This functional engagement often involves strong microglial reactivity and a robust increase in numbers, termed microgliosis. In the adult brain, microglia persist without input from circulating hematopoietic stem cell-derived progenitors during steady-state¹⁰⁻¹², originating exclusively from prenatal macrophage progenitors during early embryonic development. These macrophage precursors develop from erythromyeloid progenitors in the embryonic yolk sac¹³⁻¹⁶. Recent studies using transcriptomics have revealed phenotypic and functional heterogeneity of microglia across developmental stages, steady state, and disease conditions¹⁷⁻²². However, the specific contributions of these microglial subsets to neuronal damage and regeneration in neurodegenerative disorders, e.g., Alzheimer's disease (AD) or Huntington's disease, remain to be fully explored²³.

Alzheimer's disease (AD), affecting over 55 million people worldwide, is characterized by accumulating protein aggregates such as extracellular amyloid plaques, intraneuronal tau tangles, and cerebral amyloid angiopathy, leading to neuronal loss, inflammation, and vascular dysfunction. Activated microglia accumulate at sites of protein aggregation, particularly found at extracellular amyloid plaques. Studies based on sophisticated single-cell analysis of microglial profiles recognized distinct cellular states to neurodegenerative diseases recapitulated in AD-like mouse models²⁴⁻²⁶ or in AD in humans²⁷⁻³⁰. Among these states, "disease-associated microglia" (DAM) were described and correlated with plaque-associated microglial states in mice^{24,31,32}. This concept was refined as "microglial neurodegenerative phenotype" (MgnD) to better delineate the precise disease context of microglial activation during neurodegeneration³¹. AD-associated DAM/MgnD exhibit a notable gene signature, including high expression levels of genes such as *Itgax, Clec7a, Trem2, Apoe, Lpl,* and *Cst7.* Trem2-dependent signaling has been highlighted as an essential component for neurodegeneration-associated microglial clustering and limitation of AD pathology³³⁻³⁵.

The differentiation of microglia into this state is seen as a major contributor to disease progression, suggesting modulation of this cell state as a potential therapeutic approach.

However, despite their significance, microglial spatial heterogeneity remains poorly understood, especially concerning potential cell-directed therapies for AD. Little is known about the dynamics of spatially distributed microglial cell states in AD and their selective contributions to neurodegeneration *in vivo.* Current research has mainly focused on pathology-associated microglia cell states..

Studies and publications have proposed targeting prominent plaque-associated microglia during the disease pathology, with particular attention to the Trem2-ApoE pathway as a target.

JP2022081543A discloses methods and compositions that include antibodies, e.g., monoclonal, chimeric, humanized antibodies, antibody fragments, and the like, that specifically bind a TREM2 protein for prevent, reduce, or treat dementia.

Plaque-associated microglia (PAM) dynamically differentiate at the amyloid plaque site from adjacent non-plaque-associated microglia (nonPAM) during disease pathology. This process is characterized by clonal proliferation and subsequent differentiation of nonPAM in response to amyloid deposition. Therefore, cellular kinetics of these disease-associated microglial states at the amyloid plaque sites, their functional and spatial relationships with nonPAM and their differential modulation during disease by intrinsic and extrinsic factors, including peripheral stimuli, are still largely unexplored. Furthermore, it remains unclear how such modulation may alter disease pathogenesis and when and by which mechanisms plaque-associated microglial subsets, as well as bystander microglia, respond to environmental stimuli, allowing for modulation of their functional profiles.

None of the inventions mentioned above provide a solution to specifically foster and target non-plaque-associated microglia (nonPAM) during neurodegeneration. Moreover, it is essential to note that blocking immune activation or treating with immunosuppressive substances to modulate plaque-associated microglia could lead to severe side effects in elderly patients, including uncontrolled infections. Furthermore, therapeutic approaches targeting microglia in neurodegenerative diseases are currently rare and not yet available for patient care in clinics.

Thus, an unmet need exists for ameliorating or treating neurodegenerative disease progressions such as Alzheimer's disease or Huntington's disease. More specifically, given the current state of the art, there remains a significant demand for improved approaches and techniques to target pathological microglia associated with neurodegenerative diseases.

### SUMMARY OF THE INVENTION

In light of the prior art, the technical problem underlying the invention was providing alternative or improved means for treating or preventing a neurodegenerative disease in a subject. The present invention seeks to provide such means while avoiding the disadvantages known in the prior art.

A further objective of the invention was to provide alternative or improved means for microglia-mediated therapies of neurodegenerative diseases.

These problems are solved by the features of the independent claims. Preferred embodiments of the present invention are provided by the dependent claims.

The present invention, in one aspect, relates to a colony stimulating factor 1 receptor (CSF1R) ligand for use in the treatment or prevention of a neurodegenerative disease in a subject.

In embodiments, the CSF1R ligand is a recombinant CSF1.

In embodiments, the CSF1R ligand is a small molecule or antibody activating CSF1R.

In embodiments, the CSFR1 ligand is a small molecule or an antibody. In other embodiments, the CSF1R ligand is a mimetic of said ligand.

The person skilled in the art is familiar with various techniques to generate recombinant proteins, small molecules, antibodies, or mimetics.

In some embodiments, commercially available CSF1 recombinant proteins provide a further option for selection by skilled individuals without undue effort.

In embodiments, the CSF1R ligand is a recombinant human CSF1 protein. Non-limiting examples of commercially available recombinant human CSF1 proteins are Human CSF-1/M-CSF Recombinant Protein (#8929, Cell Signaling), Abnova^{™} CSF1 (Human) Recombinant Protein (16222300), Recombinant Human M-CSF Protein (216-MC, biotechne, R&D systems), Active Recombinant Human CSF-1/M-CSF Protein (AB-RP01221, Hycultec). In some embodiments, the CSF1R ligand is tagged.

In embodiments, the CSF1R ligand is a recombinant CSF1 protein. In other embodiments, the CSF1R ligand is a recombinant IL34 protein.

In embodiments, the CSF1R ligand binds to CSF1R. In embodiments, the CSF1R ligand activates CSF1R. In further embodiments, the CSF1R ligand initiates the CSF1-CSF1R signaling cascade.

In embodiments, the CSF1 recombinant protein, the small molecule, and/or the antibody promote phagocytosis of amyloid pathology. In embodiments, the recombinant protein, the small molecule, and/or the antibody promote tissue homeostasis.

In embodiments, the CSF1R ligand is able to cross the blood-brain barrier (BBB).

One major advantage of the present invention is that, in embodiments, the CSF1R ligand can cross the BBB. The ability to cross the BBB enables targeted treatment within the brain. This may further lead to improved treatment efficacies and minimized side effects. Thus, the ability to deliver the CSF1R ligand effectively to the brain may provide a new therapeutic avenue for modulating neurodegenerative diseases.

In embodiments, the CSF1R ligand modulates the differentiation of non-plaque associated microglia (nonPAM) to plaque-associated microglia (PAM) or directly the function of PAM or nonPAM, preferably within the CNS.

The findings of two spatially and functionally distinct microglial cell populations, namely PAM and nonPAM, offer significant therapeutic advantages. Subset-specific targeting is highly desirable in a disease setting to ensure that the homeostatic functions of microglia in unaffected brain regions are maintained. The present invention effectively addresses this necessity, emphasizing its potential to preserve overall brain health during targeted interventions.

The examples below further show that a number of target genes and pathways could serve as potential modulators of nonPAM during the disease. In embodiments, nonPAM express higher levels of colony-stimulating factor 1 receptor (CSF1R) than PAM.

In one embodiment, peripheral injection of the Csf1r ligand colony stimulating factor 1 (CSF1) during the early stages of AD decreases PAM expansion from nonPAM.

In other embodiments, peripheral injection of the Csf1r ligand colony stimulating factor 1 (Csf1) during the early stages of AD leads to amelioration of disease pathology.

In embodiments, nonPAM exhibit a higher expression and/or accessible gene locus for *Csf1rthan* PAM. In embodiments, PAM exhibit a lower expression and/or accessible gene locus for Csf1rthan nonPAM.

In embodiments, PAM exhibit a characteristic gene signature including signature genes of disease-associated microglia (DAM) and/or microglial neurodegenerative phenotype (MgnD).

In embodiments, PAM represent a functionally exhausted microglial population. In further embodiments, PAM have a limited capacity to react upon peripheral stimulation.

In embodiments, biological functions are differentially activated in non-PAM than in PAM.

In embodiments, nonPAM exhibit enriched pathways linked to differentially accessible regions (DARs) with increased chromatin accessibility compared to PAM. In embodiments, non-PAM exhibit an increased chromatin accessibility compared to PAM. In one embodiment, increased chromatin accessibility in nonPAM are associated with accessibility changes in gene loci connected to positive regulation of DNA demethylation, DNA demethylation of male pronucleus, regulation of DNA demethylation, chromatin reprogramming in the zygote, RNA destabilization, negative regulation of multicellular organism growth, desensitization of G-protein coupled receptor protein signaling pathway by arrestin, cellular response to granulozyte macrophage colony-stimulating factor stimulus, vasodilation by norepinephrine-epinephrine involved in regulation of systemic arterial blood pressure, heart generation, positive regulation of male germ cell proliferation, C5 methylation of cytosine, urate biosynthetic process, and/or thyroid hormone transport.

In embodiments, PAM adapt distinct epigenetic characteristics during pathology at the site of amyloid plaques. In embodiments, nonPAM resemble molecular features of homeostatic microglia.

In embodiments, the differentiation of nonPAM to PAM comprises the clonal expansion of PAMs.

As shown in the examples below, the murine mouse model for AD demonstrates that non-plaque associated microglia (nonPAM) dynamically give rise to plaque-associated microglia (PAM) at amyloid deposits by clonal expansion. It was further tested how clonal expansion and differentiation of nonPAM to PAM are modulated by peripheral factors such as gut dysbiosis (antibiotic (ABX) depletion of endogenous microbiota) or chronic inflammation (low dose application of lipopolysaccharides (LPS)) and how this modulation affects AD disease pathology. Both treatments seem to actively modulate the transition of nonPAM to PAM in the early stages of AD but not in the late disease stages. ABX-treatment decreased clonal expansion of PAMs at the amyloid deposits, whereas LPS dynamically expanded PAMs and increased amyloid deposition.

In embodiments, the CSF1R ligand actively modulates differentiation of nonPAM to PAM. In embodiments, the CSF1R ligand allows nonPAM differentiation to amyloid-restrictive PAM.

In embodiments, the CSF1R ligand decreases clonal expansion from nonPAM to PAM.

In embodiments, PAM are accumulating microglia at the extracellular amyloid deposits and express one or more of the genes CLEC7A, AXL, TREM2, APOE, and/or ITGAX.

In embodiments, activated microglia are primarily found at extracellular amyloid plaques, and microglia accumulate at plaque sites.

As shown in the examples below, high-throughput transcriptomic profiling of microglia in a mouse model for AD revealed substates of microglia with a highly activated gene expression profile and expression of activation markers, such as *Clec7a, Axl, Trem2, Apoe,* and *Itagx,* which are widely accepted as a core signature of plaque-associated microglia in the CNS.

In embodiments, genes associated with microglial activation are *Clec7a, Axl, Trem2, Apoe,* and *Itagx.* In embodiments of the invention, the genes mentioned above represent five examples of genes associated with microglial activation. In further embodiments of the invention, other (yet unknown) genes (may) exist, such as e.g. *Bhlhe40, Csf1, Cst7, Ctsb, Ctsd, Ctsl, Cybb, Fabp5, Fth1, Gnas, Gpnmb, Grn, Il1b, Lgals3, Lilrb4, Lpl, Lyz2, Msr1, Nos2, Spp1, Tfec, Tyrobp, Vegfa,* that are associated with microglial activation.

In embodiments, genes associated with homeostatic microglia signature are *P2η12, Csf1r, Cx3cr1, Tmem119, Pu.1,* and *Sall1.* In embodiments of the invention, the genes mentioned above represent five examples of genes associated with homeostatic microglia. In further embodiments, other (yet unknown) genes (may) exist that are associated with microglia activation. In embodiments, nonPAM express high levels of *Tmem119, P2ry12* and *Cx3cr1.*

In embodiments, PAM and nonPAM retain their respective disease-associated or homeostatic gene signature upon CSF1R ligand treatment.

In embodiments, CSF1R ligand treatment does not change the core signatures of nonPAM and PAM.

As shown in the examples below, single-cell transcriptomic profiling revealed gene-specific regulations in nonPAM and PAM. However, nonPAM were much more responsive to external stimuli and/or peripheral changes compared to PAM in the early stages of neurodegenerative diseases such as AD. This was further confirmed by analyzing the chromatin accessibility of both microglia subpopulations, which revealed differentially accessible gene loci in nonPAM compared to PAM.

In embodiments, nonPAM are more transcriptionally responsive to external stimuli and/or therapeutic modulations than PAM, preferably in the early stages of neurodegenerative diseases.

In embodiments, nonPAM are transcriptionally responsive to external stimuli and/or therapeutic modulations, such as the treatment with CSF1R ligand.

In embodiments, PAM differentiation is transcriptionally responsive to treatment with CSF1R ligand and results in a modulated PAM state,

In embodiments, the CSF1R ligand enhances phagocytic capacity, preferably for amyloid deposits, metabolic programming, migratory capacity, cell survival, inhibition so cellular senescent-associated secretory programs of PAM, induces the release of neurotrophic factors, and/or ameliorates PAM expansion.

In embodiments, the CSF1R ligand ameliorates amyloid pathology.

In embodiments, the CSF1R ligand enlarges the CD68+ lysosomal compartment in PAM. In embodiments, the CSF1R ligand increases microglial phagocytosis, preferably at the site of amyloid pathology.

In embodiments, the CSF1R ligand improves the functional fitness, such as phagocytosis of the microglial subsets, allowing amyloid restriction.

In embodiments, the CSF1R ligand ameliorates PAM expansion.

In embodiments, the CSF1R ligand reduces pathology-driven transition of nonPAM to PAM.

In embodiments, the CSF1R ligand reduces the number of amyloid plaques. In embodiments, the CSF1R ligand reduces the size of amyloid plaques.

In embodiments, the CSF1R ligand upregulates genes associated with metabolic regulation, preferably towards autophagy and/or oxidative phosphorylation. In embodiments, the CSF1R ligand induces autophagy. In embodiments, the CSF1R ligand induces oxidative phosphorylation. In embodiments, the CSF1R ligand reduces pro-inflammatory immune response.

In embodiments, the subject is experiencing or suffering from one or more of (neuro) cognitive impairment, impaired motoric skills, loss of smell, difficulties with language, clinical symptoms of dementia, attention deficits, and/or impaired memory.

In embodiments, the subject exhibits Cerebral Amyloid Angiopathy (CAA) pathology.

In embodiments, the subject is suspected or diagnosed to exhibit abnormal deposits of amyloid beta (Aβ) within their brain and/or central nervous system (CNS), preferably comprising extracellular amyloid plaques, and/or neurofibrillary tau tangles.

In embodiments, the subject is suspected or diagnosed to exhibit abnormal deposits of amyloid beta (Aβ) deposits within small- to medium-sized blood vessels of the brain and/or leptomeninges.

In embodiments, the CSF1R ligand reduces soluble amyloid β-peptide Aβ₁₋₄₀ and/or Aβ₁₋₄₂ levels. In further embodiments, the CSF1R ligand reduces insoluble amyloid β-peptide Aβ₁₋₄₀ and/or Aβ₁₋₄₂ levels.

In other embodiments, the CSF1R ligand does not affect β-amyloid precursor protein (APP) processing.

In embodiments, the neurodegenerative disease is Alzheimer's disease (AD), Lewy body dementia, Parkinson's disease (PD), multiple sclerosis (MS), motor neuron disease (MND), Huntington disease (HD), progressive supranuclear palsy (PSP), corticobasal degeneration (CBD), multiple system atrophy (MSA), amyotrophic lateral sclerosis (ALS), schizophrenia, depression, and/or an autism-spectrum disorder, preferably Alzheimer's disease (AD), multiple sclerosis (MS), and autism-spectrum disorders.

In embodiments, a patient suffers from a neurodegenerative disease.

In embodiments, a patients suffers from Alzheimer's disease (AD), Lewy body dementia, Parkinson's disease (PD), multiple sclerosis (MS), motor neuron disease (MND), Huntington disease (HD), progressive supranuclear palsy (PSP), corticobasal degeneration (CBD), multiple system atrophy (MSA), amyotrophic lateral sclerosis (ALS), schizophrenia, depression, and/or an autism-spectrum disorder.

In embodiments, the CSF1R ligand modulates differentiation of nonPAM to PAM, thereby ameliorating neurodegenerative disease progression.

In embodiments, the CSF1R ligand ameliorates PAM expansion at early stages of neurodegenerative diseases progression.

In embodiments, wherein the treatment reduces inflammation, such as type I or type II interferon-mediated inflammation and/or NFkB activation within the central nervous system (CNS) of the subject.

Reactive microglia promote neuroinflammation and induce NFkB signaling.

Thus, in embodiments, the CSF1R ligand reduces type I or type II interferon signaling.

In further embodiments, the CSF1R ligand reduces NFkB activation.

In other embodiments, the CSF1R ligand reduces genes, gene sets, and/or pathways associated with inflammatory responses. In embodiments, the CSF1R ligand reduces inflammatory genes. In other embodiments, the CSF1R ligand reduces inflammatory signaling pathways. In embodiments, the CSF1R ligand alleviates pathways associated with inflammation.

In further embodiments, the CSF1R ligand increases mitochondrial features and metabolic fitness.

In one aspect, the present invention relates to a pharmaceutical composition comprising the CSF1R ligand according to any one of the preceding claims for use in the treatment or prevention of a neurodegenerative disease, additionally comprising a pharmaceutically acceptable carrier.

In embodiments, the composition is administered intracerebrally, intrathecally, intravenously or subcutaneously.

In a preferred embodiment, the composition is administered by intravenous injection.

This method offers greater convenience for patients, particularly those of advanced age, enabling them to undergo treatment regularly with ease.

In another aspect, the present invention relates to a colony stimulating factor 1 receptor (CSF1R) ligand for use in the modulation of the differentiation of non-plaque associated microglia (nonPAM) to plaque-associated microglia (PAM).

The various aspects of the invention are unified by, benefit from, are based on and/or are linked by the structural and/or functional features, including functional properties and beneficial technical effects, CSF1R ligand for use in the treatment or prevention of a neurodegenerative disease in a subject described herein. The features disclosed in the context of the CSF1R ligand also apply to and are considered disclosed in the context of the pharmaceutical composition comprising the CSF1R ligand, and vice versa.

The embodiments of the invention aim to i) modulate the differentiation of nonPAM to PAM to ameliorate disease progression, ii) support PAM in efficiently phagocytosing and restricting amyloid deposits, and iii) support neuronal circuits by release of neurotrophic factors. Since engaging nonPAM in an undiseased setting did not show any negative effects on the microglia population, the invention demonstrates a safe approach for patients.

### DETAILED DESCRIPTION OF THE INVENTION

The present invention relates to a colony stimulating factor 1 receptor (CSF1R) ligand for use in the treatment or prevention of a neurodegenerative disease in a subject.

All words and terms used herein shall have the same meaning commonly given to them by the person skilled in the art unless the context indicates a different meaning. All terms used in the singular shall include the plural of that term and vice versa.

### Genes and proteins

**Recombinant proteins** are genetically engineered proteins produced by manipulating genes and employing specific expression systems. In therapeutic contexts, recombinant proteins are used to replace, increase, or inhibit specific proteins and/or treat various conditions. These recombinant proteins encompass a wide range of therapeutic agents, including but not limited to hormones, interferons, interleukins, growth factors, tumor necrosis factors, blood clotting factors, thrombolytic drugs, and enzymes. These proteins are instrumental in treating various diseases such as diabetes, dwarfism, myocardial infarction, congestive heart failure, cerebral apoplexy, multiple sclerosis, neutropenia, thrombocytopenia, anemia, hepatitis, rheumatoid arthritis, asthma, Crohn's disease, and cancers therapies. Therapeutic recombinant proteins have evolved through three generations. The first generation consists of proteins with native structures, while the second generation introduces proteins with enhanced properties, including pharmacokinetics, biodistribution, specificity, efficacy, and reduced side effects. The third generation features recombinant proteins optimized for novel routes of administration, novel formulations, heightened efficacy, and enhanced safety profiles. Basic methods for generating recombinant proteins involve isolating and cloning genes into expression vectors, followed by expression in suitable host systems and purification. The skilled person is familiar with techniques to generate recombinant proteins.

The terms "recombinant protein" and "recombinant ligand" may be used interchangeably.

In embodiments, the CSF1R ligand is a recombinant protein.

**Colony-stimulating factor 1 receptor** (CSF1**R),** also known as "macrophage colony-stimulating factor receptor (M-CSFR)" or "CD115", is a cell-surface protein encoded by the *CSF1R* gene. It acts as a receptor for colony-stimulating factor 1 (CSF1) and interleukin-34 (IL-34). Predominantly expressed in myeloid cells, CSF1R signaling plays crucial roles in the survival, proliferation, and differentiation of various myeloid cell types. Its activation is regulated by alternative promoters and transcription factors, impacting tissue-specific expression. Structurally, CSF1R is a tyrosine kinase transmembrane receptor belonging to the CSF1/PDGF receptor family. Upon ligand binding, it undergoes dimerization and autophosphorylation, initiating downstream signaling cascades essential for myeloid cell function. CSF1R signaling is implicated in numerous diseases, including but not limited to cancer, neurodegeneration, and inflammatory bone disorders. Additionally, it influences non-myeloid cells, such as neural progenitor cells, contributing to neurogenesis and neurological disorders. In microglia, CSF1R signaling plays a pivotal role in development, survival, and maintenance, influencing neuroinflammatory responses. During embryonic development, CSF1R signaling facilitates the migration of microglia precursor cells to the brain and their subsequent differentiation and expansion. Subsequently, in perinatal stages, microglia aid in synaptic pruning, which is crucial for refining neuronal circuits. In adulthood, CSF1R is essential for microglial proliferation and survival. Dysregulation of CSF1R signaling in microglia is implicated in neurodegenerative diseases, affecting neuronal function and viability.

In embodiments, the CSF1R recombinant ligand is used to treat or prevent neurodegenerative diseases in a subject.

In embodiments, the CSF1R recombinant ligand is used to prevent the progression of neurodegenerative diseases.

In embodiments, the CSF1R recombinant ligand is used to decelerate the progression of neurodegenerative diseases.

In embodiments, the CSF1R recombinant ligand is a first, second or a third-generation recombination protein.

As disclosed herein, a **ligand** is a molecule, atom, or ion that binds reversibly to a protein, forming a complex to serve a specific biological purpose, whereby enzymes are also included under ligands. Ligand-target interactions occur through weak intermolecular forces such as ionic bonds, hydrogen bonds, Van der Waals forces, and hydrophobic effects. Ligands include substrates, inhibitors, activators, signaling lipids, and neurotransmitters, amongst many other molecules, and they can trigger conformational changes in a protein, altering functional state. Affinity measures the strength of the interaction between a ligand and its binding site on a protein, with high-affinity binding indicating strong attraction between the molecules. Ligand binding affinity is crucial for determining the physiological response triggered by the ligand, whether it acts as an agonist, antagonist, or partial agonist. Various methods, including radioligand binding assays and spectroscopic techniques, are employed to study protein-ligand interactions and determine binding affinities.

The terms "CSF1R ligand" and "CSF1-like ligand" may be used interchangeably and describe a ligand binding to CSF1R.

In certain embodiments, the CSF1R ligand is CSF1.

In other embodiments, the CSF1R ligand is IL34.

**Small molecules,** with a molecular weight of less than 900 Daltons, constitute a diverse group of organic compounds that play essential roles in various biological processes and pharmacological applications. Unlike larger molecules such as proteins and nucleic acids, small molecules can regulate biological functions by binding to specific targets, including receptors and enzymes. These compounds can exert their effects by modulating signaling pathways, inhibiting enzymatic activity, or disrupting protein-protein interactions. Non-limiting examples of small molecules are metabolites (e.g., sugars), signaling molecules (e.g., hormones, neurotransmitters, cytokines), natural products (e.g., alkaloids, glycosides, terpenes, antibiotics), drugs, toxins, poisons, lipids (e.g., triglycerides, phospholipids, cholesterol), carbohydrates (e.g., glucose, starch, cellulose), amino acids, nucleotides (e.g., adenine, guanine, thymine, cytosine), heterocyclic compounds (e.g., alkaloids, purines, pyrimidines), and/or aromatic compounds (e.g., benzene, toluene, naphthalene).

As used herein, an **antibody** generally refers to a protein consisting of one or more polypeptides substantially encoded by immunoglobulin genes or fragments of immunoglobulin genes. Where the term "antibody" is used, the term "antibody fragment" may also be considered to be referred to. The recognized immunoglobulin genes include the kappa, lambda, alpha, gamma, delta, epsilon, and mu constant region genes and the myriad immunoglobulin variable region genes. Light chains are classified as either kappa or lambda. Heavy chains are classified as gamma, mu, alpha, delta, or epsilon, defining the immunoglobulin classes IgG, IgM, IgA, IgD, and IgE. The basic immunoglobulin (antibody) structural unit is known to comprise a tetramer or dimer. Each tetramer is composed of two identical pairs of polypeptide chains, each pair having one "light" (L) (about 25 kD) and one "heavy" (H) chain (about 50-70 kD). The N-terminus of each chain defines a variable region, primarily responsible for antigen recognition. The terms "variable light chain" and "variable heavy chain" refer to these variable regions of the light and heavy chains, respectively. A variable region of an antibody refers to the variable region of the antibody light chain or the variable region of the antibody heavy chain, alone or in combination. The variable regions of the heavy and light chains each consist of four framework regions (FR) connected by three complementarity-determining regions (CDRs), also known as hypervariable regions. The CDRs in each chain are held together in close proximity by the FRs and, with the CDRs from the other chain, contribute to the formation of the antigen-binding site of antibodies. Optionally, the antibody, or the immunological portion of the antibody, can be chemically conjugated to or expressed as a fusion protein with other proteins.

In embodiments, the CSF1R ligand is a small molecule activating CSF1R.

In other embodiments, the CSF1R ligand is an antibody activating CSF1R.

CSF1R ligands are also commercially available and may, in embodiments, be employed for use in the treatment or prevention of a neurodegenerative disease in a subject.

The CSF1R ligand may also include any mimetics resembling the structure and function of a native CSF1R ligand.

Dectin-1, encoded by the **CLEC7A** gene, belongs to the C-type lectin/C-type lectin-like domain (CTL/CTLD) superfamily, characterized by its extracellular C-type lectin-like domain fold and cytoplasmic domain containing a partial immunoreceptor tyrosine-based activation motif. It acts as a pattern-recognition receptor for β-glucans from fungi and plants, facilitating innate immune responses. Dectin-1 expression is found on various immune cells, including macrophages, dendritic cells, and B cells. Upon ligand binding, dectin-1 triggers intracellular signaling via the ITAM-like motif, leading to the activation of pathways involving Syk, PKC-δ, CARD9, BCL10, and NF-κB, resulting in the production of inflammatory cytokines and chemokines. Additionally, dectin-1 operates as a co-stimulatory molecule, activating T cells upon recognition of endogenous ligands. The gene is closely linked to other CTL/CTLD superfamily members on chromosome 12p13 within the natural killer gene complex region.

In embodiments, the CSF1R ligand modulates PAM expressing CLEC7A. In embodiments, the CSF1R ligand modulates PAM that accumulate at extracellular amyloid deposits and express CLEC7A.

The AXL gene encodes the tyrosine-protein kinase receptor AXL, a member of the Tyro3-Axl-Mer (TAM) receptor tyrosine kinase subfamily. AXL functions as a cell surface receptor that transduces signals from the extracellular matrix into the cytoplasm by binding growth factors like GAS6, regulating various physiological processes, including cell survival, proliferation, migration, and differentiation. Structurally, AXL possesses an extracellular domain composed of two immunoglobulin-like motifs and two fibronectin type-!!! motifs. Upon ligand binding, AXL undergoes dimerization and autophosphorylation, leading to downstream activation of signaling pathways such as PI3K-AKT-mTOR, MEK-ERK, NF-κB, and JAK/STAT. AXL is expressed in normal tissues and plays a role in the efficient clearance of apoptotic material and modulation of immune responses. In cancer, AXL is implicated as a driver of diverse cellular processes critical for tumor development, growth, and metastasis. Additionally, AXL has been implicated in chronic fibrotic diseases and plays a role as a host cell receptor for several viruses, including Marburg, Ebola, Lassa, and SARS-CoV-2, suggesting its involvement in viral infection mechanisms.

In embodiments, the CSF1R ligand modulates PAs expressing AXL. In embodiments, the CSF1R ligand modulates PAM that accumulate at extracellular amyloid deposits and express AXL.

**Triggering receptor expressed on myeloid cells 2 (TREM2)** is a protein encoded by the TREM2 gene on chromosome 6p21.1 in humans. This gene undergoes alternative splicing, resulting in the production of different isoforms of the TREM2 protein. Structurally, TREM2 is a transmembrane protein with an extracellular region, a membrane-traversing segment, and an intracellular component. The extracellular region contains an Ig-like V-type domain responsible for ligand binding, while the intracellular component interacts with signaling proteins like DAP10 and DAP12. Enzymes such as ADAM10 and ADAM17 can cleave the ectodomain, releasing soluble TREM2 (sTREM2) into the serum and cerebral spinal fluid. Functionally, TREM2 is expressed on various immune cells, including but not limited to macrophages, dendritic cells, osteoclasts, and microglia. It binds anionic molecules, glycoproteins, lipids, and bacterial products, leading to downstream signaling through proteins like DAP10 and DAP12. This signaling modulates immune responses, inflammation, and phagocytosis. In the brain, TREM2 is involved in synaptic pruning by microglia, shaping neuronal circuitry. Mutations or variants in the TREM2 gene are associated with neurodegenerative disorders such as Alzheimer's disease (AD) and Nasu-Hakola disease (PLOSL). TREM2 expression is elevated in various cancer types and correlates with poor prognosis in some cases. Dysregulation of TREM2 signaling contributes to the pathogenesis of diseases like inflammatory bowel diseases (IBD) and liver disease.

In embodiments, the CSF1R ligand modulates PAM expressing TREM2. In embodiments, the CSF1R ligand modulates PAM that accumulate at extracellular amyloid deposits and express TREM2.

**Apolipoprotein E (APOE)** is a protein involved in lipid metabolism in mammals. It is encoded by the ApoE gene located on chromosome 19. APOE belongs to the apolipoprotein family and is found in various lipoprotein particles circulating in the body, including chylomicron remnants, VLDL, IDL, and some HDL. Its interaction with the low-density lipoprotein receptor (LDLR) is essential for the normal processing of triglyceride-rich lipoproteins. APOE is primarily produced by the liver and macrophages in peripheral tissues and plays a vital role in cholesterol metabolism. In the central nervous system, astrocytes are the main producers, and APOE transports cholesterol to neurons via specific receptors, crucial for brain function. It acts as the principal cholesterol carrier in the brain and also qualifies as a checkpoint inhibitor of the classical complement pathway. The APOE gene is polymorphic, with three major alleles: APOE-ε2, APOE-ε3, and APOE-ε4. These alleles, differing by only one or two amino acids, significantly affect APOE structure and function. The ε4 variant, in particular, is associated with an increased risk for Alzheimer's disease (AD) and cardiovascular diseases. ApoE polymorphisms have diverse effects, with some isoforms offering protective effects against cognitive decline. Beyond its role in lipid metabolism, APOE is implicated in various biological processes, including immunoregulation and cognition. It interacts with multiple receptors involved in lipoprotein uptake, modulates inflammation, and influences cellular responses to infections. While the ε4 allele is a significant risk factor, other factors like mid-life cholesterol levels and blood pressure contribute to disease risk.

In embodiments, the CSF1R ligand modulates PAM expressing APOE. In embodiments, the CSF1R ligand modulates PAM that accumulate at extracellular amyloid deposits and express ApoE.

CD11c, also known as **Integrin alpha X (ITGAX),** is a gene that encodes the CD11c protein, a member of the integrin family. Integrins are heterodimeric integral membrane proteins consisting of alpha and beta chains. CD11c combines with the beta 2 chain (ITGB2) to form a leukocyte-specific integrin known as inactivated-C3b (iC3b) receptor 4 (CR4). This integrin complex plays a crucial role in various immune functions, including the adherence of neutrophils and monocytes to stimulated endothelial cells and the phagocytosis of complement-coated particles. CD11c is predominantly found on human dendritic cells, monocytes, macrophages, neutrophils, and some B cells. Its expression induces cellular activation and contributes to triggering neutrophil respiratory bursts. Moreover, CD11c is expressed in certain leukemias, such as hairy cell leukemias, acute nonlymphocytic leukemias, and some B-cell chronic lymphocytic leukemias. Functionally, CD11c is involved in various processes, including phagocytosis, cell migration, cytokine production by monocytes/macrophages, and induction of T-cell proliferation by Langerhans cells. CD11c binds to complement fragment (iC3b), provisional matrix molecules (fibrinogen), and the Ig superfamily cell adhesion molecule, ICAM-1. It has been shown to recognize ICAM-2 and VCAM-1, contributing to monocyte capture and transmigration on inflamed endothelial cells.

In embodiments, the CSF1R ligand modulates PAM expressing ITGAX. In embodiments, the CSF1R ligand modulates PAM that accumulate at extracellular amyloid deposits and express ITGAX.

In embodiments of the invention, CLEC7A, AXL, TREM2, APOE, and ITAGX represent possible activation markers of PAM. In embodiments, the CSF1R ligand modulates PAM expressing CLEC7A, AXL, TREM2, APOE, and/or ITAGX.

In further embodiments of the invention, the CSF1R ligand can regulate PAM expressing other activation markers or PAM expressing other activation markers in addition to the activation markers mentioned above.

**"Gene expression"** is the process by which information encoded in genes is utilized to synthesize functional gene products, typically through the transcription of genes into RNA, which is then translated into proteins. However, RNA can also serve as functional end products that regulate gene expression and protein translation, including non-coding RNAs like IncRNAs, rRNAs, tRNAs, microRNAs, and siRNAs. Methods for measuring gene expression include but are not limited to Northern blotting, microarray, qPCR, dPCR, RNA-Seq, and NGS.

In embodiments, the expression of genes of the present invention can be analyzed using Northern blotting, microarray, qPCR, dPCR, RNA-Seq, and/or NGS. A "high" or "low" gene expression does not require a threshold value. Rather, the expression of a gene may be measured and assessed relative to other genes in the same cell and/or tissue under similar conditions. A person skilled in the art can measure and assess gene expression without undue effort.

### Molecules

**Amyloid beta (Aβ)** is a product of the amyloid precursor protein (APP) proteolysis. It forms the primary component of amyloid plaques. Aβ peptides aggregate into various oligomeric forms, with soluble oligomers considered most neurotoxic. Aβ's physiological role remains unclear, but it is suggested that it participates in kinase activation, oxidative stress protection, and cholesterol regulation. Dysregulation of Aβ clearance mechanisms, including the glymphatic system, contributes to its accumulation. Genetic mutations in APP lead to familial Alzheimer's disease, while Aβ elevation is observed in sporadic cases. Therapeutic strategies targeting Aβ include immunotherapy, Aβ oligomerization inhibition, and Aβ-degrading enzyme enhancement.

In embodiments, the subject is suspected or diagnosed to exhibit abnormal deposits of amyloid beta within their brain and/or CNS.

In embodiments, deposits of Aβ are abnormal within the brain and/or CNS of a subject.

**Amyloid plaques,** neuritic plaques, or senile plaques are hallmark lesions found in the brains of individuals with neurodegenerative diseases, e.g., Alzheimer's disease, characterized by the abnormal accumulation of protein fragments known as amyloid beta (Aβ). These plaques predominantly occur in the grey matter of the brain and are often associated with degenerative neuronal elements, microglia, and astrocytes. While some plaques are a normal part of brain aging, neurodegenerative diseases, such as Alzheimer's disease, are characterized by abundant plaques and neurofibrillary tangles. Amyloid plaques vary in size and shape, ranging from small, wispy accumulations to larger, dense, or diffuse masses. They form when Aβ proteins misfold and aggregate into oligomers and longer polymers, ultimately leading to the deposition of insoluble amyloid fibrils. Amyloid plaques can be visualized using various staining techniques and are typically immunoreactive to antibodies directed against Aβ. The anatomical distribution of amyloid plaques in Alzheimer's disease follows a progressive pattern, beginning in neocortical areas and subsequently spreading to deeper brain regions. Specifically, plaques initially appear in association areas of the neocortex, followed by involvement of allocortical regions, the hippocampal formation, amygdala, basal ganglia, diencephalon, midbrain, medulla oblongata, pons, and cerebellum. At end-stage Alzheimer's disease, plaques can be found in most brain areas, with the spinal cord being relatively spared but still occasionally affected.

In embodiments, deposits of Aβ are abnormal within the brain and/or CNS of a subject, preferably comprising amyloid plaques.

In embodiments, PAM accumulate at amyloid plaque sites.

In embodiments, the CSF1R ligand enhances phagocytic capacity for amyloid deposits.

**Tau proteins** are primarily expressed in neurons of the central nervous system (CNS) and are crucial for regulating the assembly and stabilization of microtubules. Encoded by the MAPT gene, tau exists in six isoforms in the adult human brain, generated through alternative splicing, with variations in N-terminal domains and repeated binding domains. Its subcellular localization varies, with predominant distribution in axonal regions, dendritic spines, and even the nucleus, influenced by specific isoforms. Structurally, tau comprises a projection domain and a microtubule-binding domain, with a proline-rich region facilitating interactions with signaling proteins. Post-translational modifications, notably phosphorylation, regulate tau's function, impacting its binding affinity to microtubules and aggregation propensity, particularly evident in neurodegenerative diseases like Alzheimer's disease (AD). Tau aggregation characterizes tauopathies, with multiple factors influencing this process, including specific phosphorylation sites and truncation. Tau's main functions include regulating microtubule dynamics, axonal transport, synaptic plasticity, and even roles in neurogenesis and nucleic acid integrity. Furthermore, tau can propagate pathology through various mechanisms, including cell-to-cell transmission facilitated by extracellular release and internalization.

In embodiments, hyperphosphorylated intraneuronal tau tangles are abnormal within the brain and/or CNS of a subject.

**Neurofibrillary tangles (NFTs)** are intracellular aggregates of hyperphosphorylated tau protein, primarily associated with Alzheimer's disease (AD) but also present in other tauopathies. The formation of NFTs involves the hyperphosphorylation of tau, leading to its aggregation into insoluble structures within neurons. These tangles progress through different stages, from early formations to mature tangles and, ultimately, ghost tangles. Various factors contribute to NFT formation, including mutated tau genes, traumatic brain injury, and potential links to aluminum exposure. NFTs play a significant role in cognitive impairment, and their presence correlates with neuron loss in diseases like AD.

In embodiments, deposits of hyperphosphorylated intraneuronal tau tangles are abnormal within the brain and/or CNS of a subject, preferably comprising neurofibrillary tangles.

**Neurotrophic factors (NTFs)** are a diverse group of biomolecules primarily composed of peptides or small proteins, crucial for the growth, survival, and differentiation of neurons during development and in the mature nervous system. They exert their effects by signaling through tyrosine kinases, particularly receptor tyrosine kinases. NTFs are essential in promoting neuronal survival, inducing synaptic plasticity, and modulating long-term memory formation. They also contribute to initial neuronal growth and development in both the central and peripheral nervous systems, with the potential for regenerating damaged neurons. NTFs are categorized into families such as neurotrophins, glial cell-line derived neurotrophic factor family ligands (GFLs), and neuropoietic cytokines, each with distinct signaling mechanisms. Notably, Brain-derived neurotrophic factor (BDNF), a prominent neurotrophin, plays a pivotal role in synaptic activity, long-term synaptic memory formation, and the pathophysiology of various neurological and psychiatric disorders. Additionally, BDNF has extraneuronal functions in non-neuronal tissues, implicating its involvement in diverse physiological processes beyond the nervous system.

### Cells and Tissues

The **blood-brain barrier (BBB)** is a highly selective semipermeable border of endothelial cells that regulates the transfer of solutes and chemicals between the circulatory and central nervous systems, thus protecting the brain from harmful or unwanted substances in the blood. It is formed by endothelial cells of the capillary wall, astrocyte end-feet, and pericytes embedded in the capillary basement membrane. This system allows the passage of some small molecules by passive diffusion and the selective and active transport of various nutrients, ions, organic anions, and macromolecules crucial to neural function. The BBB restricts the passage of pathogens, solutes, and large or hydrophilic molecules into the cerebrospinal fluid while allowing the diffusion of hydrophobic molecules and small non-polar molecules. Specialized brain structures called circumventricular organs (CVOs) have highly permeable capillaries, unlike the BBB. These organs enable rapid detection of circulating signals and facilitate the transport of brain-derived signals into the circulating blood, serving as points of bidirectional blood-brain communication for neuroendocrine function. The BBB comprises endothelial cells, astrocyte end-feet, and pericytes, with tight junctions between endothelial cells forming a selective diffusion barrier. Circumventricular organs lack the tight junctions present in BBB capillaries, allowing for the diffusion of blood-borne molecules.

The ability of a ligand to cross the BBB depends on its lipophilicity, molecular weight, charge, blood binding etc. The person skilled in the art is aware of the specific characteristics necessary for a compound/ligand to successfully cross the BBB. In embodiments, the CSF1R ligand can cross the blood-brain barrier (BBB). In embodiments, the CSF1R ligand is a lipid soluble molecule. In embodiments, the CSF1R ligand is positively charged.

**Microglia** are resident immune cells of the central nervous system (CNS) responsible for maintaining tissue homeostasis, surveillance, and immune defense. They originate from the yolk sac and are distributed throughout the brain and spinal cord. Microglia exist in various forms, including ramified, reactive, non-phagocytic, phagocytic, amoeboid, gitter cells, , and juxtavascular. These cells play multiple roles, including scavenging cellular debris, phagocytosing foreign materials, regulating extracellular signaling, presenting antigens, exerting cytotoxic effects, and promoting tissue repair. **Plaque-associated microglia (PAM)** are a distinct population of myeloid cells attracted to insoluble Amyloid β (Aβ) plaques in the brains of Alzheimer's disease (AD) patients. They form dense clusters around these plaques and undergo functional and morphological transformations. PAM express known myeloid markers shared among macrophage progeny and microglia, but their molecular signature has only been indirectly inferred. These cells are characterized by specific disease-associated gene expression signatures and play critical roles in plaque formation, compaction, and neuroinflammation. In contrast, **non-plaque-associated microglia (nonPAM)** exhibit only minor morphological alterations compared to control microglia. However, both PAM and nonPAM display significant temporal diversity in microglial morphology during AD progression.

The **central nervous system (CNS)** comprises the brain and spinal cord, each with distinct structures and functions. The brain consists of two hemispheres responsible for higher cognitive functions, emotion, and sensory processing. Deeper brain structures like the basal ganglia and thalamus regulate motor coordination and sensory relay, respectively. The hypothalamus governs homeostatic functions such as appetite and temperature regulation. The brainstem, including the medulla, pons, and midbrain, controls vital functions like breathing, heart rate, and arousal. The cerebellum coordinates voluntary movements and balance. Additionally, the spinal cord relays sensory information to the brain and transmits motor commands from the brain to the body. Thus, these CNS components integrate sensory input, coordinate motor responses, regulate physiological functions, and mediate complex cognitive processes essential for adaptation and survival.

### Cellular processes

**Phagocytosis** is a cellular process whereby a cell engulfs large particles, typically greater than 0.5 µm in diameter, through its plasma membrane, forming an internal compartment called the phagosome. This process is pivotal in removing pathogens and cell debris, contributing significantly to the immune response and tissue homeostasis. Professional phagocytes such as macrophages, neutrophils, monocytes, dendritic cells, and osteoclasts efficiently execute phagocytosis, aided by specific receptors recognizing opsonins or molecular patterns on target particles. Following detection, phagocytic receptors initiate signaling pathways that remodel the cell membrane, leading to the formation of the phagosome. Subsequently, the phagosome matures into a phagolysosome through fusion with lysosomes, where engulfed material is degraded, aided by various enzymes and reactive oxygen species. Phagocytosis is essential for maintaining health and is crucial in immune defense mechanisms. As used herein, "phagocytic capacity" refers to a cell's ability to engulf and digest foreign particles, pathogens, or debris.

In one embodiment, the CSF1R ligand enhances phagocytic capacity.

**Cell migration** is a fundamental process crucial for developing and maintaining multicellular organisms. It encompasses various movements, from large-scale migrations of epithelial sheets during gastrulation to the migration of individual cells in nervous system development. In adult organisms, cell migration is essential for immune responses, wound repair, and tissue homeostasis.

However, aberrant cell migration is implicated in various pathologies, including cancer metastasis. Mechanisms of cell migration involve complex interactions between the cytoskeleton, membrane dynamics, and signaling pathways. Cells can migrate individually, such as leukocytes during immune responses, or collectively as multicellular clusters during processes like wound healing.

**Cellular senescence** is a state in which cells undergo irreversible growth arrest due to various stress signals, particularly persistent DNA damage. Initially observed as a limit to cell proliferation in culture, cellular senescence has since been recognized as a phenomenon that occurs in vivo in response to stresses such as DNA damage, oxidative stress, and exposure to specific oncogenes. It serves as a protective mechanism to prevent the spread of damaged cells and potential malignant transformation. The induction of cellular senescence involves the activation of specific pathways controlled by molecules such as cyclin-dependent kinase inhibitors (CDKIs) like p16 and p21. These molecules are upregulated in response to DNA damage, stabilizing key proteins like p53 and retinoblastoma protein (pRB), ultimately leading to cell cycle arrest. Notably, mutations or inactivation of these pathways are common in human cancers. One significant aspect of cellular senescence is the development of the **"senescence-associated secretory phenotype (SASP)",** characterized by the secretion of various inflammatory cytokines, chemokines, growth factors, and proteases. SASP factors not only reinforce senescence in the cells themselves but also act in a paracrine manner, inducing senescence in neighboring cells, a process termed paracrine senescence. Moreover, SASP factors can recruit immune cells to remove senescent cells and contribute to tissue repair in damaged tissues. However, SASP also has deleterious effects, including aging-associated inflammation and promotion of cancer progression. Persistent SASP can lead to chronic inflammation and contribute to the development of various age-related diseases, including cancer, atherosclerosis, and osteoarthritis.

In another embodiment, CSF1R ligand modulates cellular senescence of PAM during amyloid pathology.

**Inflammation** is a vital component of the body's defense mechanism, playing a crucial role in recognizing and removing harmful stimuli while initiating the healing process. It can manifest as acute or chronic, each with distinct characteristics and durations. Acute inflammation typically occurs rapidly in response to tissue damage caused by trauma, microbial invasion, or exposure to toxic compounds. The classic signs of redness, swelling, heat, pain, and loss of function characterize it. Acute inflammation involves mobilizing immune cells, particularly neutrophils and macrophages, to the injury site, facilitated by cytokines and chemokines. This response is essential for eliminating the initial cause of injury and initiating tissue repair. Acute inflammation is a short-term process, usually resolving within a few days upon removal of the harmful stimulus. Chronic inflammation is a prolonged and sustained inflammatory response lasting for months or years. It involves different immune cells, such as macrophages, lymphocytes, and plasma cells, and is associated with diseases like diabetes, cardiovascular disease, allergies, and chronic obstructive pulmonary disease (COPD). Chronic inflammation can be caused by various factors, including obesity, smoking, stress, and poor diet. Common signs and symptoms of chronic inflammation include body pain, fatigue, mood disorders, gastrointestinal complications, and infection susceptibility.

In one embodiment, CSF1R ligand reduces pro inflammatory activation of PAM during amyloid pathology.

### Pathology and clinical symptoms

**Neurodegenerative diseases** encompass a group of disorders characterized by the progressive loss of neurons and their functions, ultimately leading to impaired cognitive and motor abilities. These diseases are marked by the accumulation of misfolded proteins in the brain, disruption of neuronal circuits, and cell death. Common pathological features include the formation of protein aggregates, such as amyloid-beta plaques and tau tangles in Alzheimer's disease, and Lewy bodies in Parkinson's disease. While the exact causes vary between diseases, genetic mutations, oxidative stress, inflammation, and impaired protein degradation pathways play significant roles in their development and progression.

### Pharmaceutical composition

As used herein, the terms "patient," "individual," and "subject" are often used interchangeably and refer to any animal that exhibits a symptom of a disease, disorder, or condition that can be treated with the colony stimulating factor 1 receptor (CSF1R) ligand and methods disclosed herein. In preferred embodiments, a subject includes any animal that exhibits symptoms of a disease, disorder, or condition associated with the central nervous system, e.g., (neuro) cognitive impairment, impaired motoric skills, loss of smell, difficulties with language, clinical symptoms of dementia, attention deficits, and/or impaired memory and/or neurodegenerative disease is Alzheimer's disease (AD), Lewy body dementia, Parkinson's disease (PD), multiple sclerosis (MS), motor neuron disease (MND), Huntington disease (HD), progressive supranuclear palsy (PSP), corticobasal degeneration (CBD), multiple system atrophy (MSA), or amyotrophic lateral sclerosis (ALS) that can be treated with methods disclosed herein. Suitable subjects include laboratory animals (such as mice, rats, rabbits, or guinea pigs), farm animals, and domestic animals or pets (such as cats or dogs). Non-human primates and, preferably, human patients are included.

As used herein, the terms "(medical) disease," "(medical) disorder," and "(medical) condition" are often used interchangeably.

As used herein, "treatment" or "treating" includes any beneficial or desirable effect on the symptoms or pathology of a disease or pathological condition and may include even minimal reductions in one or more measurable markers of the disease or condition being treated. Treatment can optionally involve either the reduction or amelioration of symptoms of the disease or condition or the delaying of the progression of the disease or condition. "Treatment" does not necessarily indicate complete eradication or cure of the disease, condition, or associated symptoms. The phrase "therapeutically effective" is intended to include, within the scope of sound medical judgment, excessive toxicity, irritation, and/or other problems or complications but commensurate with a reasonable benefit/risk ratio.

As used herein, "prevent" and similar words such as "prevented," "preventing," or "prophylactic," etc., indicate an approach for preventing, inhibiting, or reducing the likelihood of the occurrence or recurrence of a disease or condition. It also refers to delaying the onset or recurrence of a disease or condition or delaying the occurrence or recurrence of the symptoms of a disease or condition. As used herein, "prevention" and similar words also include reducing the intensity, effect, symptoms, and/or burden of a disease or condition prior to the onset or recurrence of the disease or condition.

The present invention relates to a colony stimulating factor 1 receptor (CSF1R) ligand for use in the treatment or prevention of a neurodegenerative disease in a subject.

In embodiments, the CSF1R ligand for use in the treatment or prevention of a neurodegenerative disease in a subject comprises administering an effective amount, e.g., a therapeutically effective amount of the CSF1R inhibitor and the bone marrow population contemplated herein. The quantity and frequency of administration will be determined by such factors as the condition of the patient and the type and severity of the patient's disease, although clinical trials may determine appropriate dosages.

### Administration

Pharmaceutical compositions comprising the CSF1R ligand for administration to a subject can include, in embodiments, at least one further pharmaceutically acceptable additive such as carriers, thickeners, diluents, buffers, preservatives, surface active agents, and the like in addition to the molecule of choice. Pharmaceutical compositions can also include one or more additional active ingredients such as antimicrobial agents, anti-inflammatory agents, anesthetics, and the like. The pharmaceutically acceptable carriers useful for these formulations are conventional. The person skilled in the art is aware of compositions and formulations suitable for pharmaceutical delivery of the CSF1R ligand disclosed herein. In general, the nature of the carrier will depend on the particular mode of administration being employed. For instance, parenteral formulations usually contain injectable fluids that include pharmaceutically and physiologically acceptable fluids such as water, physiological saline, balanced salt solutions, aqueous dextrose, glycerol, or the like as a vehicle. For solid compositions (for example, powder, pill, tablet, or capsule forms), conventional non-toxic solid carriers can include, for example, pharmaceutical grades of mannitol, lactose, starch, or magnesium stearate. In addition to biologically neutral carriers, pharmaceutical compositions to be administered can contain minor amounts of non-toxic auxiliary substances, such as wetting or emulsifying agents, preservatives, and pH buffering agents and the like, for example, sodium acetate or sorbitan monolaurate.

The CSF1R ligand can, in embodiments, be combined with pharmaceutically acceptable carrier substances as required to approximate physiological conditions, such as pH adjusting and buffering agents, tonicity adjusting agents, wetting agents, and the like, for example, sodium acetate, sodium lactate, sodium chloride, potassium chloride, calcium chloride, sorbitan monolaurate, and triethanolamine oleate. For solid compositions comprising the CSF1R ligand, conventional non-toxic pharmaceutically acceptable vehicles can be used, which include, for example, pharmaceutical grades of mannitol, lactose, starch, magnesium stearate, sodium saccharin, talcum, cellulose, glucose, sucrose, magnesium carbonate, and the like. Liquid pharmaceutical compositions, whether they are solutions, suspensions, or other like forms, may include one or more of the following: sterile diluents such as water for injection, saline solution, preferably physiological saline, Ringer's solution, isotonic sodium chloride, fixed oils such as synthetic mono or diglycerides which may serve as the solvent or suspending medium, polyethylene glycols, glycerin, propylene glycol or other solvents; antibacterial agents such as benzyl alcohol or methylparaben; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in ampoules, disposable syringes, or multiple dose vials made of glass or plastic. An injectable pharmaceutical composition is preferably sterile.

A CSF1R ligand comprised in a (pharmaceutical) composition can be administered to subjects by a variety of mucosal administration modes, including by oral, rectal, intraocular, intranasal, intrapulmonary, or transdermal delivery, intramuscular, intraocular, subcutaneous, intravenous, intraarterial, intra-articular, intraperitoneal, intrathecal, intracerebroventricular, or parenteral routes.

In accordance with the disclosure herein, a prophylactically or therapeutically effective amount of pharmaceutical combination may, in embodiments, be administered to a subject in need of such treatment for a time and under conditions sufficient to prevent, inhibit, and/or ameliorate a selected condition or one or more symptom(s) thereof, wherein the condition is caused by PAM at amyloid deposits.

The attending clinician can vary dosage to maintain a desired concentration at a target site (for example, the lungs or systemic circulation). Higher or lower concentrations can be selected based on the mode of delivery, for example, trans-epidermal, rectal, oral, pulmonary, or intranasal delivery versus intravenous or subcutaneous delivery. Dosage can also be adjusted based on the release rate of the administered formulation, for example, of an intrapulmonary spray versus powder, sustained release oral versus injected particulate or transdermal delivery formulations, and so forth.

The instant disclosure also includes kits, packages, and multi-container units containing the herein-described pharmaceutical combination or pharmaceutical compositions comprising the same and/or means for administering the same for use in the prevention and treatment of conditions described herein and other conditions in human subjects.

It will be understood that particular embodiments described herein are shown by way of illustration and not as limitations of the invention. The principal features of this invention can be employed in various embodiments without departing from the scope of the invention. Those skilled in the art will recognize or be able to ascertain, using most routine study, numerous equivalents to the specific procedures described herein. Such equivalents are considered to be within the scope of this invention and are covered by the claims. All publications and patent applications mentioned in the specification indicate the skill level of those skilled in the art to which this invention pertains. All publications and patent applications are herein incorporated by reference to the same extent as if each individual publication or patent application was specifically and individually indicated to be incorporated by reference.

The use of the word "a" or "an" when used in conjunction with the term "comprising" in the claims and/or the specification may mean "one," but it is also consistent with the meaning of "one or more," "at least one," and "one or more than one." The term "or" in the claims is used to mean "and/or" unless explicitly indicated to refer to alternatives only or the alternatives are mutually exclusive. However, the disclosure supports a definition of only alternatives and "and/or." Throughout this application, where relevant, the term "about" indicates that a value includes the inherent variation of error for the device, the method employed to determine the value or the variation among the study subjects.

### FIGURES

The invention is demonstrated by examples in the following figures. The figures are to provide a further description of potentially preferred embodiments that enhance the support of one or more non-limiting embodiments of the invention.

### Brief description of the figures:

**Figure 1****:** Plaque-associated microglia clonally expand at amyloid plaques.
**Figure 2****:** Tracing of PAM and non-PAM expansion dynamics during amyloid pathology in *Cx3cr1^{CreERT2}R26^{Confetti}5xFAD⁺* animals.
**Figure 3****:** Individual non-plaque-associated microglia give rise to clones of expanding plaque-associated microglia at the plaque sites.
**Figure 4****:** Microglial clonality around amyloid plaques is modulated by peripheral stimuli, gut microbiota and disease stage.
**Figure 5****:** Minor modulation of plaque load by peripheral stimuli and gut microbiota at early and late stage of amyloid pathology.
**Figure 6****:** Transcriptional plasticity of non-PAM towards peripheral stimuli and gut dysbiosis is age-dependent.
**Figure 7****:** Non-plaque-associated microglia show transcriptional plasticity towards peripheral stimuli and gut dysbiosis at early stages of amyloid pathology.
**Figure 8****:** Quality control of chromatin accessibility data and gene ontology analysis.
**Figure 9****:** Distinct chromatin accessibility landscapes in microglial subsets depending on their spatial relationship to pathology.
**Figure 10****:** Csf1 treatment reduces the number of PAM and elevates phagocytosis.
**Figure 11****:** Engagement of Csf1r by Csf1 mitigates clonal expansion of PAM, elevates phagocytic capacity of PAM and attenuates amyloid pathology at early stage of disease
**Figure 12****:** Csf1 treatment beneficially modulates functional and metabolic features of non-PAM-derived PAM making these cells competent to restrict amyloid pathology at early stages of neurodegeneration.

### Detailed description of the figures:

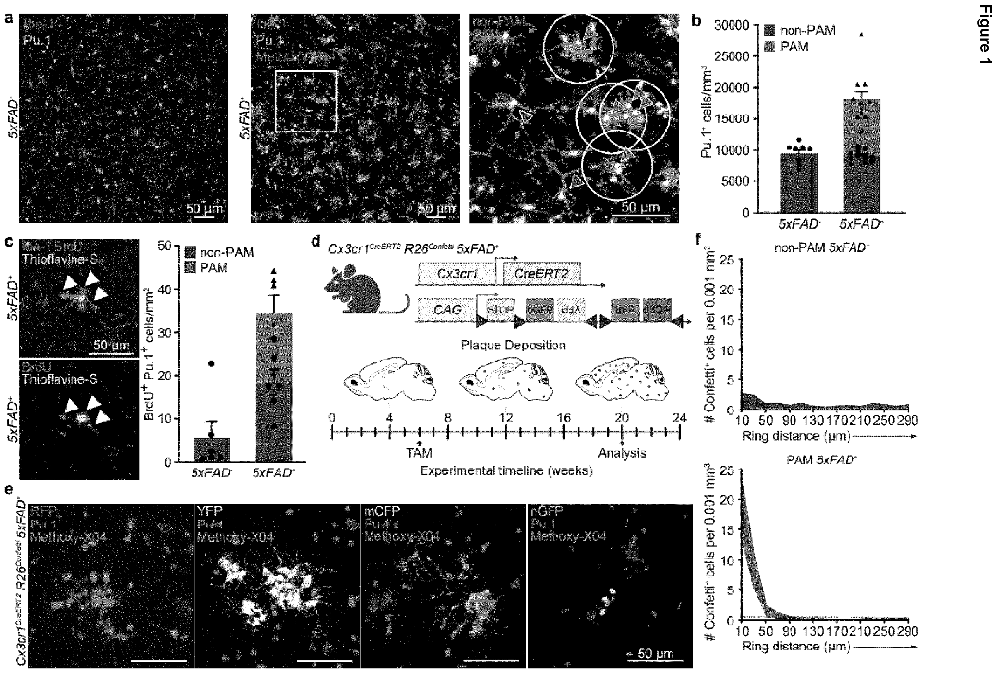

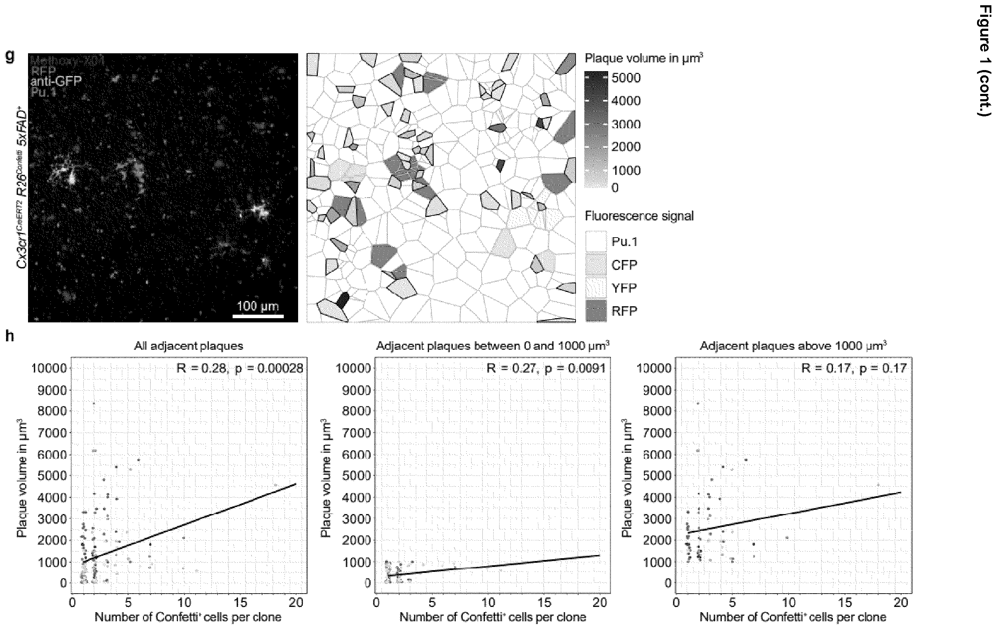

**Figure 1****: Plaque-associated microglia clonally expand at amyloid plaques.**
**(a)** Representative confocal images from frontal cortices of 44-week-old *5xFAD⁻* (left) and *5xFAD⁺* animals (center, right). Immunofluorescence for Iba-1 (microglia, magenta), Methoxy-X04 (amyloid beta, blue) and nuclear Pu.1 (microglia, cyan). Zoom (right) depicts exemplars of Pu.1⁺Iba-1⁺ plaque-associated microglia (PAM, red arrowheads) and Pu.1⁺Iba-1⁺ non-plaque-associated microglia (non-PAM, blue arrowheads). White circles indicate 30 µm radius rings around amyloid plaques, within which only cells with direct contact to plaques were defined as PAM. Scale bar = 50 µm.
**(b)** Quantification of Pu.1⁺ microglia per mm³ separated in PAM (red bar, individual mice represented by triangles) and non-PAM (blue bars, individual mice represented by circles) in frontal cortices of 20-week-old *5xFAD*⁺animals and negative (*5xFAD⁻)* littermates (n = 8 - 11). Mean ± s.e.m is shown.
**(c)** Left panels: Representative fluorescent images of a 20-week-old *5xFAD⁺* animal treated with BrdU. Example of BrdU incorporation (red) in Iba-1⁺ microglia (magenta) in contact with a Thioflavine-S⁺ amyloid plaque (cyan) is shown. Arrowheads indicate BrdU⁺ Iba-1⁺ PAM. Scale bar = 50 µm. Right panel: Quantification of BrdU⁺ Pu.1⁺ PAM (red bar, individual mice represented by triangles) and non-PAM (blue bars, individual mice represented by circles) per mm² in frontal cortices of 20-week-old *5xFAD⁺* animals and negative *(5xFAD⁻)* littermates (n = 6). Mean ± s.e.m is shown.
**(d)** Experimental scheme of *Cx3cr1^{CreERT2}R26^{Confetti}5xFAD⁺* animals.
**(e)** Representative confocal images from the frontal cortices of *Cx3cr1^{CreERT2}R26^{Confetti}5xFAD⁺* animals. Confetti⁺ microglia clones were found to be present at the plaque site as nuclear GFP (nGFP, green), cytoplasmic YFP (yellow), RFP (red) or membrane-tagged CFP (mCFP, cyan). Immunofluorescence of nuclear Pu.1 (microglia, magenta) and Methoxy-X04 (amyloid beta, blue) are also shown. Scale bars = 50 µm.
**(f)** Monte Carlo simulation: Microglial densities of Confetti⁺ non-PAM (blue, upper panel) and PAM (red, lower panel) of 20-week-old *Cx3cr1^{CreERT2}R26^{Confetti}5xFAD⁺* animals (n = 11) shown relative to randomized datasets (purple). Densities are displayed relative to measured distances (i.e. ring distance; µm) between Confetti⁺ cells. Means per group and 98 % confidence intervals are shown for experimental datasets and randomized datasets.
**(g)** Left panel: Representative image of Confetti⁺ PAM clones at plaque sites in 16-week-old *Cx3cr1^{CreERT2}R26^{Confetti}5xFAD⁺* animals. Immunofluorescence for anti-GFP (green), anti-RFP (red), Methoxy-X04 (amyloid beta; red) and Pu.1 (microglia, magenta) is shown. Scale bar = 100 µm. Right panel: Representative Voronoi grid visualizing plaque sizes and the positioning of Confetti- and Confetti⁺ Pu.1⁺ microglial cells from the representative image shown. Confetti⁻Pu.1⁺ cells are shown in white, CFP⁺Pu.1⁺ cells in cyan, YFP⁺ Pu.1⁺ cells in yellow and RFP⁺ Pu.1⁺ cells in red. Volume of Methoxy-X04⁺ plaques is color-coded as indicated in legend.
**(h)** Left panel: Correlation between Confetti⁺ microglia clone size and all adjacent Methoxy-X04⁺ plaques of all sizes. Colors of individual points represent separately analyzed images (technical replicates, N = 17) across multiple biological replicates (n = 4). Black line indicates linear regression. R = 0.28. *** *p = 0.00028.* Center panel: Correlation between Confetti⁺ microglia clone size and maximal 1000 µm³ large Methoxy-X04⁺ plaques. Colors of individual points represent separately analyzed images (technical replicates, N = 17) across biological replicates (n = 4). Black line shows linear regression. R = 0.27. ** *p = 0.0091.* Right panel: Relationship between Confetti⁺ microglia clone size and Methoxy-X04⁺ plaques above 1000 µm³. Colors of individual points represent separately analyzed images (technical replicates; N = 17) from biological replicates (n = 4). Black line shows linear regression. R = 0.17. ns p = *0.17.*

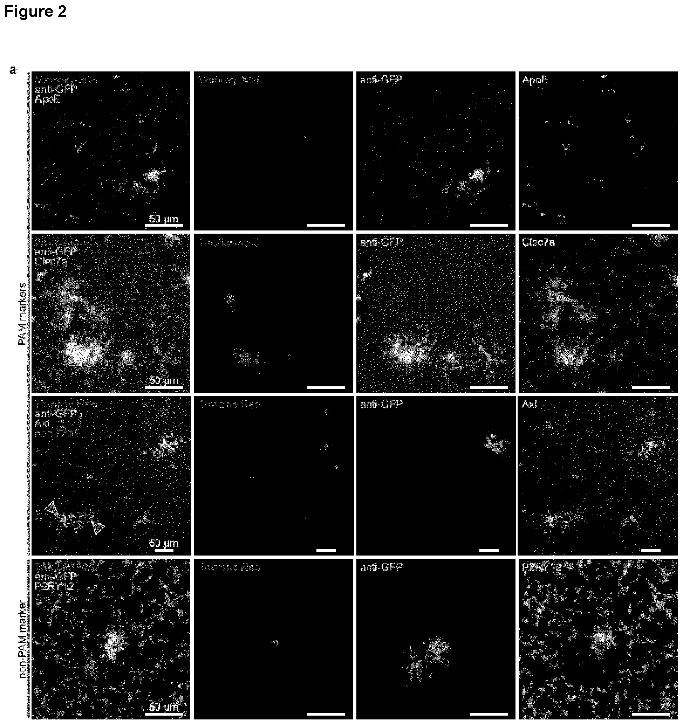

**Figure 2****: Tracing of PAM and non-PAM expansion dynamics during amyloid pathology in *Cx3cr1^{CreERT2}R26^{Confetti}5xFAD⁺* animals.**
(a) Illustrative confocal images of Confetti⁺ microglia in 20-week-old *Cx3cr1^{CreERT2}R26^{Confetti}5xFAD⁺* animals. Immunofluorescence for anti-GFP (Confetti⁺, green), and thioflavine-S/Thiazine Red/Methoxy-X04 (blue) are depicted together with the following markers (cyan): upper three rows: ApoE, Clec7a (higher magnification and individual channels from Figure 2) and Axl; lower row: P2RY12 (higher magnification and individual channels from Figure 1). Arrowheads indicate non-PAM labelled by Axl. Scale bars = 50 µm.

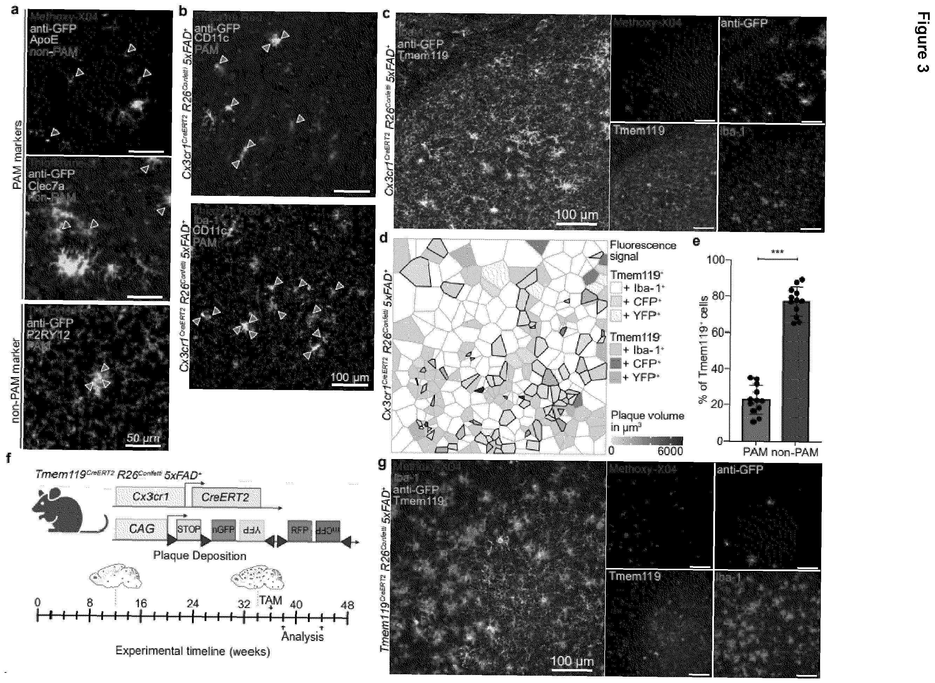

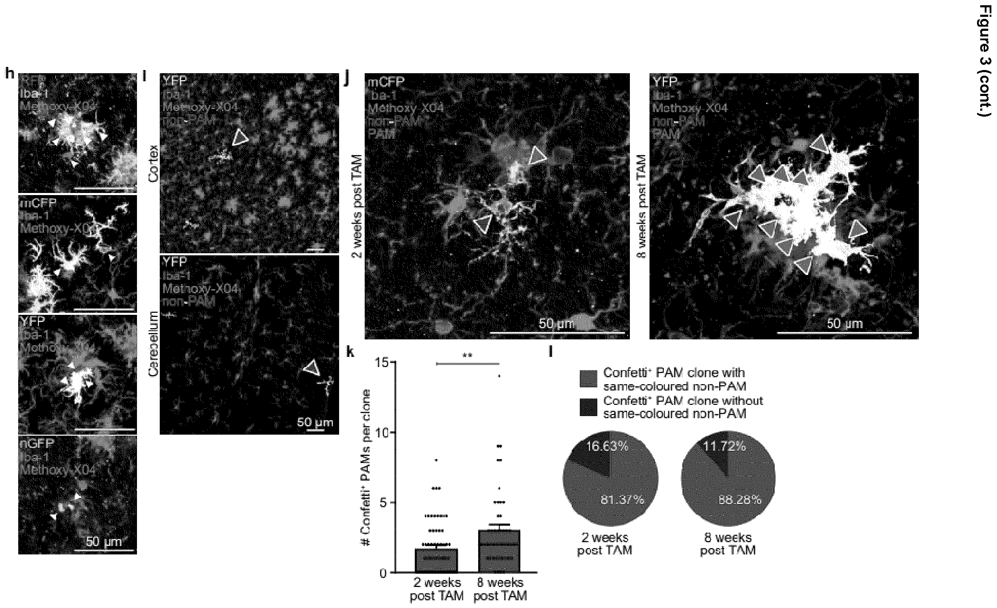

**Figure 3****: Individual non-plaque-associated microglia give rise to clones of expanding plaque-associated microglia** at **the plaque** sites.
**(a)** Typical confocal images of Confetti⁺ microglia in 20-week-old *Cx3cr1^{CreERT2}R26^{Confetti}5xFAD⁺* individuals. Immunofluorescence for anti-GFP (Confetti⁺, green), and thioflavine-S/thiazine red/Methoxy-X04 (amyloid beta; blue) are shown alongside the following markers (cyan): upper two panels: PAM markers ApoE and Clec7a; lower panel: non-PAM marker P2RY12. Blue arrowheads indicate non-PAM and red arrowheads highlight PAM. Scale bars = 50 µm.
**(b)** Upper panel: Representative immunofluorescence for anti-GFP (Confetti⁺, green), and thiazine red (blue) together with CD11c (cyan). Lower panel: Confocal images of Iba-1⁺CD11c⁺ PAM restricted to amyloid plaques in 20-week-old *Cx3cr1^{CreERT2}R26^{Confetti}5xFAD⁺* animals. Thiazine red is shown in blue, Iba-1 (microglia) is shown in red and CD11c is displayed in cyan. Red arrowheads indicate CD11c⁺ PAM. Scale bars = 100 µm.
**(c)** Left panel: Illustrative confocal image of Confetti⁺ microglia in 20-week-old *Cx3cr1^{CreERT2}R26^{Confetti}5xFAD⁺* animals. Immunofluorescence for lba-1 (red), anti-GFP (Confetti⁺, green), and Methoxy-X04 (blue) are presented together with Tmem119 (cyan). Scale bars = 100 µm. Right panels: Individual channels are shown.
**(d)** Voronoi grid visualizing plaque sizes and the positioning of Confetti- and Confetti⁺ Tmem119⁺Iba-1⁺ microglia (non-PAM) and Confetti- and Confetti⁺Tmem119⁻ Iba-1⁺ microglia (PAM) in 20-week-old *Cx3cr1^{CreERT2}R26^{Confetti}5xFAD⁺* mice. Tmem119⁺ Iba-1⁺ microglia are shown in white, CFP⁺ Tmem119⁺ Iba-1⁺ microglia in cyan, YFP⁺ Tmem119⁺ Iba-1⁺ microglia in yellow, Tmem119⁻Iba-1⁺ microglia in grey, CFP⁺ Tmem119- Iba-1⁺ microglia in green and YFP⁺ Tmem119⁻Iba-1⁺ microglia in orange. Volume of Methoxy-X04⁺ plaques is color-coded as indicated in legend.
**(e)** Quantification of Tmem119⁺ PAM (red) and non-PAM (blue) from 20-week-old *Cx3cr1^{CreERT2}R26^{Confetti}5xFAD⁺* individuals (n = 12). Dots represent individual animals. Mean ± s.e.m. is shown. **** p <0.001.*
**(f)** Experimental scheme of *Tmem119^{CreERT2}R26^{Confetti}5xFAD⁺* mice.
**(g)** Characteristic confocal images from frontal cortices in 44-week-old adult *Tmem119^{CreERT2}R26^{Confetti}5xFAD⁺* animals 8 weeks after tamoxifen (TAM) application. Confetti labelling was observed in both Tmem119⁺Iba-1⁺non-PAM and Tmem119⁻ Iba-1⁺PAM. Images are shown for Methoxy-X04 (blue), anti-GFP (Confetti⁺, green), Tmem119 (cyan) and Iba-1 (red). Scale bars = 100 µm.
**(h)** Characteristic confocal images of Confetti⁺ Iba-1⁺ microglia (arrowheads) clones at amyloid plaques in 44-week-old *Tmem119^{CreERT2}R26^{Confetti}5xFAD⁺* animals 8 weeks after TAM application. Clones are found in all Confetti colors: RFP (red), mCFP (cyan), YFP (yellow), and nGFP (green). Immunofluorescence for Iba-1 (cyan (uppermost panel) and magenta (lower panels), and Methoxy-X04 (amyloid beta, blue) is shown. Scale bars = 50 µm.
**(i)** Typical confocal pictures of single labelled Confetti⁺ Iba-1⁺ non-PAM (arrowheads) in the cortex (upper panel) and cerebellum (lower panel) of 44-week-old *Tmem119^{CreET2R}R26^{Confetti}5xFAD⁺* animals 8 weeks after TAM application. Immunofluorescence for Iba-1 (magenta), Methoxy-X04 (blue), and YFP (yellow). Scale bars = 50 µm.
**(j)** Representative confocal images from frontal cortices in adult *Tmem119^{CreERT2}R26^{Confetti}5xFAD⁺* animals 2 weeks (left panel) or 8 weeks (right panel) after tamoxifen (TAM) application. Confetti labelling was observed in both Iba-1⁺ (magenta) non-PAM (blue arrowheads) and Iba-1⁺ PAM (red arrowheads). Immunofluorescence for Methoxy-X04 (amyloid beta, blue), YFP (yellow) and mCFP (cyan) is shown. Scale bars = 50 µm.
**(k)** Quantification of same-colored Confetti⁺ PAM per clone in *Tmem119^{CreER}R26R^{Confetti}5xFAD⁺* animals 2 weeks and 8 weeks after TAM application. Mean ± s.e.m. is shown. Symbols represent individual clones. Colors of individual points indicate separately analyzed images (n = 21 - 25) from biological replicates (n = 2).
**(l)** Quantification of the percentage of same-colored Confetti⁺ PAM clones that were either associated (red) or not associated (dark red) with an individual same-colored Confetti⁺ non-PAM. Pie charts represent distribution at 2 weeks (left) and 8 weeks (right) after TAM injection.

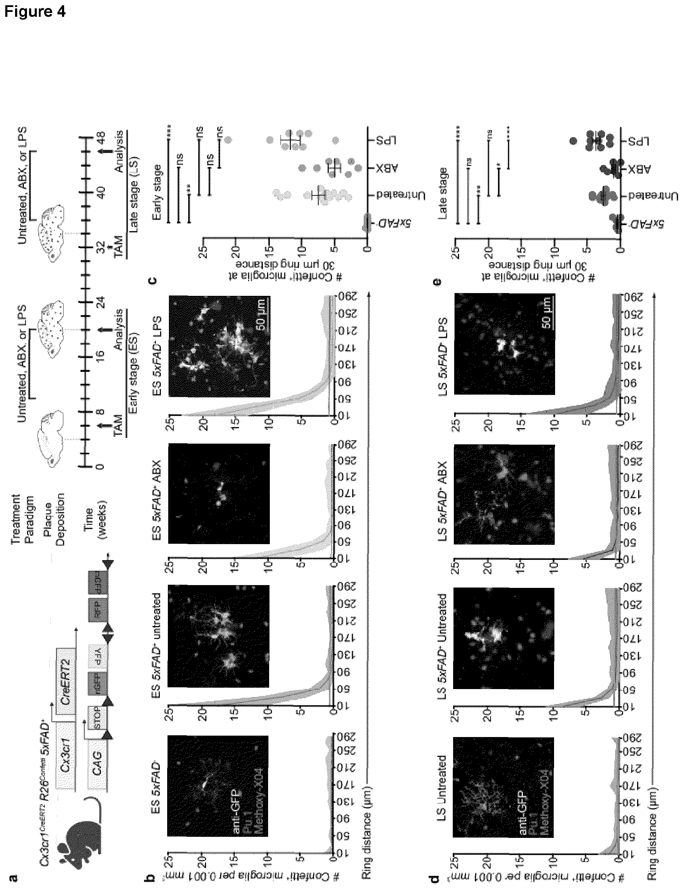

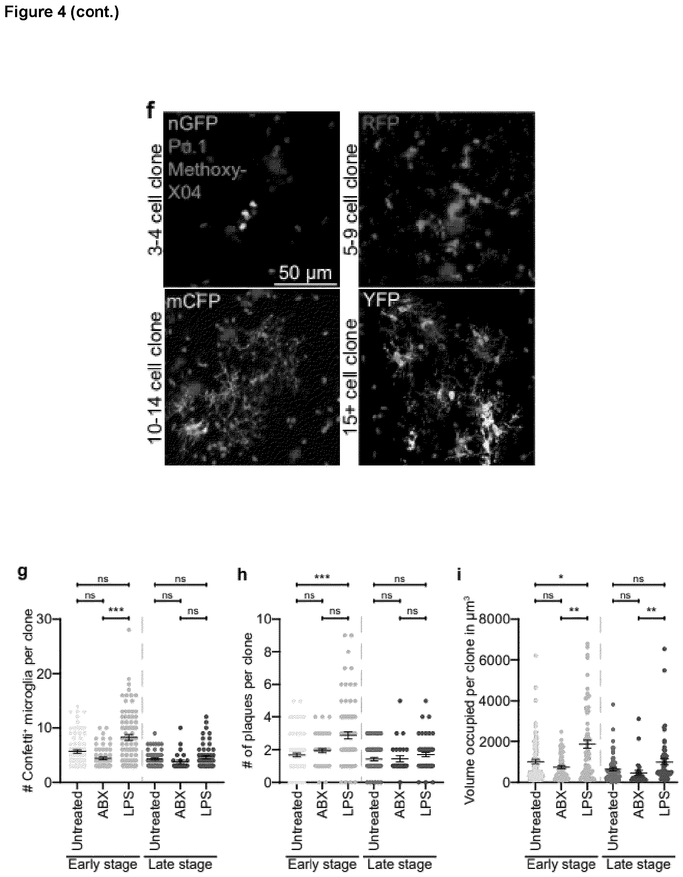

**Figure 4****: Microglial clonality around amyloid plaques is modulated by peripheral stimuli, gut microbiota and disease stage.**
**(a)** Experimental scheme for treatment paradigms in *Cx3cr1^{CreERT2}R26^{Confetti}5xFAD⁺* animals and controls. ABX: antibiotics, LPS: lipopolysaccharides, ES: early stage of disease, LS: late stage of disease, TAM: Tamoxifen.
**(b)** Monte Carlo simulation: Densities of Confetti⁺ microglia in untreated *Cx3cr1C^{reERT2}R26^{Confetti}5xFAD⁻*control littermates (pink, n = 8) and *Cx3cr1^{CreERT2}R26^{Confetti}5xFAD⁺* animals that were either untreated (green, n = 13), or systemically treated with ABX (blue, n = 8) or LPS (orange, n = 9) at ES, relative to randomized datasets (purple). Densities are displayed relative to measured distances (i.e. ring distance; µm) between Confetti⁺ cells. Means per group and 98 % confidence intervals are shown for experimental datasets and randomized datasets. Representative confocal images of anti-GFP labeled Confetti⁺ (yellow) Pu.1⁺ (magenta) microglia in relation to Methoxy-X04 (blue) are shown for each group. Scale bar = 50 µm.
**(c)** Quantification of Confetti⁺ cells located in a 30 µm ring distances. Each symbol represents one animal. Mean ± s.e.m. is shown. ***p < 0.01, ***p < 0.001,* ns: not significant.
**(d)** Monte Carlo simulation: Densities of Confetti⁺ microglia in untreated *Cx3cr1CreE^{RT2}R26^{Confetti}5xFAD⁻*control littermates (dark pink, n = 6) and *Cx3cr1^{CreERT2}R26^{Confetti}5xFAD⁺* animals that were either untreated (dark green, n = 11), or systemically treated with ABX (dark blue, n = 8) or LPS (red, n = 9) at LS, relative to randomized datasets (purple). Densities are displayed relative to measured distances (i.e. ring distance; µm) between Confetti⁺ cells. Means per group and 98 % confidence intervals are shown for experimental datasets and randomized datasets. Representative confocal images of anti-GFP labeled Confetti⁺ (yellow) Pu.1⁺ (magenta) microglia in relation to Methoxy-X04 (amyloid beta, blue) are shown for each group. Scale bar = 50 µm.
**(e)** Quantification of Confetti⁺ cells located in a 30 µm ring distance. Each symbol represents one animal. Mean ± s.e.m. is shown. Each symbol represents one animal. Mean ± s.e.m. is shown. *p < *0.05, **p < 0.01, ***p < 0.001.*
**(f)** Representative confocal images representing a range of same-colored Confetti⁺ clone sizes of Pu.1⁺ (magenta) microglia in *Cx3cr1^{CreERT2}R26^{Confetti}5xFAD⁺* animals: 3 - 4 nGFP⁺ (green) cells; 5 - 9 RFP⁺ (red) cells; 10 - 14 mCFP⁺ (cyan) cells; and 15 or more YFP⁺ (yellow) cells. Methoxy-X04 (blue) is shown for amyloid plaques. Scale bar = 50 µm.
**(g)** Quantification of Confetti⁺ microglial cells per clone. Each dot represents one clone. N = 27 - 87 clones per group were analyzed for each group across all animals. Mean ± s.e.m. is shown. ***p *< 0.001,* ns: not significant.
**(h)** Quantification of the number of associated plaques per Confetti⁺ clone. Each dot represents one clone. N = 27 - 87 clones per group were analyzed for each group across all animals. Mean ± s.e.m. is shown. ****p < 0.001,* ns: not significant.
**(i)** Quantification of volume occupied per clone in all groups analyzed. Each dot represents one clone. N = 27 -87 clones per group were analyzed for each group across all animals. Mean ± s.e.m. is shown. *p < *0.05, **p* < *0.01.*

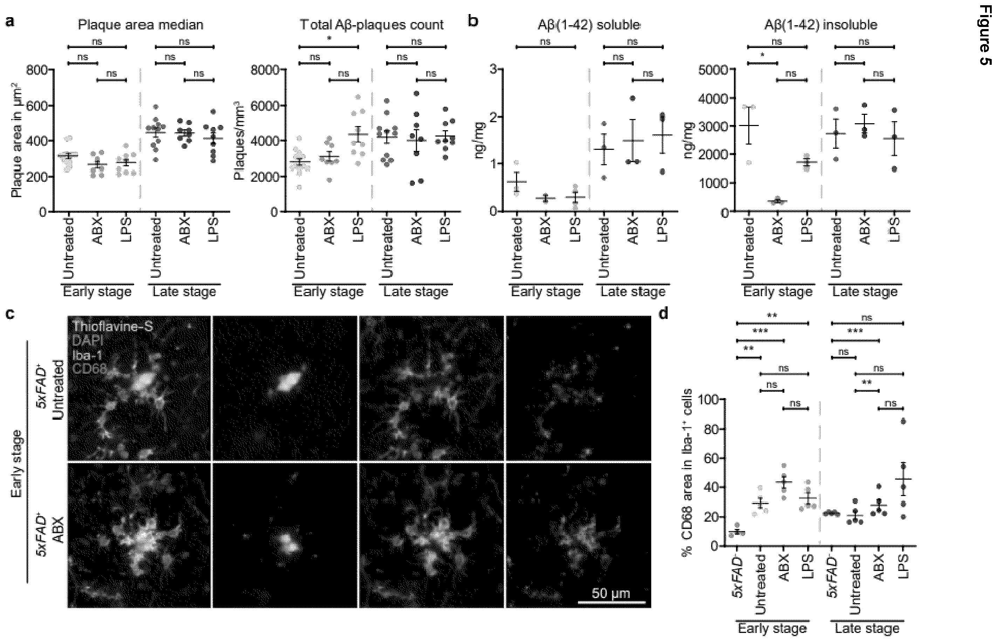

**Figure 5****: Minor modulation of plaque load by peripheral stimuli and gut microbiota at early and late stage of amyloid pathology.**
**(a)** Quantification of number of plaques (left) and quantification of individual plaque sizes (right) in the frontal cortices of untreated, ABX-, and LPS-treated *Cx3cr1^{CreERT2}R26^{Confetti}5xFAD⁺* mice at an early and late stage of disease. Left: Each symbol represents one animal, n = 8 - 11 per group. Mean ± s.e.m. is shown. *p < *0.05,* ns: not significant. Right: Each symbol represents one analyzed plaque; n = 1,838 - 2,972 plaques per group, n = 8 - 11 animals per group. Median ± interquartile range is shown. ns: not significant.
**(b)** ELISA of human Aβ(1-42) peptides in soluble (left panel) and insoluble (right panel) fractions of cortices from untreated, ABX- and LPS-treated *Cx3cr1 ^{CreERT2}R26^{Confetti}5xFAD⁺* animals at an early and late stage of disease. Each symbol represents one animal, n = 2 - 4 per group. Mean ± s.e.m. is shown. *p < *0.05,* ns: not significant.
**(c)** Representative images of CD68⁺ PAM in untreated (upper panel) or ABX-treated (lower panel) *Cx3cr1^{CreERT2}R26^{Confetti}5xFAD⁺* animals at an early stage of disease. Immunofluorescence for CD68 (magenta), Thioflavine-S (amyloid beta, cyan), Iba-1 (microglia, green) and DAPI (blue) is shown. Scale bar = 50 µm.
**(d)** Quantification of the percentage of CD68⁺ area in Iba-1⁺ microglial cell bodies of untreated, ABX- and LPS-treated *Cx3cr1^{CreERT2}R26^{Confetti}5xFAD⁺* animals at an early and late stage of pathology compared to untreated control littermates. Each symbol represents one animal, n = 4 - 5 per group. Mean ± s.e.m. is shown. **p < *0.01, ***p* < *0.001.*

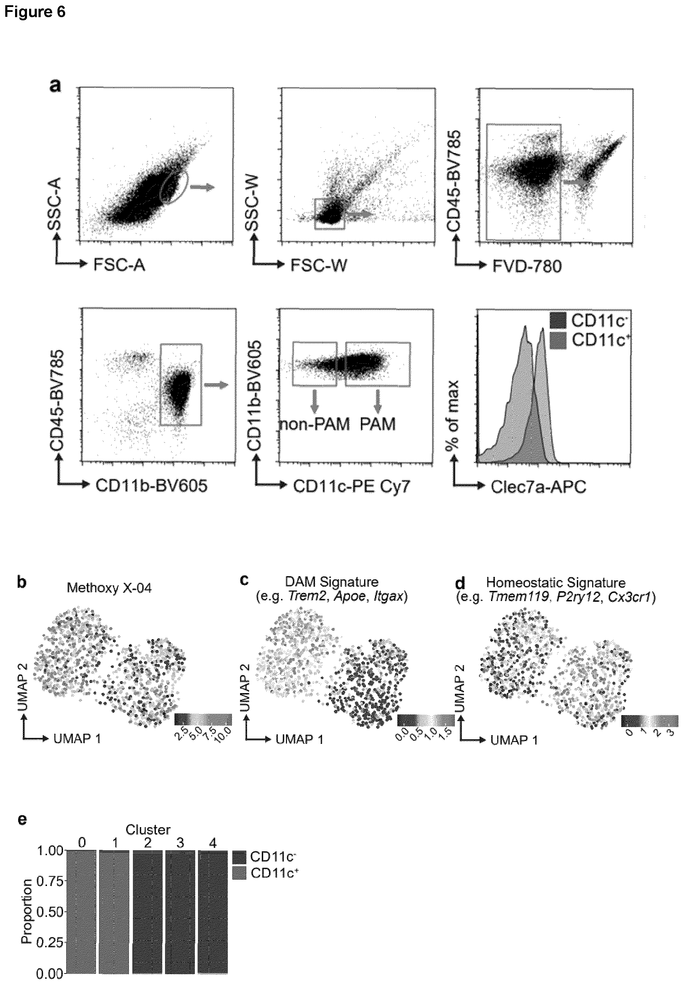

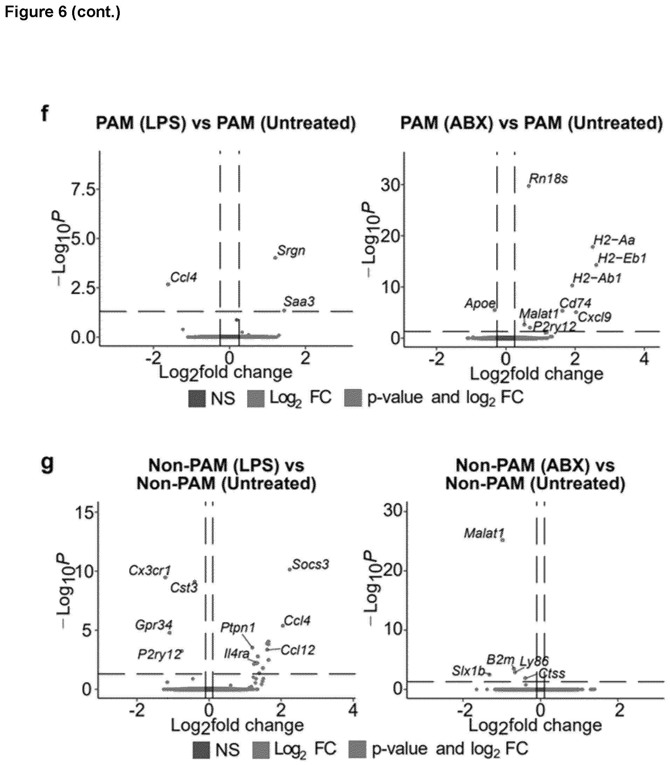

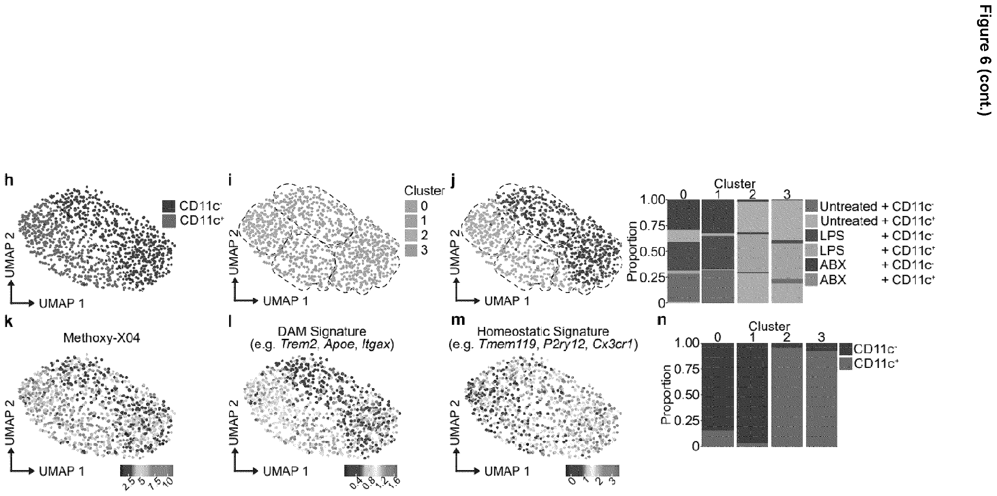

**Figure 6****: Transcriptional plasticity of non-PAM towards peripheral stimuli and gut dysbiosis is age dependent.**
**(a)** FACS sorting strategy of non-PAM and PAM from *5xFAD⁺* animals at an early and late stage of pathology for subsequent scRNA-seq and ATAC-seq. SSC: side scatter, FSC: forward scatter. Histogram for Clec7a expression is shown on sorted microglia populations, CD11c⁺ microglia are shown in red and CD11c- microglia in blue.
**(b)** UMAP feature plot depicting Methoxy-X04 levels of index-sorted non-PAM and PAM at an early stage of pathology. Scale refers to fluorescence intensity. Clusters and experimental conditions are consistent with Figure 4.
**(c)** UMAP plot depicting the expression of genes associated with microglial activation (Apoe, *Axl, Bhlhe40, Clec7a, Csf1, Cst7, Ctsb, Ctsd, Ctsl, Cybb, Fabp5, Fth1, Itgax, Gnas, Gpnmb, Grn, Il1b, Lgals3, Lilrb4, Lpl, Lyz2, Msr1, Nos2, Spp1, Tfec, Trem2, Tyrobp, Vegfa)* at an early stage of pathology. Scale refers to transcript counts of DAM core signature.
**(d)** UMAP presentation showing microglial genes associated with a homeostasis (The following genes were included in the signature: *P2ry12, Csf1r, Cx3cr1, Tmem119, Pu.1, Sall1*) at an early stage of pathology. Scale refers to transcript counts of homeostatic core signature.
**(e)** Stacked bar plot depicting the relative composition of microglial clusters (C0-C4) in respect to their CD11c signal: CD11c⁻ (blue) and CD11c⁺ cells (red).
**(f)** Volcano plots with pseudo bulk gene expression comparing differential gene expression between PAM from LPS-treated animals and untreated animals (left panel), and between PAM from ABX-treated animals and untreated animals (right panel) at late stage of disease. The -Log10-transformed adjusted P value (P adjusted, y-axis) is plotted against the Log2-transformed fold change (FC) in expression between the indicated groups (x-axis). Genes under Log2 FC and -Log10 P value cut off are shown in grey (NS). Genes above Log2 FC but under P value cut off are shown in green and genes above Log2 FC and -Log10 P value cut off are shown in red.
**(g)** Volcano plots with pseudobulk gene expression comparing differential gene expression between non-PAM from LPS-treated and untreated animals (left panel), and between non-PAM from ABX-treated animals and untreated animals (right panel) at late stage of disease. The -Log10-transformed adjusted P value (P adjusted, y-axis) is plotted against the Log2-transformed fold change (FC) in expression between the indicated groups (x-axis). Genes under Log2 FC and -Log10 P value cut off are shown in grey (NS). Genes above Log2 FC but under P value cut off are shown in green and genes above Log2 FC and -Log10 P value cut off are shown in red.
**(h)** UMAP plot demonstrating the distribution of analyzed CD11c⁻ (blue) and CD11c⁺ (red) microglia from untreated, LPS- and ABX-treated *Cx3cr1^{CreERT2}R26^{Confetti}5xFAD⁺* animals at a late stage of pathology. Each symbol represents one cell (n = 1,017 cells).
**(i)** UMAP plot visualizing the distribution of cell clusters (C0-C3) identified by unsupervised clustering of all analyzed cells from untreated, LPS- and ABX-treated *Cx3cr1^{CreERT2}R26^{Confetti} 5xFAD⁺* animals at a late stage of pathology. Each color represents one distinct cell cluster. Each cluster is outlined by dotted lines. Each symbol represents one cell (n = 1,017 cells).
**(j)** Left: UMAP plot visualizing the distribution of CD11c- and CD11c⁺ cells from untreated, LPS- and ABX-treated *Cx3cr1^{CreERT2}R26^{Confetti}5xFAD⁺* animals at a late stage of pathology relative to the identified cell clusters (C0-C3, dotted outlines) (see color legend, right). Right: Stacked bar plot depicting the relative composition of microglial clusters in respect to the cells' CD11c signal and their treatment group (see color legend).
**(k)** UMAP feature plot depicting Methoxy-X04 levels of index-sorted non-PAM and PAM at a late stage of pathology. Scale refers to fluorescence intensity. Clusters and experimental conditions are consistent with Figure 4.
**(l)** UMAP plot displaying microglial activation genes (Apoe, *Axl, Bhlhe40, Clec7a, Csf1, Cst7, Ctsb, Ctsd, Ctsl, Cybb, Fabp5, Fth1, Itgax, Gnas, Gpnmb, Grn, Il1b, Lgals3, Lilrb4, Lpl, Lyz2, Msr1, Nos2, Spp1, Tfec, Trem2, Tyrobp, Vegfa)* at a late stage of pathology. Scale refers to transcript counts of DAM core signature.
**(m)** UMAP feature plot depicting the expression of homeostatic microglial genes *(P2ry12, Csf1r, Cx3cr1, Tmem119, Pu.1, Sall1)* at a late stage of pathology. Scale refers to transcript counts of homeostatic core signature.
**(n)** Stacked bar plot depicting the relative composition of microglial clusters (C0-C3) at a late stage of pathology in respect to their CD11c signal: CD11c⁻ (blue) and CD11c⁺ cells (red).

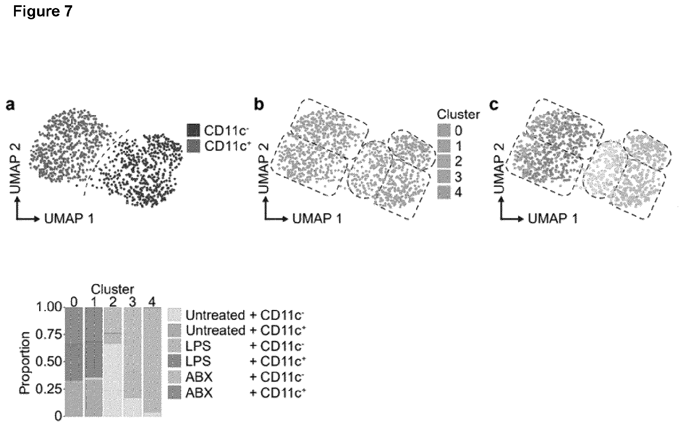

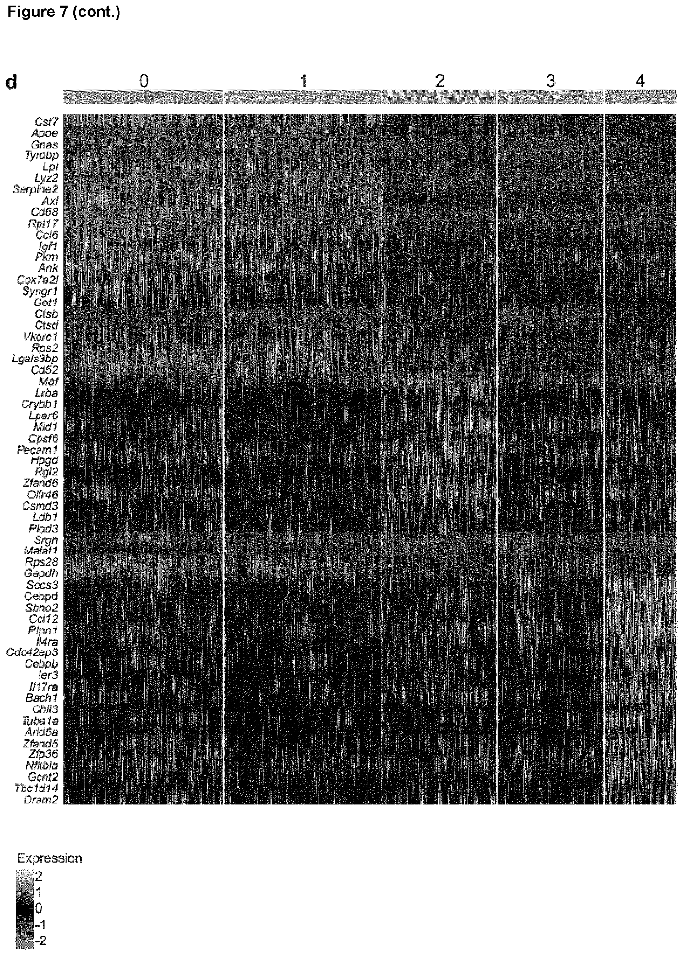

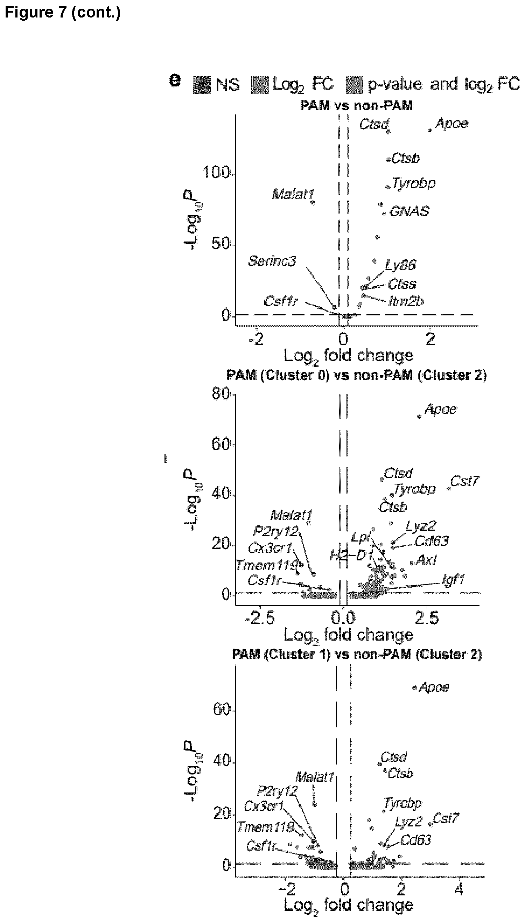

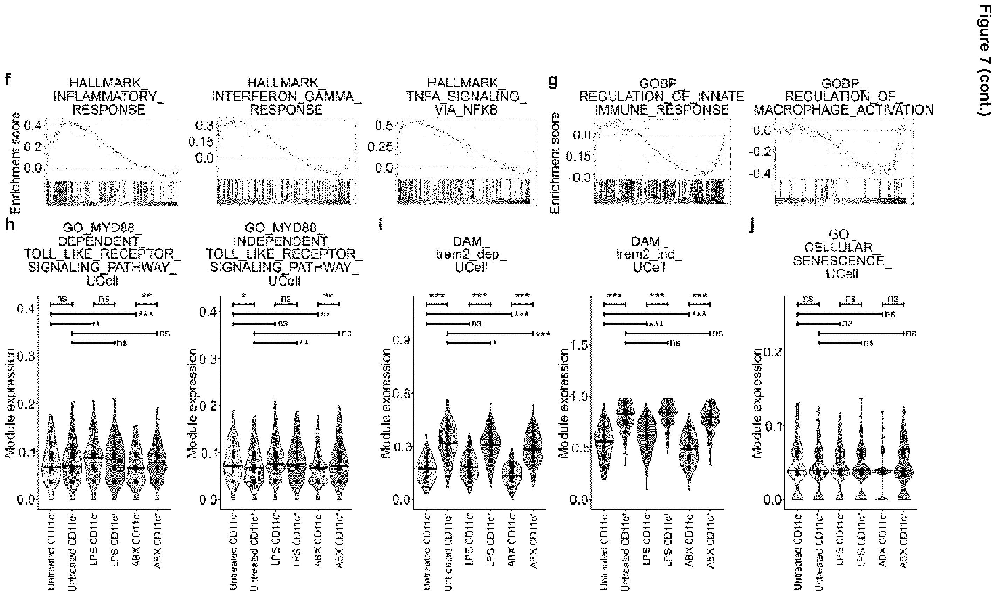

**Figure 7****: Non-plaque-associated microglia show transcriptional plasticity towards peripheral stimuli and gut dysbiosis at early stages of amyloid pathology.**
**(a)** UMAP plot demonstrating the distribution of analyzed CD11c- non-PAM (blue) and CD11c⁺ PAM (red) from untreated, LPS- and ABX-treated *Cx3cr1^{CreERT2}R26^{Confetti}5xFAD⁺* animals at an early stage of disease. Each dot represents one cell (n = 1,095).
**(b)** UMAP plot visualizing the distribution of cell clusters (C0-C4) identified by unsupervised clustering of all analyzed cells from untreated, LPS- and ABX-treated *Cx3cr1^{CreERT2}R26^{Confetti}5xFAD⁺* animals at an early stage of disease. Each color represents one distinct cell cluster; each cluster is outlined by dotted lines. Each dot represents one cell (n = 1,095).
**(c)** Left: UMAP plot visualizing the distribution of CD11c⁻ and CD11c⁺ cells from untreated, LPS- and ABX-treated *Cx3cr1^{CreERT2}R26^{Confetti}5xFAD⁺* animals at an early stage of disease relative to the identified cell clusters (C0-C4, dotted outlines) (see color legend, right). Right: Stacked bar plot depicting the relative composition of microglial clusters in respect to the cells' CD11c signal and their treatment group (see color legend).
**(d)** Heatmap presenting normalized expression of the 20 most differentially expressed (DE) genes per cluster. Expression levels are encoded by color as shown in the color legend.
**(e)** Volcano plots with pseudo bulk gene expression comparing differential gene expression between all analyzed PAM and non-PAM (upper panel), between PAM cluster 0 and non-PAM cluster 2 (middle panel), and between PAM cluster 1 and non-PAM cluster 2 (lower panel). The -Log10-transformed adjusted P value (P adjusted, y-axis) is plotted against the Log2-transformed fold change (FC) in expression between the indicated cell groups or clusters (x-axis). Genes under Log2 FC and -Log10 P value cut off are shown in grey (NS). Genes above Log2 FC but under P value cut off are shown in green and genes above Log2 FC and -Log10 P value cut off are shown in red.
**(f)** Gene set enrichment analysis (GSEA) of the pseudobulk differential gene expression (DGE) in non-PAM versus non-PAM after LPS treatment. HALLMARK_INFLAMMATORY_RESPONSE: Normalized enrichment score (NES) = 2, *p* = 0.0, False discovery rate (FDR) q = 0.0. HALLMARK_INTERFERON_GAMMA_RESPONSE: NES = 1.62, *p* = 0.0, FDR q = 0.002. HALLMARK_TNFA_SIGNALING_VIA_NFKB: NES = 2.66, *p* = 0.0, FDR q = 0.0. Enrichment scores and gene hits are plotted.
**(g)** Gene set enrichment analysis (GSEA) of the pseudobulk DGE in non-PAM compared to non-PAM following ABX treatment. *GOBP_REGULATION_OF_INNATE_IMMUNE_RESPONSE*: NES = -1.51, *p* = 0.002, FDR q = 0.13. *GOBP_REGULATION_OF_MACROPHAGE_ACTIVATION*: NES = -1.34, *p* = 0.1, FDR q = 0.15. Enrichment scores and gene hits are plotted.
**(h-i)** Violin plots depicting the expression of the indicated gene ontology modules. Typical LPS-associated MyD88-dependent and the MyD88-independent signaling pathway were compared between PAM and non-PAM. Similarly, cellular senescence was explored. The module expression was quantified using the UCell algorithm. The indicated p-values represent the results of pairwise two-tailed Mann-Whitney U-tests.

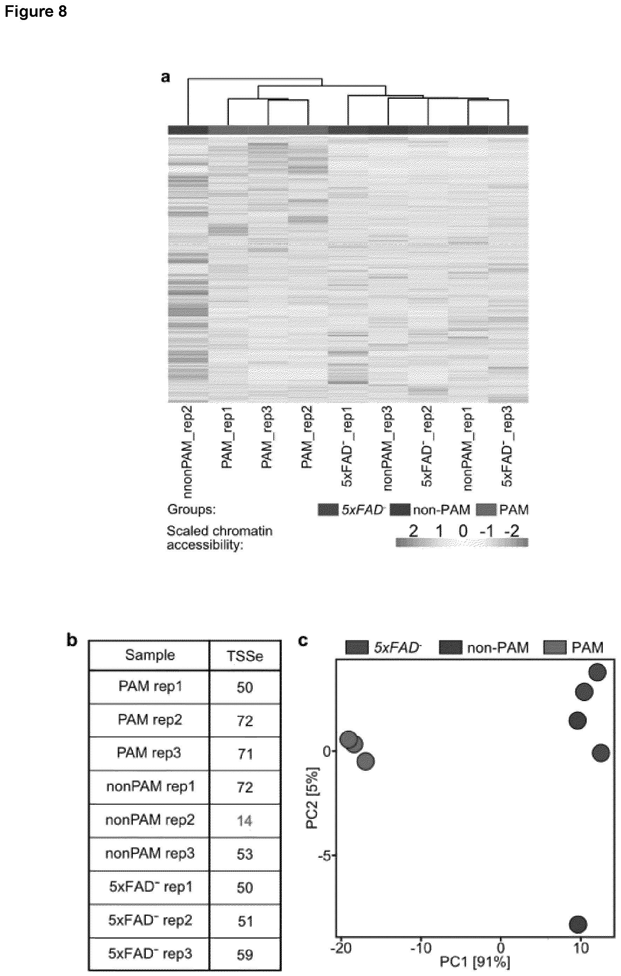

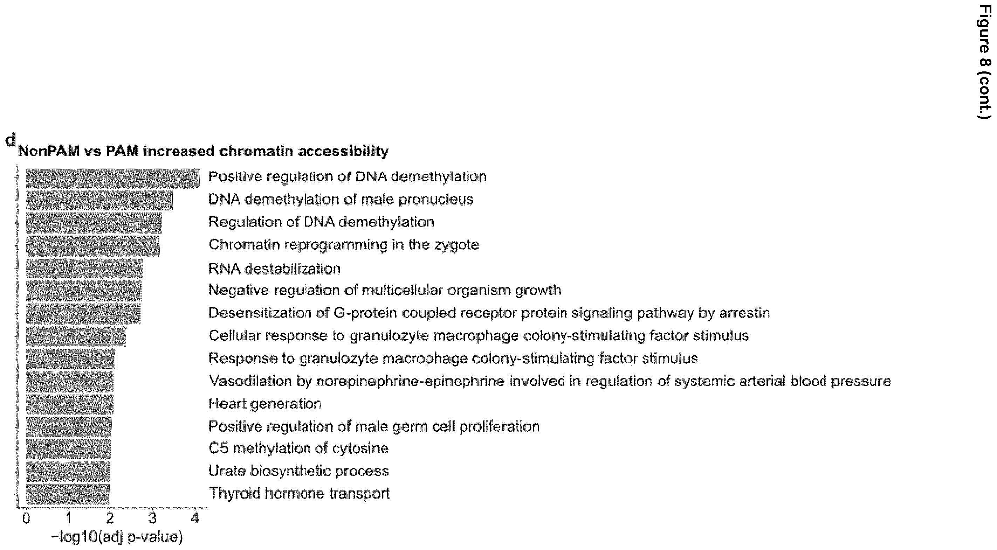

**Figure 8****: Quality control of chromatin accessibility data and gene ontology analysis.**
**(a)** Heat map displaying chromatin accessibility in consensus ATAC-Seq peak set (59,293 total peaks) across all samples. Each column of the heat map corresponds to an individual sample. Each row corresponds to a region of the consensus peak list. Normalized counts (DESeq2) from each sample are scaled per region.
**(b)** Transcriptional start site enrichment (TSSe) scores for all ATAC-seq datasets. Datasets with TSSe > 15 were used for downstream analysis. Non-PAM rep2 was removed before downstream analysis.
**(c)** Principal component analysis (PCA) of chromatin accessibility profiles in microglia from 5xFAD-(black) or 5xFAD+ mice (non-PAM: blue and PAM: red). Each symbol represents microglia isolated from one mouse.
**(d)** Significantly enriched pathways of genes linked to DARs with increased chromatin accessibility in non-PAM compared to PAM.

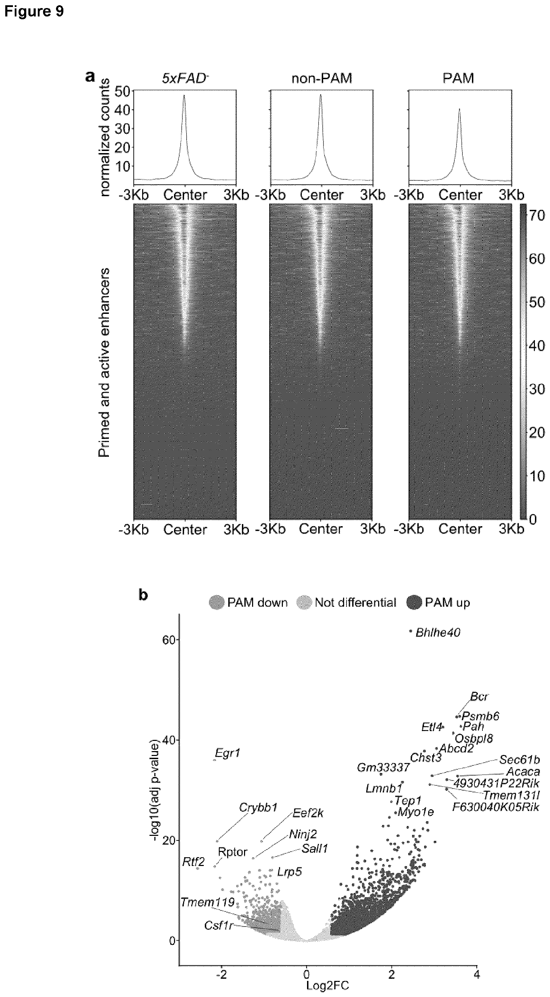

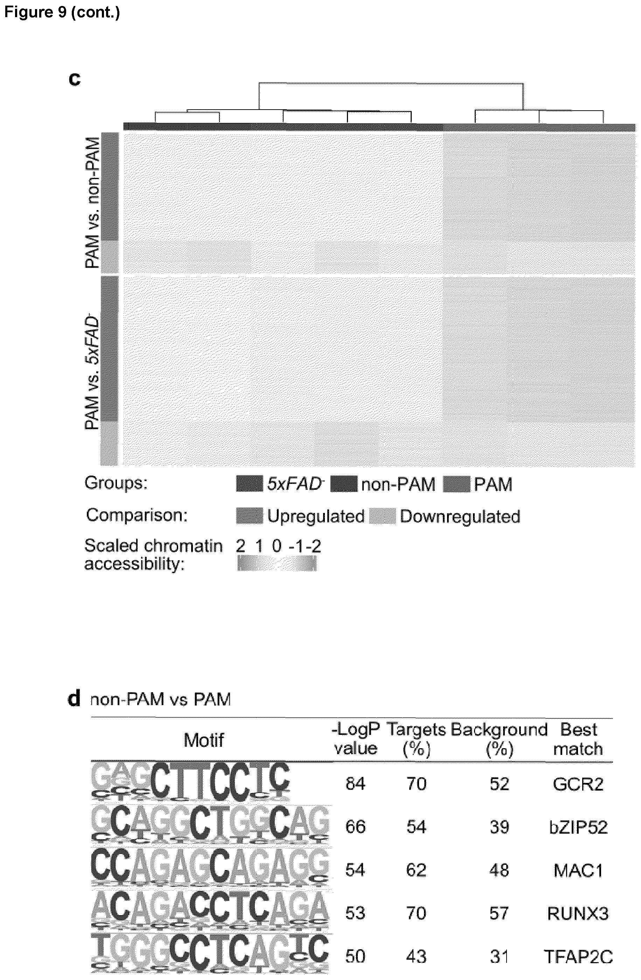

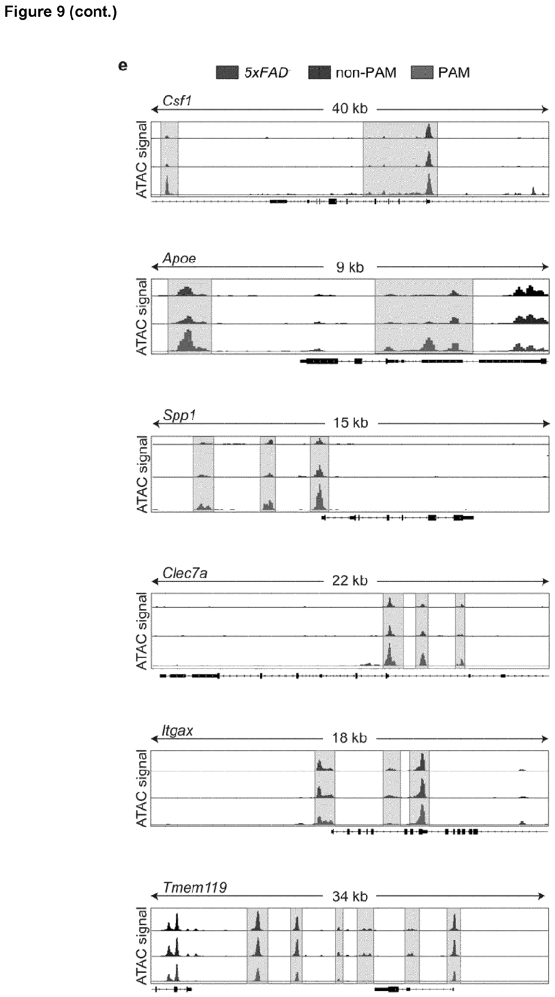

**Figure 9****: Distinct chromatin accessibility landscapes in microglial subsets depending on their spatial relationship to pathology.**
**(a)** Heat maps of chromatin accessibility in microglia from *5xFAD⁻* or *5xFAD⁺* mice (non-PAM and PAM) at 22,929 primed and active microglial enhancer regions.
**(b)** Volcano plot of chromatin accessibility differences of peaks between PAM and non-PAM. 4,360 peaks were more accessible in PAM (red; PAM up) and 1,301 showed higher accessibility in non-PAM (blue; PAM down). Differentially accessible regions (DARs; FDR < 0.05, log₂ (fold change) ≥ 0.584 or ≤ -0.584) were associated with nearby genes using ChIPSeeker. Labelling on the plot refers to predicted target genes. For DAR to gene annotations please see Supplementary Table 1.
**(c)** Heat map depicting chromatin accessibility for individual samples at DARs. The upper part of the heatmap shows DARs of PAM vs non-PAM, the lower part DARs of PAM vs WT. Comparison between PAM and non-PAM revealed 5,661 DARs with 4,360 regions showing a higher accessibility and 1,301 regions with decreased accessibility. In the comparison of PAM vs WT, out of a total of 7637 DARs, 5,848 showed increased accessibility and 1,789 showed decreased accessibility (FDR <0.05, log₂ (fold change) ≥ 0.584 or ≤ -0.584).
**(d)** Transcription-factor (TF) binding motifs enriched in DARs with higher accessibility in non-PAM compared to PAM.
**(e)** Integrative genome viewer (IGV) tracks displaying normalized profiles of *Csf1, Apoe, Spp1, Clec7a, Itgax, Tmem119, Csf1r,* loci in microglia from *5xFAD⁻* (black) or *5xFAD⁺* mice (non-PAM: blue, PAM: red). For display, DARs are highlighted, and data from all replicates were merged (n = 3 for 5xFAD- and PAM, n = 2 for non-PAM).

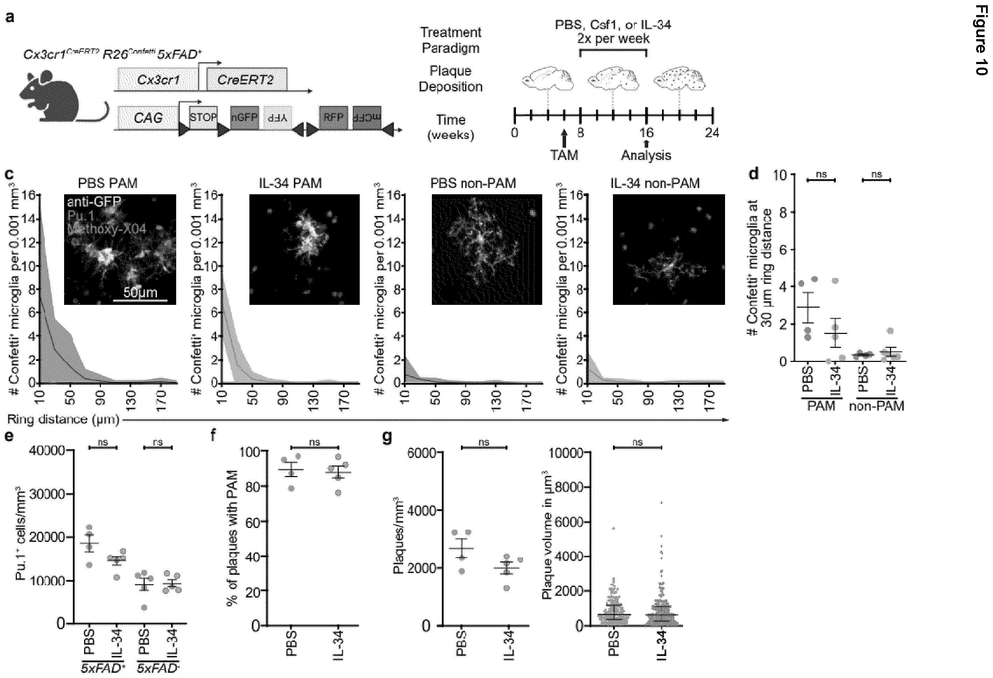

**Figure 10****: Csf1 treatment reduces the number of PAM and elevates phagocytosis.**
**(a)** Experimental scheme for Csf1 and IL-34 treatment of *Cx3cr1^{CreERT2}R26^{Confetti}5xFAD⁺* mice.
**(b)** Monte Carlo simulation: Densities of Confetti⁺ PAM and non-PAM in *Cx3cr1^{CreERT2}R26^{Confetti}5xFAD⁺* animals after treatment with IL-34 (red) or PBS (blue) at early stage of disease (n = 4-5 per group), relative to randomized datasets (pink). Densities are displayed relative to measured distances (i.e. ring distance; µm) between Confetti⁺ cells. Means per group and 98 % confidence intervals are shown for experimental datasets and randomized datasets. Representative confocal images of anti-GFP labeled Confetti⁺ (yellow) Pu.1⁺ (magenta) microglia in relation to Methoxy-X04 (amyloid beta, blue) are shown for each group. Scale bar = 50 µm.
**(c)** Quantification of Confetti⁺ cells from *Cx3cr1^{CreERT2}R26^{Confetti}5xFAD⁺* animals after treatment with IL-34 (red) or PBS (blue) at an early stage of disease. Each symbol represents one animal, n = 4 - 6 per group. ns: not significant.
**(d)** Quantification of Pu.1⁺ cells per mm³ in *Cx3cr1^{CreERT2}R26^{Confetti}5xFAD⁺* animals and littermate controls after treatment with IL-34 (red) or PBS (blue) at an early stage of disease. Each symbol represents one animal, n = 4 - 6 per group. Mean ± s.e.m. is shown. ns: not significant.
**(e)** Quantification of the percentage of plaques with associated PAM in *Cx3cr1^{CreERT2}R26^{Confetti}5xFAD⁺* animals after treatment with IL-34 (red) or PBS (blue) at an early stage of disease. Each symbol represents one animal, n = 4 - 5 per group. Mean ± s.e.m. is shown. ns: not significant.
**(f)** Quantification of number of plaques per mm³ (left) and quantification of individual plaque sizes (right) in the frontal cortices of *Cx3cr1^{CreERT2}R26^{Confeffi}5xFAD⁺* mice after treatment with IL-34 or PBS at an early stage of disease. Left: Each symbol represents one animal, n = 4-5 per group. Mean ± s.e.m. is shown. ns: not significant. Right: Each symbol represents one analyzed plaque, n = 547 - 555 plaques per group, n = 4 - 5 animals per group. Median ± interquartile range is shown. ns: not significant.

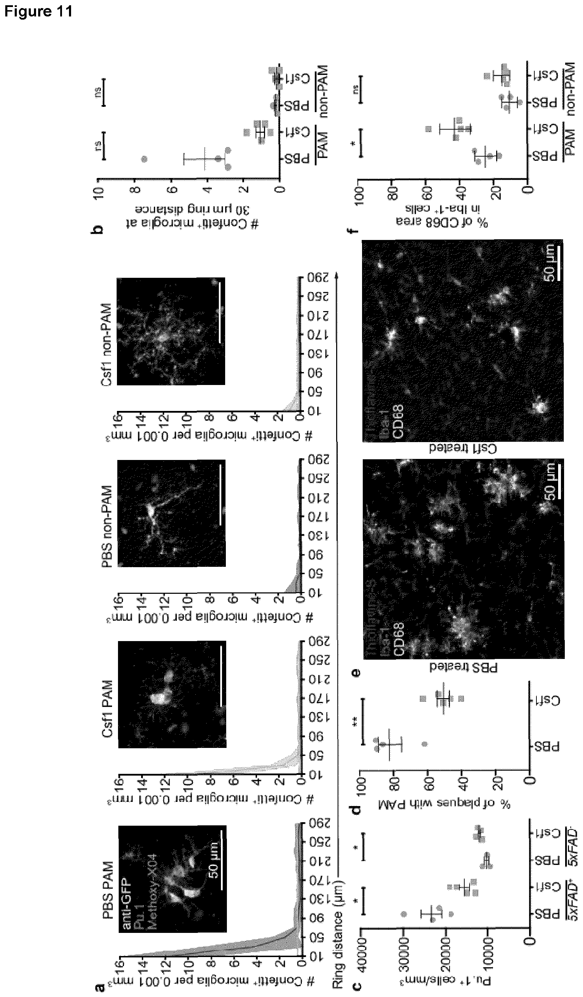

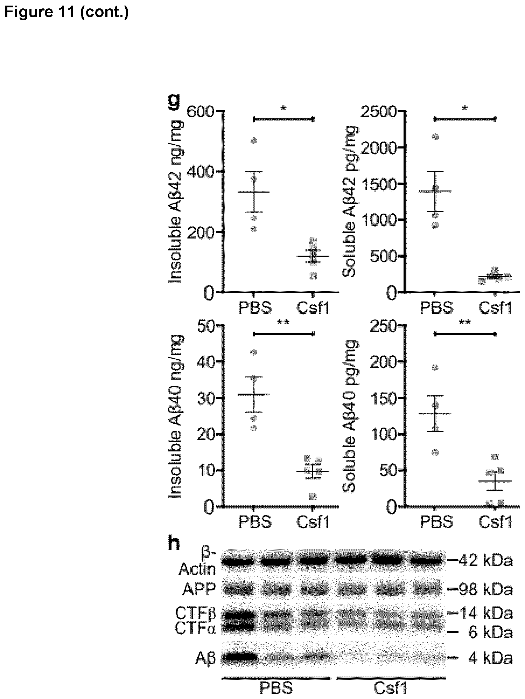

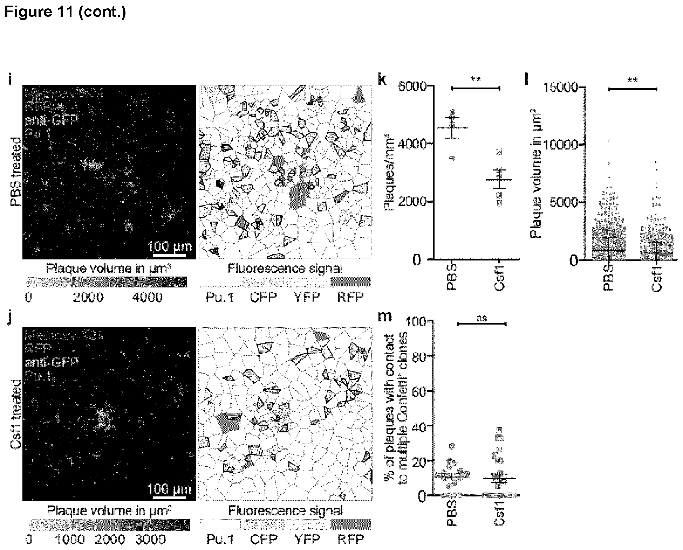

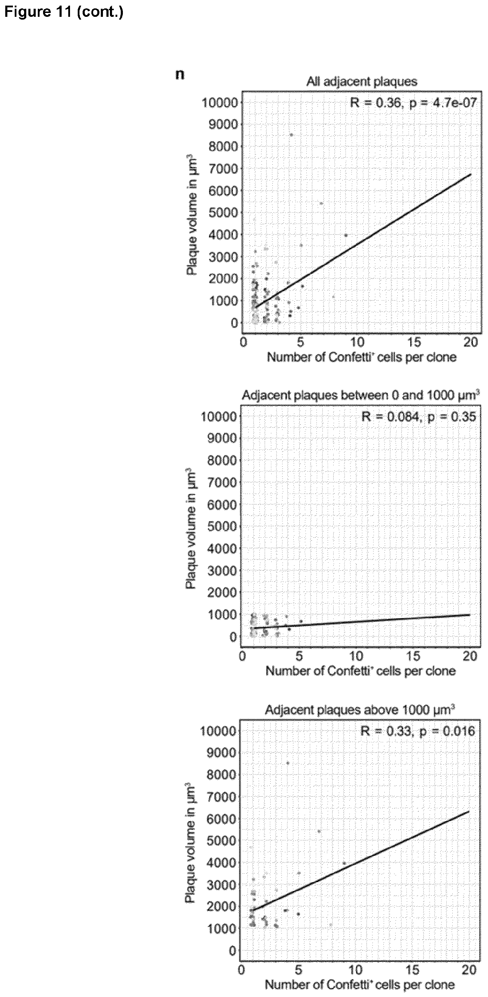

**Figure 11****: Engagement of Csf1r by Csf1 mitigates clonal expansion of PAM, elevates phagocytic capacity of PAM and attenuates amyloid pathology at early stage of disease.**
**(a)** Monte Carlo simulation: densities of Confetti⁺ PAM and non-PAM in *Cx3cr1^{CreERT2}R26^{Confetti}5xFAD⁺* animals after treatment with Csf1 (green) or PBS (blue) at early stage of disease (n = 4 - 5 per group), relative to randomized datasets (pink). Densities are displayed relative to measured distances (i.e. ring distance; µm) between Confetti⁺ cells. Means per group and 98 % confidence intervals are shown for experimental datasets and randomized datasets. Representative confocal images of anti-GFP labeled Confetti⁺ (yellow) Pu.1⁺ (magenta) microglia in relation to Methoxy-X04 (amyloid beta, blue) are shown for each group. Scale bars = 50 µm.
**(b)** Quantification of Confetti⁺ cells from *Cx3cr1^{CreERT2}R26^{Confetti}5xFAD⁺* animals after treatment with Csf1 (green) or PBS (blue) at an early stage of disease. Each symbol represents one animal, n = 4 - 5 per group. Mean ± s.e.m. is shown. ns: not significant.
**(c)** Quantification of Pu.1⁺ cells per mm³ in *Cx3cr1^{CreERT2}R26^{Confetti}5xFAD⁺* animals and littermate controls after treatment with Csf1 (green) or PBS (blue) at an early stage of disease. Each symbol represents one animal, n = 4 - 5 per group. Mean ± s.e.m. is shown. **p < 0.05*
**(d)** Quantification of plaques with PAM in *Cx3cr1^{CreERT2}R26^{Confetti}5xFAD⁺* animals after treatment with Csf1 (green) or PBS (blue) at an early stage of disease. Each symbol represents one animal, n = 4 - 5 per group. Mean ± s.e.m. is shown. ***p < 0.01.*
**(e)** Typical confocal pictures of CD68⁺Iba-1⁺ microglia from *Cx3cr1^{CreERT2}R26^{Confetti}5xFAD⁺* mice after treatment with PBS (left panel) or Csf1 (right panel) at an early stage of disease. Immunofluorescence is shown for CD68 (cyan), Iba-1 (red), and thioflavine-S (blue). Scale bars = 50 µm.
**(f)** Quantification of CD68⁺Iba-1⁺ PAM and non-PAM in *Cx3cr1^{CreERT2}R26^{Confeffi}5xFAD⁺* animals after treatment with Csf1 (green) or PBS (blue) at an early stage of disease. Each symbol represents one animal, n = 4-5 per group. Mean ± s.e.m. are shown. **p < 0.05.*
**(g)** ELISA of human Aβ(1-40) and Aβ(1-42) peptides in insoluble (left panels) and soluble fractions (right panels) of *Cx3cr1^{CreERT2}R26^{Confetti}5xFAD⁺* animals after treatment with Csf1 (green) or PBS (blue) at an early stage of disease. Each symbol represents one animal, n = 4 - 5 per group. Mean ± s.e.m. is shown. **p < 0.05, **p < 0.01.*
**(h)** Representative Western blots for β-actin, amyloid precursor protein (APP), C-terminal fragment (CTF)-β, CTF-α and human β-amyloid (Aβ) in brain lysates from the frontal cortices from *Cx3cr1^{CreER}R26R^{Confetti}5xFAD⁺* mice treated with CSF1 or PBS, respectively. Each lane represents one animal, n = 3 per group.
**(i)** Left panel: Characteristic immunofluorescence from the frontal cortex in *Cx3cr1^{CreERT2}R26^{Confetti}5xFAD⁺* animals treated with PBS. Labelling for Methoxy-X04 (blue), anti-RFP (red), anti-GFP (green) and Pu.1 (magenta). Scale bar = 100 µm. Right panel: Voronoi grid thereof visualizing plaque sizes and the positioning of Confetti- and Confetti⁺Pu.1⁺ microglia. Confetti⁻Pu.1⁺ microglia are shown in white, CFP⁺Pu.1⁺cells in cyan, YFP⁺ Pu.1⁺ microglia in yellow and RFP⁺Iba-1⁺ microglia in red. Color code as indicated in legend.
**(j)** Left panel: Confocal image of frontal cortex in *Cx3cr1^{CreERT2}R26^{Confetti}5xFAD⁺* animals treated with Csf1. Immunofluorescence for Methoxy-X04 (blue), anti-RFP (red), anti-GFP (green) and Pu.1 (magenta). Scale bar = 100 µm. Right panel: Voronoi grid thereof visualizing plaque sizes and the positioning of Confetti- and Confetti⁺Pu.1⁺ microglia. Confetti⁻Pu.1⁺ microglia are displayed in white, CFP⁺Pu.1⁺ cells in cyan, YFP⁺Pu.1⁺ microglia in yellow, and RFP⁺Iba-1⁺ microglia in red. Color code as indicated in legend.
**(k)** Quantification of number of plaques in frontal cortices from *Cx3cr1^{CreERT2}R26^{Confetti}5xFAD⁺* mice after treatment with Csf1 or PBS at an early stage of disease. Each symbol represents one animal, n = 4 - 5 per group. Mean ± s.e.m. is shown. **p < 0.05.*
**(l)** Quantification of individual plaque sizes after treatment with Csf1 or PBS at an early stage of disease. Each symbol represents one analyzed plaque; N = 1,016 - 1,346 plaques per group, n = 4-5 animals per group. Median ± interquartile range is shown. **p < 0.05.*
**(m)** Quantification of plaques associated with multiple Confetti⁺ PAM clones in frontal cortices *Cx3cr1^{CreERT2}R26^{Confetti}5xFAD⁺* individuals after treatment with Csf1 or PBS at an early stage of disease. Each symbol represents represent individual analyzed images (n = 17 - 25), n = 4 - 5 animals per group. Mean ± s.e.m. is shown. ns: not significant.
**(n)** Left panel: Correlation between Confetti⁺ microglia clone size and volume of adjacent Methoxy-X04⁺ plaques of all sizes in *Cx3cr1^{CreERT2}R26^{Confetti}5xFAD⁺* mice after treatment with Csf1. Colors represent individually analyzed images (technical replicates; n = 25) across multiple biological replicates (n = 5). Black line indicates linear regression for Csf1-treated animals. R (Pearson correlation coefficient) = 0.36. *** *p* = *4.7e⁻⁰⁷.* Center panel: Correlation between Confetti⁺ microglia clone size and Methoxy-X04⁺ plaques of maximal 1000 µm³ after treatment with Csf1. R = -0.084. ns *p = 0.35.* Right panel: Correlation between Confetti⁺ microglia clone size and Methoxy-X04⁺ plaques of less than 1000 µm³ in site from *Cx3cr1^{CreERT2}R26^{Confetti}5xFAD⁺* mice after treatment with Csf1. Colors represent individually analyzed images (technical replicates; n = 25) across multiple biological replicates (n = 5). Black line indicates linear regression for Csf1-treated animals. R = 0.33. **p = 0.016.*

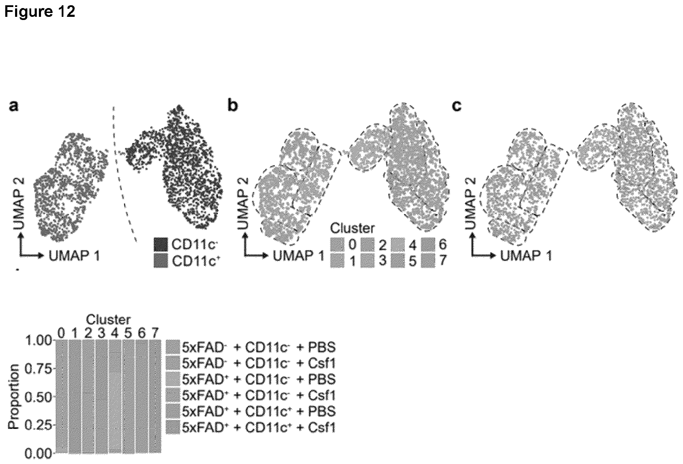

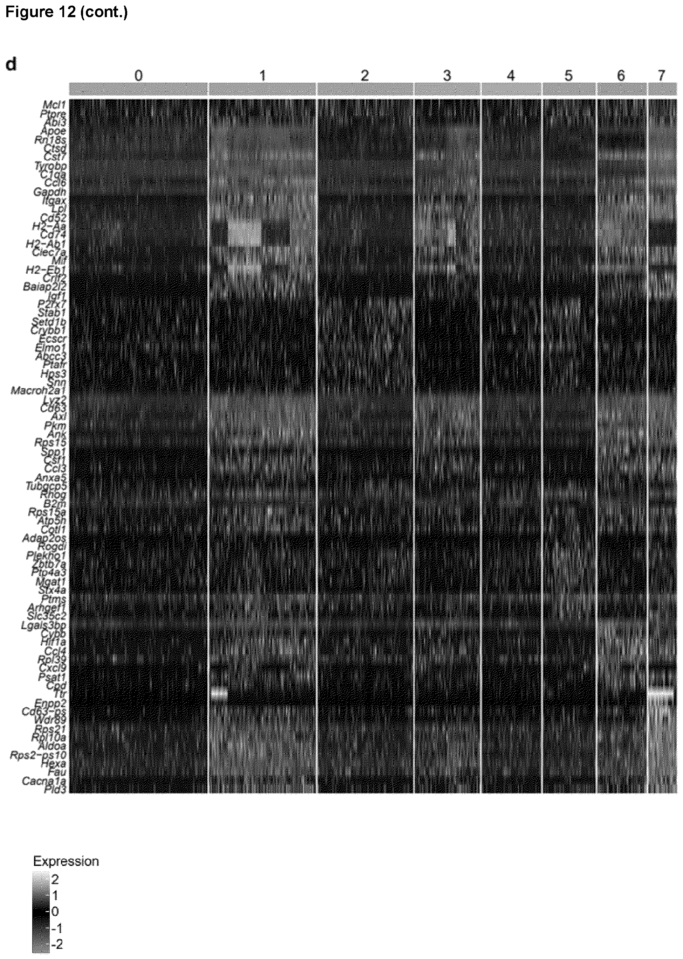

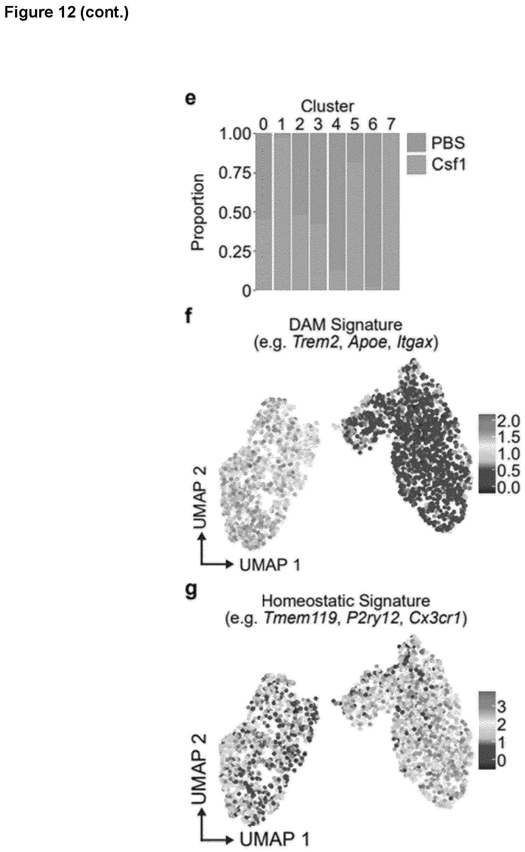

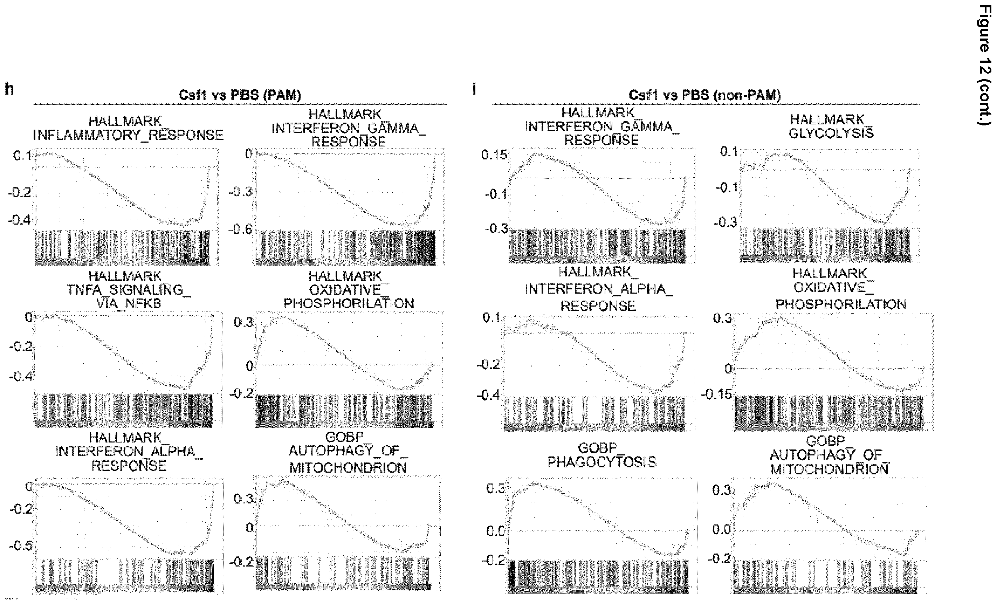

**Figure 12****: Csf1 treatment beneficially modulates functional and metabolic features of non- PAM-derived PAM making these cells competent to restrict amyloid pathology at early stages of neurodegeneration.**
**(a)** UMAP plot demonstrating the distribution of analyzed CD11c⁻ (blue) and CD11c⁺ (red) microglia from PBS- or Csf1-treated *Cx3cr1^{CreERT2}R26^{Confetti}5xFAD⁺* animals and controls at an early stage of disease. Each dot represents one cell (n = 2,687).
**(b)** UMAP visualizing the distribution of cell clusters (C0-C7) identified by unsupervised clustering of all analyzed cells from PBS- or Csf1-treated *Cx3cr1^{CreERT2}R26^{Confeffi}5xFAD⁺* animals and littermate controls at an early stage of disease. Each color represents one distinct cell cluster. Each cluster from b is highlighted by dotted lines.
**(c)** Left: UMAP representation displaying the distribution of clusters according to the treatment regimens (PBS- or Csf1-treated *Cx3cr1^{CreERT2}R26^{Confetti} 5xFAD⁺* animals and littermate controls at an early stage). Cell clusters from (b) (C0-C7) are displayed by dotted lines. Right: Stacked bar blot depicting the relative composition of microglial clusters (C0-C7) in respect to their treatment group.
**(d)** Heatmap presenting Log2 FC of the 20 most differentially expressed genes (DGE) per cluster. Expression levels are encoded by color as shown in the color legend.
**(e)** Stacked bar plot depicting the relative composition of microglial clusters (C0-C7) in respect to their treatments.
**(f)** UMAP feature plot depicting the expression of genes associated with microglial activation (Apoe, *Axl, Bhlhe40, Clec7a, Csf1, Cst7, Ctsb, Ctsd, Ctsl, Cybb, Fabp5, Fth1, Itgax, Gnas, Gpnmb, Grn, Il1b, Lgals3, Lilrb4, Lpl, Lyz2, Msr1, Nos2, Spp1, Tfec, Trem2, Tyrobp, Vegfa).* Scale refers to transcript counts of homeostatic core signature.
**(g)** UMAP representing the expression of genes associated with a homeostatic microglia signature *(P2η12, Csf1r, Cx3cr1, Tmem119, Pu.1, Sall1).* Scale refers to transcript counts of homeostatic core signature.
**(h)** Gene set enrichment analysis (GSEA) all PAM after Csf1 compared to PBS treatment. Plots for the running sum of S are shown for defined gene sets together with the maximum enrichment score (ES).
**(i)** GSEA of all non-PAM after Csf1 treatment. Plots for the running sum of S are shown for defined gene sets together with the maximum enrichment score (ES).

### EXAMPLES

The invention is demonstrated by way of the examples disclosed below. The examples provide technical support for and a more detailed description of potentially preferred, non-limiting embodiments of the invention.

### Summary of the Examples

In order to demonstrate the functionality and beneficial properties of the inhibition of the whole blood compatible matrix described herein, the following examples are to be considered:
- Methods
- Adjacent non-plaque-associated microglia are constantly attracted to amyloid plaques where they clonally expand and adopt a disease-associated profileclone sizes of PAM are modulated by gut microbiota and peripheral stimuli in an age-dependent manner.
- Only non-PAM exhibit a high transcriptional responsiveness during neurodegeneration.
- Distinct chromatin accessibility landscapes separate non-PAM from PAM
- Engagement of CSF1 R by CSF1 but not IL-34 modulates clonal expansion and phagocytosis in the PAM subset thereby improving amyloid pathology
- CSF1 shapes the inflammatory and metabolic gene expression profiles of both PAM and non-PAM rather than blocking cell shifts.

### Example 1: Methods

*Mice:* Female *5xFAD(Tg6799), C57BI*/*6J, Cx3cr1*^{*CreERT2*/}*⁺R26*^{*Confetti*/}*⁺, Tmem119*^{*CreERT2*/}*⁺ R26^{Confetti}, Cx3cr1*^{*CreERT2*/}*⁺R26*^{*Confetti*/}*⁺5xFAD⁺,* and *Tmem119*^{*CreERT2*/}*⁺R26*^{*Confetti*/}*⁺5xFAD⁺* mice were used in this study ^{11 77-79}. All animals were bred and housed in a specific-pathogen-free facility and given food and water *ad libitum. 5xFAD, Cx3cr1^{CreERT2}* and *R26^{Confetti}* were genotyped as previously described ^{7, 77}. Genotyping for *Tmem119^{CreERT2}* was according to Jackson Laboratory (stock no. 031820). All quantification experiments were performed in a blinded manner by assignment of unidentifiable numbers to mice, tissues and images for data acquisition and processing. For all experiments on *Cx3cr1*^{*CreERT2*/}*⁺R26*^{*Confetti*/}*⁺5xFAD⁺* mice in the study, 5 mg of tamoxifen was dissolved at 45°C in a shaker at 750 rpm to a concentration of 10 mg/ml in corn oil and applied subcutaneously. For scRNA-seq and ATAC-seq experiments on *Cx3cr1*^{*CreERT2*/}*⁺R26*^{*Confetti*/}*⁺5xFAD⁺* mice and all experiments on *Tmem119*^{*CreERT2*/}*⁺ R26^{Confetti} 5xFAD⁺* mice in the study, 10 mg of tamoxifen was dissolved at 42°C in a shaker at 750 rpm to a concentration of 40 mg/ml in corn oil and applied subcutaneously. Animal studies were approved by the Regional Councils of Freiburg, Germany and performed in accordance to the respective national, federal and institutional regulations.

*Lipopolysaccharide (LPS) treatment:* Mice were intraperitoneally injected with 1 mg per kg body weight of LPS (purified from E. *coli* 0127:B8, Sigma L3129, lot 037M4067V) dissolved in a final concentration of 0.1 mg/ml in phosphate-buffered saline (PBS) twice a week for 8 weeks.

*Antibiotics (ABX) treatment:* For depletion of the microbiota, SPF mice were provided with drinking water containing 1mg/ml Cefoxitin (Santa Cruz Biotechnology), 1 mg/ml Gentamicin (Sigma-Aldrich), 1mg/ml Metronidazole (Sigma-Aldrich), and 1mg/ml Vancomycin (Hexal) for 8 weeks *ad libitum.* Antibiotics were renewed every other day and the water bottles with antibiotics were light protected.

*Csf1 and IL-34 treatment:* Mice were intraperitoneally injected with recombinant murine Csf1 (Peprotech,#315-02) or IL-34 (BioLegend, #577602). Animals were injected intraperitoneally twice weekly with either sterile PBS (100 µl), a solution containing 10 ng/µl murine Csf1 (final injection: 40 µg/kg) dissolved in sterile PBS, or a solution containing 450 ng/µl murine IL-34 (final injection: 100 µg/kg) dissolved in sterile PBS for 8 weeks. For scRNA-seq analysis of PAM and non-PAM from Csf1 treated mice, all animals were analyzed after 4 weeks.

*Administration of 5-bromo-2'-deoxyuridine (BrdU):* 0.1 mg of BrdU per gram of body weight (Sigma-Aldrich, B5002) were injected intraperitoneally twice a day over 5 days prior to sacrifice. The working solution was prepared by dissolving BrdU at 8 mg/ml in sterile PBS.

*Immunohistochemistry:* Mice were anesthetized (i.p. 100 mg Ketamine and 5 mg xylazine per kg body weight) and transcardially perfused with PBS as described before ⁸⁰. Brains were dissected and postfixed for 4-6 h at 4 °C in 4 % paraformaldehyde (PFA), washed with PBS, and incubated in 30 % sucrose in PBS at 4 °C until fully enriched for cryoprotection. Samples were embedded in OCT (Tissue-Tek) and frozen in a pure ethanol bath cooled with dry ice prior to sectioning. 60 µm sagittal brain sections were obtained with a cryostat (Leica) for all free-floating immunohistochemistry experiments, with the following exceptions, which were conducted with 30µm sagittal brain sections: BrdU (Figure 1c; see below); ApoE, Clec7a, Axl, and P2RY12 (Figure 3a; Figure 2); CD11c (Figure 3b; Figure 2); CD68 (Figure 11e, Figure 5c). Sections were washed in wash buffer (WB; 0.1% Triton-X in PBS) to remove residual OCT before permeabilization in blocking buffer (BB; 0.5% Triton-X 100, 5% bovine serum albumin (BSA), 5% normal donkey serum, 0.1% NaN3 in PBS) for 1 h on a gentle shaker at room temperature (RT). Primary antibodies of interest were dissolved in BB and incubated overnight at 4 °C, including: 1:200 rabbit anti-Pu.1 (Cell Signaling, 2258S); 1:1000 chicken anti-GFP (Abcam ab13970); 1:100 rabbit anti-P2RY12 (Anaspec 55043A); 1:100 rat anti-Clec7a (InVivoGen mabg-mdect); 1:50 hamster anti-CD11c (Novus NB110-97871); 1:500 rabbit anti-lba-1 (Abcam ab178846 or Wako 019-19741); 1:200 goat anti-lba-1 (Novus NB100-1028); 1:500 rabbit anti-Tmem119 (Abcam ab209064); 1:100 rat anti-CD68 (Biorad MCA1957); 1:200 goat anti-ApoE (Merck AB947); 1:100 goat anti-Axl (R&D systems AF854). Following incubation with primary antibodies, tissues were washed 6 times with WB. Secondary antibodies of interest were diluted 1:1000 in BB and incubated for 2 h at RT, including: donkey anti-rabbit Alexa Fluor 488 (Invitrogen A21206), donkey anti-rabbit Alexa Fluor 568 (Invitrogen A10042), donkey anti-rabbit Alexa Fluor 647 (Invitrogen A-31573), donkey anti-goat Alexa Fluor 647 (Life Technologies A21447), chicken anti-rat Alexa Fluor 647 (Invitrogen A21472), donkey anti-hamster Alexa Fluor 647 (Invitrogen A21451) and donkey anti-Chicken Alexa Fluor 488 (Jackson ImmunoResearch Europe Ltd 703-545-155). Following incubation with secondary antibodies, tissues were again washed 6 times with WB. For the staining of amyloid beta plaques, Methoxy-X04 (Tocris #4920), Thioflavine-S (Sigma Aldrich T1892), and Thiazine Red (Morphisto #12990) was used. A stock solution for Methoxy-X04 was achieved by first diluting to 10 µg/µl in DMSO, then further diluting 15µl in 5ml of 40% Ethanol and 60% H₂O. A working solution was achieved by dissolving the stock solution 1:4 in PBS. Thioflavine-S was diluted 1:1400 from a 0.05% in 100% ethanol stock solution, and Thiazine Red was diluted 1:20 from a 0.01% in 100% ethanol stock solution. Either Methoxy-X04, Thioflavine-S or Thiazine Red were left to incubate for 10 min following staining of sections with primary and secondary antibodies; excess would be removed through 4 washes with WB prior to mounting. Sections were mounted with ProLong Diamond Antifade Mountant (Life Technologies, P36961).

For BrdU staining (Figure 1c), 30µm mouse brain sagittal sections were permeabilized as described before, followed by DNA denaturation in 2 M HCl for 18 min at 37 °C. Sections were re-equilibrated in PBS at pH 8.5 and washed in PBS until the tissue quality prior to DNA denaturation was regained. Samples were blocked for 60 min at RT with BB before overnight incubation with 1:15 mouse anti-BrdU antibody (Roche 11170376001) at 4 °C. Brief washes with PBS were performed before overnight incubation with 1:500 chicken anti-GFP (Abcam ab13970) and 1:1500 rabbit anti-RFP (Rockland 600-401-379) at 4 °C. Alexa Fluor 568-conjugated donkey anti-mouse IgG (Life Technologies A-10037), Alexa Fluor 488-conjugated donkey anti-chicken (Jackson ImmunoResearch Europe Ltd 703-545-155) and Alexa Fluor 647-conjugated donkey anti-rabbit IgG (Invitrogen A-31573) were used at 1:1,000, together with nuclear counterstain 4',6-diamidino-2-phenylindole (DAPI, 1:5000, Sigma Aldrich) for 2 h on a gentle shaker at RT. Sections were then mounted as described above.

*Microscopy:* All images of immunohistochemistry experiments conducted with 60 µm sections were taken on a Leica SP8 microscope or a Leica SP8X with white light laser, using a 20×, glycerine immersion 0.95 NA W lens at a resolution of 1024 × 1024 pixels and Z-step size between 1 and 1.04µm. Subsequent analyses were performed on a minimum of 3 Z-stacks imaged from the frontal cortex of each mouse. All images were acquired on a Keyence BZ-9000 inverted fluorescence microscope using a 20 x 0.75 NA objective lens: BrdU (Figure 1c); ApoE, Clec7a, and P2RY12 (Figure 3a); CD11c (Figure 3b); CD68 (Figure 11e); ApoE, CD11c, Clec7a, and Axl, and P2RY12 (Figure 2); and CD68 (Figure 5c).

*Image analysis and quantification:* Microglia labelling (e.g. Confetti⁺, Pu.1⁺, and/or Iba-1⁺) and other quantifications (e.g. number of Methoxy-X04⁺ plaques) was conducted through an image analysis workflow designed in the open source KNIME Analytics Platform (KNIME AG, Zurich, Switzerland). The workflow incorporates a number of techniques (e.g. signal erosion and dilation, signal thresholding, and signal size filtration) to quantify signal in an unbiased manner. Volumes of Methoxy-X04+ plaques were calculated by multiplying the sum of signal positive pixels by the known image voxel dimensions. Analyzed images were then converted to HDF5 via the imaged Bioformats analyzer and HDF5 plugins, in order to quality control KNIME-generated quantifications with the iRoCS (Interactive Arabidopsis Root Analysis; Computer Vision Group Freiburg, https://lmb.informatik.uni-freiburg.de/resources/opensource/iRoCS/) Toolbox. The area or volume, as appropriate, was then calculated for each analyzed image and used to normalize quantifications where relevant.

*Voronoi gridding, linear regression analysis (plaque size and microglial clone size):* Center coordinates of microglia cell bodies (e.g. Confetti⁺, Pu.1⁺, and/or Iba-1⁺) and Methoxy-X04⁺ plaques calculated via KNIME and iRoCS were Z-projected into a single plane to compute a two-dimensional Voronoi mesh in R Studio (version 4.4.0) using the bleiglas package (https://github.com/nevrome/bleiglas), Voro++ (https://math.lbl.gov/voro++/), and their associated dependencies. Voronoi cells of microglia were coloured based on their fluorescence signal whilst Voronoi cells of Methoxy-X04+ plaques were coloured based on their calculated volume. The resulting tessellation was used to establish contact relationships between Voronoi cells in order to quantify the size of Confetti⁺ microglia clones, as well as to determine clone-to-plaque contacts. Information of clone-to-plaque contacts were used to establish linear regressions between microglia clone size and amyloid beta plaque size (Figure 1h, Figure 11n).

*Monte Carlo Simulation:* The number of Confetti⁺ cells that shared the same colour label was quantified through repeated measures taken from 10 to 290 µm (Figure 1, 4, 11) or 10 to 190 µm (Figure 10). Starting from a Confetti⁺ microglia, a 10 µm-radius ring was defined, and the number of same-coloured microglia that were found within that volume (10 µm XY * height of image in Z) was recorded. Following this, the ring radius increases by 20 µm, quantifying the number of same-coloured microglia within the 10-30 µm ring. This process repeats till the last ring, at a 270-290 µm radius from the origin cell. These calculations are performed on multiple images per biological replicate, before being averaged per replicate, and then averaged by experimental group. Following this, a Monte Carlo (MC) simulation is performed, to evaluate whether the observed Confetti+ densities at various distances are the result of clonal expansion or random recombination. During this simulation, the location of all Confetti⁺ cells are shuffled; this is achieved by switching the location of a Confetti⁺ cell with the location of a random Pu.1⁺ cell. Following 10000 shuffling simulations and quantifications, a baseline is established, which is then used to represent Confetti labelling that results purely from random recombination, i.e. the randomized dataset. The 98 % confidence interval is then calculated for both the experimental and the randomized dataset. At distances where these confidence intervals do not overlap, the null hypothesis-that the Confetti labelling seen in the image is a result of random recombination-is rejected with a significance of p<0.02. A full mathematical description is provided elsewhere ^{7, 16}.

*ELISA:* For the quantification of soluble and insoluble, Aβ40 and 42 species, tissue from the frontal cortex was homogenized (10% w/v) in PBS + protease inhibitor and sequentially extracted with PBS (soluble fraction), PBS + 0.1% Triton X-100 (membrane bound fraction) and finally with 8 M guanidine hydrochloride solution. Protein concentration in each fraction was measured with Bradford reagent (Roth) and ELISA was performed using Amyloid beta 42 Human ELISA Kit (ThermoFisher Scientific, #KHB3441) and Amyloid beta 40 Human ELISA Kit (ThermoFisher Scientific, #KHB3481) according to the manufacturer's protocol.

*Western Blot:* Total protein from frontal cortex was extracted in RIPA buffer (50mM HEPES pH7.5, 150mM NaCl, 1mM EDTA, 10% Glycerin, 1% Triton X-100, 10mM Na₄O₇P₂, Protease Inhibitor). Protein concentration was measured with Bradford reagent (Roth). Samples were separated by 4-12% NuPAGE Bis-tris mini gels using NuPAGE LDS sample buffer, NuPAGE sample reducing agent and NuPAGE MES SDS running buffer (Invitrogen). Proteins were transferred on Polyvinylidene difluoride (PVDF) membranes (Biorad) and visualized using Clarity Western ECL Substrate (Biorad). Antibodies against APP and CTFs (rabbit polyclonal antibody against the APP C-terminus, 6687, 1:1000), anti-Aβ (mouse, 1:3000, Covance, 6E10) and anti-β-actin-HRP (mouse, 1:5000, abcam, ab20272) were used.

*Cell Sorting:* Index Sorting was done using a MoFlo Astrios EQ with 405, 488, 561, and 640 nm lasers located at our lighthouse core facility with help of technical assistance. Antibodies and dyes used were: 1:500 Fixable Viability Dye in eFluor780 (Thermofisher 65-0865-14); 1:200 for all antibodies used for the dump channel in APC-Cy7 (CD3 clone 145-2C11 from BioLegend #100330, Gr1 clone RB6-8C5 from BioLegend #108423, CD19 clone 1D3 from BD #557655); 1:100 for CD45 in BV786 (clone 30-F11 BD #564225); 1:100 for CD11b in BV605 (clone M1/70 BioLegend #101257); 1:100 for CD11c in PE-Cy7 (clone N418 eBioscience #25-0114-82); 1:100 for Clec7a in APC (clone 17-5859-80 eBioscience #bg1fpj).

*scRNA-seq:* Single cortical microglia were sorted into 384-well plates. Gating strategy is provided in Suppl. Figure 3a. Cells were spun down and stored at -80°C until further processing. RNA from single cells was isolated and transcribed into cDNA. For RNA sequencing, the mCEL-Seq2 protocol was used as recently established ⁸¹⁻⁸⁷.

*scRNA-seq analysis:* Analysis of all three datasets was performed according to the following initial pipeline: The count matrices were analyzed with R Studio (version 2022.07.1 Build 554) using the Seurat package (version 5). For quality control, duplets were excluded by selecting cells with <4000 nFeatures and ambient noise was filtered by selecting cells with >200 nFeatures. The data was normalized using the "NormalizeData" function with the "LogNormalize" method, variable features were found using "FindVariableFeatures" function with the 'variance stabilizing transformation' method and selecting the top 4000 features. Data was scaled using "ScaleData" function and then reduced in dimensions using principal component analysis (PCA) with the "RunPCA" function.

For the scRNA-seq analysis of the early-stage dataset (Figure 4, Figure 6 f - g), 1095 cells remained after initial quality control and were embedded in a euclidean space using the "RunUMAP" function and the first 15 PCs. For the analysis of the late-stage dataset (Suppl. Figure 3h-n), 1017 cells were embedded as described above using the first 20 PCs and a clustering resolution of 1.0. For the subsequent analysis of the CSF1-treated scRNA-seq dataset, 2,686 cells were embedded using the first 5 PCs at a clustering resolution of 0.7. Unsupervised clustering of the cells was done running the "FindNeighbors" function for the first 15 dimensions and the "FindClusters" function with a cluster resolution of 1.0 using the Louvain algorithm. The cluster resolution was selected after analyzing all possible ramifications using the "Clustree" function from 0.1-1.5, with steps every 0.1. Differential abundance tests were calculated using a one-sided proportion test.

The heatmap of differentially expressed (DE) genes in the individual cell clusters was produced using the "DoHeatmap" function and the features were selected after calculating the top DE genes using a Wilcoxon test with the "FindAIIMarkers" function and a average log2Fc threshold of 0.1. Afterwards, genes with the following regular expressions were filtered out using grepl: "Gm\\d\\d", "G\\d\\d", "\\d\\d\\d", "mt-". The dataset was further grouped by cluster and the top 20 DE genes were selected for each cluster using the "top_n" function and ordered by avg_log2FC. The microglial activation signature was produced using the AddModuleScore and FeaturePlot for the following genes: Apoe, *Axl, Bhlhe40, Clec7a, Csf1, Cst7, Ctsb, Ctsd, Ctsl, Cybb, Fabp5, Fth1, Itgax, Gnas, Gpnmb, Grn, Il1b, Lgals3, Lilrb4, Lpl, Lyz2, Msr1, Nos2, Spp1, Tfec, Trem2, Tyrobp, Vegfa* as previously described²⁴. The homeostatic gene signature was calculated the same way for the following genes: *P2ry12, Csf1r, Cx3cr1, Tmem119, Pu.1, Sall1 ²⁴*. The expression of gene expression modules was quantified using the UCell R package version 2.4.0. The gene lists associated with gene ontology terms presented in figure 3 were obtained from the object (MSigDB_v6.0_C5_mouse within the MOFAdata R package version 1.16.1. The following gene ontology terms were visualized: GO_MYD88_DEPENDENT TOLL_LIKE_RECEPTOR SIGNALING_PATHWAY: *Ly96, Hspd1, Tlr5, Tab2, Irak3, Rps27a, Tnip1, Ubb, Map3k1, Tab1, Irak4, Cd14, Traf6, Tlr2, Map3k7, Tlr4, Cd36, Tlr1, Tlr6, Ubc, Reg3g, Irak2, Uba52, Tirap, TIr9, Myd88, Irak1, Tab3, Btk, TIr8, Tlr7, Ly96, Hspd1, Tlr5, Tab2, Irak3, Rps27a, Tnip1, Ubb, Map3k1, Tab1, Irak4, Cd14, Traf6, Tlr2, Map3k7, Tlr4, Cd36, TIr1, Tlr6, Ubc, Reg3g, Irak2, Uba52, Tirap, Tlr9, Myd88, Irak1, Tab3, Btk, Tlr8, Tlr7*; GO_MYD88_INDEPENDENT TOLL_LIKE_RECEPTOR SIGNALING_PATHWAY: *Ly96, Casp8, Ikbke, Ube2d1, Tbk1, Rps27a, Ubb, Traf3, Ripk1, Ticam1, Prkce, Cd14, Ticam2, Chuk, Tank, Traf6, Ube2d3, Tlr4, Ubc, Tnip3, Irf3, Irf7, Fadd, Ikbkb, Tlr3, Uba52, Birc2, Birc3, Ikbkg*; DAM_trem2_dep: *Spp1, Clec7a, Itgax, Lpl, Cts7, Lgals3, Gpnmb, Igf1, Mif, Cd33, Abca1, Srgap2, Ctsl, Cd63, Csf1, Cd9, Ctsa, Ank, Ctsz, Axl, Serpine2, Ccl6, Cd68, Cadm1, Cd52, Gusb, Hif1a, Lilrb4a, Trem2, Serinc3;* DAM trem2_ind: *Apoe, Ctsb, Lyz2, Fth1, Ctsd, Timp2, H2-D1, B2m, Ctsb, Tyrobp;* GO_CELLULAR_SENESCENCE: *Sirt1, Hmga2, Tbx2, Smc6, Id2, Prkcd, Nsmce2, Opa1, Hmga1, Mapk14, Cdkn1a, Srf, Smc5, Pla2r1, Prmt6, Cdkn2a, Tbx3, Mapkapk5, Cav1, Wnt16, Arntl, Hras, Kat6a, Calr, Terf2, H2afx, Ulk3, Pml, Map2k1, Magea2.* The module enrichment scores quantified by the Mann-Whitney U statistic were visualized using the VlnPlot Seurat function.

Gene Set Enrichment Analysis (GSEA) was done using the GSEA 4.3.2 software ^{84,85}. The ranked list of the gene name and avg_log2FC for the GSEA was obtained by using "FindMarkers" function with the MAST (Model-based Analysis of Single-cell Transcriptomics) test⁸⁴, setting a log fold change threshold of 0 and a min.pct of 0.001.

Volcano plots were produced using "EnhancedVolcano" function, with the avg_log2FC on the x-axis and the p_val_adj on the y-axis, a FC cut off of 0.25 and an adjusted p-value cut off of 0.05 were used.

*ATAC-seq:* To profile chromatin accessibility, we performed the Assay for Transposase Accessible Chromatin sequencing (ATAC-seq) protocol as described before86, with some modification: 50,000 microglia were sorted in 250 µl of FACS buffer (1x PBS, 2% FBS, 2mM EDTA) and pelleted by centrifugation for 5 min, 500 g at 4 °C. Cell pellets were washed once with 1x PBS and cells were pelleted by centrifugation using the previous settings. Cell pellets were resuspended in 50 µl of cold lysis buffer (10 mM Tris HCl pH 7.4, 10 mM NaCl, 3 mM MgCl2, 0.1% IGEPAL CA-630) and nuclei were pelleted by centrifugation for 10 min, 500 g at 4 °C. Nuclei were re-suspended in 50 µl reaction buffer containing 2.5 µl of Tn5 transposase and 25 µl of TD buffer (Nextera Sample preparation kit, Illumina). The reaction was incubated at 37 °C for 45 min with gentle mixing. Immediately following the transposition reaction, tagmented DNA was purified using the MinElute PCR Purification Kit (Qiagen) according to manufacturer instructions. Libraries were initially amplified by a 5-cycle PCR before each sample was assessed by RT-qPCR for the optimum number of extra PCR amplification cycles (max. 9) to reduce GC and size bias. 5 µl of indexing primers as in Buenrostro et al. 2013 and NEBNext Q5 Hot Start HiFi PCR Master Mix (New England Biolabs) were used. Library clean-up was performed twice using the NucleoMag NGS Beads (Macherey-Nagel). DNA concentration was measured with a Qubit fluorometer (Life Technologies) and library sizes and quality were determined using Bioanalyzer (Agilent). Libraries where sequenced on Illumina HiSeq 2000 for an average of 30 million unique reads per sample.

*ATAC-seq analysis:* Reads were aligned to the mouse reference genome (GRCm38/mm10) using *Bowtie2* (v2.3.4.3) ⁸⁸ and trimming the adaptors and low quality read ends using Trim Galore! (v0.4.3.1, https://github.com/FelixKrueger/TrimGalore). Mitochondrial DNA reads were removed with *BAMTools* (v2.5.1) ⁸⁹ and blacklisted genomic regions for mm10 defined by ENCODE ⁹⁰ were excluded using *bedtools intersect* (v2.3.0.0) ⁹¹. For quality control, Transcription Start Site enrichment (TSSe) scores were calculated and samples with TSSe < 15 were removed from downstream analysis according to ENCODE standards (https://www.encodeproject.org/data-standards/atac-seq/atac-encode4/). Peaks were called with MACS2 (v2.1.1.20160309.6, -shift -100 -extsize 200 -nomodel -call-summits -q 0.05) ⁹². Peaks from individual samples were combined into a consensus peak list. Differentially accessible regions (DARs) were identified using *DESeq2* (v2.11.40.7, log₂(fold change) ≥ 0.584 or ≤ -0.584, FDR < 0.05) ⁹³, followed by region annotation using *ChIPSeeker* (v1.18.0). *DeepTools2* (Version 3.5.1) was used for visualization ⁹⁴. Individual sample bigwig files were normalized with DESeq2 ⁹³ size factors before being merged with bigwigAverage ⁹⁴. *DeepTools2* (Version 3.5.1, compute Matrix, plotHeatmap) ⁹⁵ was used to visualize chromatin accessibility from merged bigwig files at previously defined microglia-specific primed and active enhancer regions ⁹⁶. Motif enrichment analysis was performed using *HOMER* (v4.11) ⁹⁷. Pathway enrichment analysis was performed using *GREAT* (v4.04)⁹⁸. The analysis workflow and visualizations were performed on the Galaxy platform ⁸⁷.

*Statistical analysis:* No statistical methods were used to predetermine sample sizes. GraphPad Prism 9 was used for all statistical testing. Prior to analysis, datasets were tested for homoscedasticity (Brown-Forsythe test) and normality (D'Agostino-Pearson omnibus, Anderson-Darling, Shapiro-Wilk, and Kolmogorov-Smirnov) to determine the appropriate statistical test. The statistical tests were as follows: Mann-Whitney test (Figure 3k, 11g, 11l, 11m; Figure 10d, 10g); Kruskal-Wallis test with Dunn's post-hoc testing (Figure 4c, 4g, 4h, 4i; Figure 5a); Ordinary one-way ANOVA with Tukey post-hoc testing (Figure 4e; Figure 5b, 5b, 5d); Unpaired t test (Figure 3e, 11b, 11c, 11d, 11f, 11g, 11k; Figure 10d, 10e, 10f, 10g); Kolmogrov-Smirnov test (Figure 11l); Brown Forsythe and Welch ANOVA with Dunnett's T3 multiple comparisons test (Figure 5a); Linear regression (Figure 1h, 11n). Differences were considered statistically significant at p < *0.05.* Data is presented as mean ± s.e.m., unless indicated otherwise.

### Example 2: Adjacent non-plaque-associated microglia are constantly attracted to amyloid plaques where they clonally expand and adopt a disease-associated profile

To gain insights into the spatial heterogeneity and dynamics of the microglia during neurodegeneration, we first analyzed the overall distribution of Pu.1⁺ microglia in the frontal cortices of 44 weeks old *5xFAD⁺* animals **(****Figure 1a****).** Compared to homogenously distributed microglia in the non-transgenic *5xFAD⁻* mice, microglia in transgenic *5xFAD⁺* animals showed an aberration in their network structure with a bimodal distribution. In fact, Pu.1⁺Iba-1⁺ microglia existed in the cortex as either plaque-associated microglia (PAM), with their processes in direct physical contact to Methoxy-X04-labeled amyloid deposits and their cell bodies typically within a 30 µm radius of said deposits, or as non-plaque-associated microglia (non-PAM), ramified cells distributed at greater distance from- and with no contact to-deposited extracellular amyloid. Quantification of Pu.1⁺ PAM and non-PAM showed a clear expansion of Pu.1⁺ cells in *5xFAD⁺* animals in the PAM compartment but retained similar numbers of ramified non-PAM as seen in controls **(****Figure 1b****).** Assessment of proliferation capacity in PAM and non-PAM revealed that 5-bromo-2'-dexoyuridine (BrdU) incorporation was detectable in PAM, and non-PAM in *5xFAD⁺* mice, indicating high proliferative capacity in both compartments whereas non-PAM in *5xFAD⁻* mice incorporated BrdU to a lesser extent **(****Figure 1c****).**

Microglia dramatically expand by clonal proliferation during development ³⁶, but maintain their adult network by random low-level proliferation ^{7, 9}. To study cell dynamics in the PAM and non-PAM compartment over time and to distinguish spatially distributed microglia and their progeny, we generated *Cx3cr1^{CreERT2}R26^{Confetti}5xFAD⁺* mice that randomly label individual microglia upon application of Tamoxifen (TAM). TAM induction was titrated to a low labeling efficiency to follow individual labeled microglia in one of the four possible fluorescent reporter proteins: nuclear green fluorescent protein (nGFP), cytoplasmic yellow fluorescent protein (YFP), cytoplasmatic red fluorescent protein (RFP), and membrane-tagged cyan fluorescent protein (mCFP) **(****Figures 1d, e****).** Remarkably, single Confetti⁺Pu.1⁺ microglial cells were sparsely distributed in 20 weeks old *5xFAD*mice (14 weeks after TAM induction) (data not shown), whereas in *5xFAD⁺* mice, homogenously labelled clusters of Confetti⁺Pu.1⁺ PAM were found in close vicinity to Methoxy-X04 amyloid deposits **(****Figures 1e, f****).** To establish the expansion dynamics of neighboring microglial cells within the PAM and non-PAM population, the proximities of each Confetti⁺ microglia to same-colored cells in the three-dimensional volume were determined and compared to randomly generated distances based on 10.000 Monte Carlo (MC)-simulated outputs of each recorded data set as described previously by us before⁹. This procedure allowed us to define random, non-clonal expansion of non-PAM, whereas PAM clonally expand at the plaque site in *Cx3cr1^{CreERT2}R26^{Confetti}5xFAD⁺* mice **(****Figure 1g****).** We next aimed to test for clonal accumulation of Confetti⁺Pu.1⁺PAM and a potential correlation of clone sizes with amyloid plaque dimensions. We therefore generated Voronoi grids of amyloid plaques and adjacent Confetti⁺Pu.1⁺PAM clones in order to visualize this spatial relationship **(****Figure 1g****).** Clone dimensions of PAM were overall highly correlative to the amyloid plaque size **(****Figure 1h****).** However, amyloid plaques of > 1000 µm³ no longer showed a correlation to the Confetti⁺ Pu.1⁺ PAM clone size, indicating that progressive amyloid deposition outcompetes clonal growth of PAM.

To determine additional reliable markers to define non-PAM and PAM beyond their distinct location, we tested various markers of previously reported DAM ²⁴ and MgnD ³¹ such as increased *CD11c, ApoE, receptor tyrosine kinase Axl, Clec7a* accompanied by a loss of homeostatic microglial core molecules such as *transmembrane protein 119 (Tmem119)* and purinergic receptor *P2η12.* Indeed, we found that even though Clec7a, ApoE and Axl were highly expressed in Confetti⁺ PAM, these markers were also partially found across the non-PAM population, whereas P2ry12 was still found on Confetti⁺ PAM clones **(****Figure 2a****,** **Figure 3a****).** In contrast, CD11c expression was highly restricted to PAM and virtually absent in the non-PAM compartment **(****Figure 3b****),** representing a reliable marker for PAM. Similarly, the homeostatic marker Tmem119 revealed a clear labeling of non-PAM, whereas PAM only showed a minor expression of Tmem119 **(****Figures 3c****-e).**

To interrogate the dynamic relationship between the non-PAM and PAM compartments, we took advantage of the selective Tmem119 expression on non-PAM and generated *Tmem119^{CreERT2}R26^{Confetti}5xFAD⁺* animals **(****Figure 3f****).** We induced recombination in 36 weeks old *Tmem119^{CreERT2}R26^{Confetti}5xFAD⁺* animals. After 8 weeks, we detected that Confetti⁺ microglia were labeled in both Tmem119⁺Iba-1⁺non-PAM and Tmem119-Iba-1⁺PAM, suggesting that initially labeled non-PAM gave rise to new PAM adjacent to the amyloid plaque sites **(****Figure 3g****).** Here, Confetti⁺ PAM clones of all four fluorescent proteins were found adjacent to amyloid deposits **(****Figure 3h****).** As expected, single labeled ramified Confetti⁺Iba-1⁺ non-PAM were found distant to amyloid plaque pathology and in unaffected brain regions such as the cerebellum **(****Figure 3i****),** as well as in *5xFAD-* mice (not shown). Clusters of Confetti⁺Iba-1⁺ PAM were observed around cortical extracellular amyloid deposits in *Tmem119^{CreERT2}R26^{Confetti}5xFAD⁺* mice, and the number of clones per cluster increased from two weeks to eight weeks after TAM injection **(****Figure 3j****).** Notably, single Confetti⁺non-PAM were always located in close proximity to Confetti⁺ PAM clones of the same colour **(****Figure 3j****),** and the percentage of PAM clones associated with an adjacent same-coloured non-PAM remained stable from two weeks to eight weeks after TAM injection **(****Figures 3k,** l). These data point to non-PAM as a highly versatile microglial population during neurodegeneration that dynamically respond to progressing amyloid pathology by giving rise to clonally expanding PAM, which show a clear adaptation from homeostatic marker expression towards an activation profile.

### Example 3: Clone sizes of PAM are modulated by gut microbiota and peripheral stimuli in an age-dependent manner

After establishing PAM as the final cell state to which non-PAM transition in neurodegeneration, we examined whether clonal expansion of PAM can be modulated by environmental factors and whether any alterations in clonality would be linked to changed microglial functions and altered amyloid burden. Environmental factors such as gut microbiota and infections, as well as age, have been previously shown to modify microglial behavior during amyloid pathology in mice³⁸⁻⁴⁰.

To investigate the impact of extrinsic signals on clonal expansion of PAM, we treated *Cx3cr1^{CreER}R26R^{Confetti}5xFAD⁺* mice peripherally with low-dose lipopolysaccharide (LPS) to model low-grade peripheral inflammation, or alternatively with antibiotics (ABX) to mimic loss of host microbiota at early or late stage of disease **(****Figure 4a****).**

Clonal expansion of PAM resulting in increased microglial accumulation at the plaque site occurred with different dynamics in young **(****Figure 4b, c****)** and aged **(****Figure 4d, e****)**
*Cx3cr1^{CreER}R26R^{Confetti}5xFAD⁺* animals. 14 weeks after TAM induction, PAM clone expansion was more prominent in young than aged *5xFAD⁺* mice, pointing to different expansion dynamics at different stages of amyloid deposition. Notably, the increased clonal accumulation of Confetti⁺ PAM in transgenic mice at early disease stages was slightly reduced in the absence of microbiota, whereas LPS application robustly increased the expansion of PAM clones **(****Figure 4b, c****).** In contrast, the clonal clustering and accumulation of Confetti⁺ PAM at the plaque site were not affected by either treatment paradigm in old *Cx3cr1^{CreER}R26R^{Confetti}5xFAD⁺* animals **(****Figure 4d, e****).** Accordingly, when we quantified clone sizes in the different treatment paradigms, we detected that PAM clone sizes, number of plaques associated with the clone as well as clone territory were prominently enhanced upon LPS challenge, but not in the absence of microbiota at early stages of disease **(****Figures 4f****- i).** During the late stages of the disease, no alteration of clone size was observed across the different treatment groups **(****Figure 4f****-i).**

We next examined whether modulation of PAM clone sizes and expansion were accompanied by modifications of amyloid pathology. While only subtle changes in the median plaque size were detectable upon any treatment in the early and late-stage groups, LPS treatment, but not ABX application, significantly increased the number of plaques in early-stage mice **(****Figure 5a****).** However, ABX- and LPS-treated groups at the early stage showed a slight decrease in levels of soluble and insoluble Aβ₁₋₄₂ **(****Figure 5b****).** Intriguingly, ABX treatment enhanced phagocytosis by PAM during the early stages of the disease, yet no changes were detectable in aged animals **(****Figure 5c, d****).** Collectively, these data suggest that the kinetics of PAM clonality is dependent on the duration of the disease course.

### Example 4: Only non-PAM exhibit a high transcriptional responsiveness during neurodegeneration

To elucidate which molecular events orchestrate and restrict clonal expansion of PAM from adjacent non-PAM, we examined the transcriptional changes in both compartments and their modulation by environmental factors during the disease course. We profiled both PAM and non-PAM from all treatment groups at an early stage by single-cell RNA sequencing (scRNA-seq) **(****Figure 6a****).** For single-cell sorting, non-PAM was defined by CD45^{lo}CD11b⁺CD11c- and PAM by CD45^{lo}CD11b⁺CD11c⁺ expression. Separation of non-PAM and PAM by CD11c expression was further confirmed by distinct expression levels of Clec7a, which was increased in amyloid-adjacent PAM, as previously described ²⁴. Subsequent analysis showed that transcriptional profiles of individual cells segregated on a Uniform Manifold Approximation and Projection (UMAP) plot according to the sorting paradigm into CD11c⁺ PAM and CD11c- nonPAM **(****Figure 7a****).** Furthermore, index-sorted PAM showed enhanced fluorescence intensity for methoxy-X04 compared to non-PAM **(****Figure 6b****).** Unsupervised clustering subdivided all annotated microglia to five major clusters, designated C0-C4 **(****Figure 7b****).** We further found that C0 and C1 were enriched in PAM, whereas C2, C3, and C4 mainly comprised non-PAM **(****Figure 7b****).** Next, we evaluated transcriptional changes induced by LPS or ABX treatment in both compartments. We observed prominent transcriptional changes in non-PAM enriched clusters between microglia from untreated and LPS- and ABX-treated mice, respectively **(****Figure 7c****).** Notably, at early disease stages, non-PAM exhibited a clear enrichment of cells in distinct clusters after ABX (C3) and LPS (C4) treatment, which were transcriptionally distinct from the untreated non-PAM cluster C2 (Figure 7c,d). In contrast, no treatment-associated microglial clusters were found in the PAM compartment since all profiled microglia were dispersed across PAM clusters (C0 and C1) without clustering according to treatment. To elucidate potential effects of the treatment paradigms on the transcriptional states of non-PAM, we compared gene expression changes between the identified clusters. Differentially expressed (DE) genes between the clusters pointed to the enrichment of genes such as *Cst7, Apoe, Axl, Igf1,* or *Ctsd* in C0 as well as C1, mainly consisting of PAM, compared to the other clusters C2-C4, which consist mainly of non-PAM **(****Figure 7d****,** **e****).**

Next, we used gene set enrichment analysis (GSEA) to decipher alterations induced in the non-PAM cell states upon ABX or LPS treatment. Our analysis revealed a profound induction of gene sets and pathways associated with inflammation, including interferon (IFN)-γ signaling and nuclear factor 'kappa-light-chain-enhancer' of activated B cells (NFκB) activation via tumor necrosis factor (TNF) signaling, in the non-PAM compartment upon LPS treatment **(****Figure 7f****).** In contrast, ABX treatment induced downregulation of proinflammatory gene sets, including genes associated with the regulation of innate immune response or regulation of macrophage activation **(****Figure 7g****).**

To test whether PAM transcriptional unresponsiveness towards exogenous treatment paradigms is due to alterations in the underlying pathways or alternatively due to cellular exhaustion, we interrogated the respective transcriptional profiles **(****Figures 7h****-j).** Genes related to the MyD88-dependent pathway as well as to senescence were not diminished in PAM compared non-PAM whereas the MyD88-independent signaling pathway was slightly altered. As expected, TREM2-dependent as well as -independent pathways were found to be differentially expressed by PAM and non-PAM **(****Figure 7i****).** Remarkably, when we analyzed adaptation of cellular states after ABX and LPS treatment in late stages of the disease, only minor transcriptional changes induced by LPS and ABX treatment were detectable across all analyzed cells **(****Figure 6f****-n).** Treatment-associated clustering was detectable in neither the PAM nor non-PAM compartment **(****Figure 6i, j****).**

Taken together, using single-cell transcriptomic profiling, we describe distinct age- and context-dependent microglial states in the non-PAM but not in the PAM population. Notably, these findings suggest non-PAM as a more responsive and modular microglia state, which could represent a potentially targetable microglia population during amyloid pathology.

### Example 5: Distinct chromatin accessibility landscapes separate non-PAM from PAM

To define the different transcriptional responsiveness of non-PAM and PAM upon environmental changes on the molecular level, we next performed ATAC-seq (assay for transposase-accessible chromatin using sequencing) to profile chromatin accessibility in microglia from *5xFAD⁻* or PAM and non-PAM from *5xFAD⁺* at early-stage of the disease. After quality control, we excluded one sample (non-PAM rep 2), which showed low signal-to-noise ratio (Transcription Start Site [TSS] enrichment; TSSe) compared to the other samples (Figure 8a,b).

PAM and non-PAM from *5xFAD⁺* and microglia from *5xFAD⁻* mice displayed very similar chromatin accessibility profiles at previously defined primed and active enhancer sites **(****Figure 9a****).** Furthermore, principle component analysis (PCA) demonstrated that non-PAM and microglia from non-transgenic mice clustered together and clearly separate from PAM **(****Figure 8c****).** Next, we identified differentially accessible regions (DAR) and found 4,360 regions with higher (PAM up) and 1,301 regions with lower accessibility (PAM down) in PAM compared to non-PAM **(****Figure 9b****,** **c****).** Chromatin accessibility levels at DARs was comparable between non-PAM and microglia from *5xFAD⁻* mice **(****Figure 9c****).** PAM down regions were associated with genes like *Egr1, Crybb1, Nunj2,* but also homeostatic microglia genes such as *Tmem119* and *Csflr.* On the other hand, PAM up regions were associated with genes involved in cellular stress response such as *Bcr, Psmb6, Pah,* or *Tep1* **(****Figure 9b****).** Moreover, we found binding motifs for the glycolytic transcriptional activator GRC2, the copper-dependent transcription factor MAC1 or the proliferation-arresting transcription factor RUNX3 enriched in peaks with higher accessibility in non-PAM **(****Figure 9d****).** Chromatin accessibility in PAM was increased at distal and proximal DARs assigned to genes with elevated expression in PAM, as well as genes of the previously reported DAM signature such as *Csf1, Apoe, Spp1, Clec7a* and *Itgax* **(****Figure 9e****).** On the other hand, non-PAM and microglia from *5xFAD⁻* mice showed higher chromatin accessibility at proximal and distal DARs associated with classical homeostatic microglia signature genes, such as *Tmem119,* but also *Csflr* **(****Figure 9e****).** Regions with higher accessibility in non-PAM were furthermore associated with genes involved in DNA methylation and chromatin reprogramming **(****Figure 8d****).** Collectively, these observations substantiate the conclusion that PAM adapt distinct chromatin accessibility profiles during pathology at the site of amyloid plaques, non-PAM are more similar to the chromatin accessibility landscape of homeostatic microglia.

### Example 6: Engagement of CSF1R by CSF1 but not IL-34 in the non-PAM compartment modulates clonal expansion and phagocytosis thereby improving amyloid pathology

Given the profound differences in the transcriptome and accessible chromatin landscapes between non-PAM and PAM, we next examined whether these two microglia populations can be targeted distinctly. Since we identified non-PAM as a dynamic and modular microglial population responding to environmental changes during early stages of AD and with distinct epigenetic characteristics compared to PAM, we hypothesize that this spatially distinct microglial state might offer a plausible candidate population to test focused therapeutic interventions. In both the scRNA-seq data (Figure **7e****)** as well as the ATAC-seq analysis **(****Figure 9e****),** we revealed higher transcript levels and an open chromatin region for *Csf1r in* non-PAM compared to PAM, making CSF1R a potential molecular target in this population.

To this end, we peripherally injected *Cx3cr1^{CreERT2}R26^{Confetti}5xFAD⁺* mice and controls with the CSF1R ligands colony stimulating factor 1 (Csf1) or interleukin (IL)-34 at early stages of amyloid pathology **(****Figure 10a****).** After Csf1 treatment, clonal expansion of Confetti⁺Pu.1⁺ PAM was diminished **(****Figures 11a, b****),** but no alterations in the expansion of non-PAM was induced. Overall microglial cell number was reduced after Csf1 treatment in comparison to PBS-injected *Cx3cr1^{CreERT2}R26^{Confetti}5xFAD⁺* animals **(****Figure 11c****).** In contrast, microglial cell number was elevated after Csf1 treatment in *Cx3cr1^{CreERT2}R26^{Confetti}5xFAD⁻* mice **(****Figure 11c****).** Furthermore, fewer amyloid plaques were associated with CD11c⁺ PAM **(****Figure** 1**1d****),** whereas Csf1 treatment enlarged the CD68⁺ lysosomal compartment in PAM but surprisingly not in non-PAM suggesting increased microglial phagocytosis at the site of amyloid pathology **(****Figures 11e, f****).** Analysis of soluble and insoluble Aβ₁₋₄₀ and Aβ₁₋₄₂ levels revealed a strong reduction upon Csf1 treatment **(****Figure 11g****),** whereas APP processing itself was not affected **(****Figure** 11**h****).** To gain further insights into the seeding and distribution of amyloid deposits and their relation to Confetti⁺ Pu.1⁺ PAM clones, we performed a semi-automated analysis with Voronoi gridding after Csf1 treatment compared to PBS-injected animals **(****Figures 11i****, j).** We observed reduced numbers and volumes of amyloid plaques in the frontal cortex after Csf1 application **(****Figures 11k, l****).** Moreover, we found no change of the amount of plaques with contact to multiple Confetti⁺ clones **(****Figure 11m****).** Csf1 not only reduced amyloid plaque volumes and PAM clone sizes, it also induced a higher correlation between these parameters **(****Figure 11n****).** Large amyloid deposits (>1000 µm³), which outcompeted PAM clones before, became now highly correlative with PAM clone size, indicating that Csf1 treatment modulated the expansion of PAM which restricted amyloid growth (Figure 11m). In contrast to Csf1, the other Csf1r ligand IL-34 did not influence clonal expansion of Confetti-labeled PAM or non-PAM compared to PBS-treated controls **(****Figure 10c****-d).** Similarly, IL-34 treatment did not affect the microglial cell number nor the decoration of amyloid plaques with PAM **(****Figure 10e,** f). Further, plaque numbers and volumes were also not altered upon IL-34 treatment **(****Figure 10g****).**

In sum, Csf1 treatment, but not IL-34 application, enhances phagocytic capacity of PAM and ameliorates PAM expansion at early stages of disease, thereby facilitating restriction of amyloid pathology. Our data further suggest that these effects subsequently modulate the pathology-driven transition of non-PAM to PAM by engagement with CSF1R, resulting in diminished disease progression.

### Example 7: CSF1 shapes the inflammatory and metabolic gene expression profiles of both PAM and non-PAM rather than blocking cell shifts

To test whether Csf1 treatment modulates the differentiation of non-PAM to PAM, we first performed scRNA-seq of both microglia populations after four weeks of Csf1 or PBS treatment in *Cx3cr1^{CreERT2}R26^{Confetti}5xFAD⁺* mice and controls at early stage of the disease. This early time point was chosen to obtain a sufficient number of PAM, in case longer Csf1 treatment would completely block the development of PAM around plaques. Transcriptomic profiling showed that non-PAM and PAM clearly segregated on a UMAP plot according to their differential gene expression **(****Figure 7a****).** Eight distinct microglial clusters, designated C0-C7, were identified when performing unsupervised clustering, with each of them showing distinct transcriptional profiles **(****Figure 12b****-d).** We next tested the distribution of analyzed PAM and non-PAM in the different treatment groups across the clusters **(****Figure 12c****,** **e****).** Clusters C1, C3, C6 and C7 were enriched in PAM, whereas C0, C2, C5 and C8 mainly included non-PAM **(****Figure 12c****,** **e****),** as indicated by the expression of disease-associated signature genes in C1, C3, C6 and C7, and the expression of homeostasis marker genes in C0, C2, C5 and C8 **(****Figures 12d****, f, g).** We identified two specific Csf1-induced clusters in the CD11c⁺ PAM population of *Cx3cr1Cre^{ERT2}R26^{Confetti}5xFAD⁺* mice, namely C1 and C7, whereas only one Csf1-induced cluster (C5) within the CD11c- microglia was observed in *Cx3cr1^{CreERT2}R26^{Confetti}5xFAD⁻*controls. However, we did not detect any clusters enriched for CD11c⁻ non-PAM from Csf1-treated *Cx3cr1^{CreERT2}R26^{Confetti}5xFAD⁺* mice. These cells were mainly allocated to C2 together with CD11c-homeostatic microglia from PBS- or Csf1-injected *Cx3cr1 ^{CreERT2}R26^{Confetti}5xFAD-* mice. Non-PAM from PBS-treated *Cx3cr1^{CreERT2}R26^{Confetti}5xFAD⁺* mice were mainly localized in C4 together with a small proportion of non-PAM from Csf1-challenged animals. Importantly, both PAM and non-PAM retained their respective "homeostatic" *(Tmem119, P2ry12, Cx3cr1* etc.) or "activated" *(Trem2, Apoe, Itgax* etc.) gene panel upon Csf1 treatment indicating that there is no therapy-induced shift of non-PAM toward PAM involving their core signatures **(****Figures 12f, g****).**

In order to better understand the beneficial effects of Csf1 on microglial dynamics and disease progression during amyloid pathology, we performed gene set enrichment analysis (GSEA) on pseudobulk gene expression analysis of PAM isolated from Csf1- or PBS- treated *Cx3cr1^{CreERT2}R26^{Confetti}5xFAD⁺* mice **(****Figure 12h****).** Here, we observed that Csf1 treatment induced a profound decrease in gene sets and pathways associated with inflammatory response, including type I or type II interferon signaling or NFκB activation via TNF-α signaling **(****Figure 12h****).** In contrast, Csf1 treatment induced an upregulation of genes associated with metabolic regulation towards autophagy and oxidative phosphorylation **(****Figure 12h****).** Importantly, similar trends were observed in non-PAM after Csf1 treatment **(****Figure 12i****)** indicating that both microglial populations are highly modulated by Csf1, even though Csf1r expression was found to be downregulated in PAM. This suggests cell-autonomous effects of Csf1 on non-PAM, whereas PAM seem to be more affected by cell-non-autonomous effects, most likely due to a modulated differentiation of Csf1 exposed non-PAM to more amyloid-competent PAM.

Taken together, our data indicate that the beneficial effect of Csf1 treatment does not per se change the core signatures of non-PAM and PAM but rather alleviates pathways of inflammation, increases mitochondrial features suggesting an improvement of the functional fitness (e.g. phagocytosis, metabolism) of these microglia and the generation of an amyloid-competent and restrictive PAM population.

### Discussion of the Examples

In this study, a combination of microglia subset-focused multicolor fate mapping mouse models, along with single-cell transcriptomic and epigenomic profiling revealed defined spatial and temporal dynamics between two spatially distinct microglia populations, designated PAM and non-PAM, during amyloid pathology. Here, we further identified non-PAM as more transcriptionally responsive to external stimuli and therapeutic modulations, such as engagement of CSF1R by treatment with Csf1. This treatment paradigm efficiently modulates differentiation of non-PAM to amyloid-restrictive PAM with subsequent amelioration of amyloid pathology.

Our analyses identified two spatially and transcriptionally distinct microglia populations during amyloid pathology: non-PAM, more resembling homeostatic microglia, and PAM, closely associated to amyloid plaques and with a characteristic gene signature of early described as "DAM/MgnD" ^{24,31}. We found dynamic microglial turnover between non-PAM and PAM, with selective clonal expansion occurring exclusively in PAM originating from adjacent individual non-PAM. Non-PAM-specific Confetti-labelling allowed us to determine that the PAM compartment is maintained by continuous input of cells derived from neighboring bystander non-PAM, which clonally expand upon plaque association, producing a PAM clone derived from the original bystander cell. The recruitment and accumulation of microglia to the plaque sites were reported previously in a study using a fate-mapping model with single-colored microglia to examine microglial kinetics at amyloid deposition ⁹. However, this study did not decipher the critical involvement and dynamic transition as well as the function of non-PAM during amyloid pathology progression.

The generation of PAM has been suggested on the one hand to be a driving force of disease progression in AD ^{33,34} and on the other hand to play a beneficial role in controlling amyloid pathology progression ⁴⁰⁻⁴². However, the functional role of non-PAM in this process and its potential contribution to the disease pathology as the origin of PAM clones has been largely neglected. Indeed, microglia associated with extracellular amyloid, possessing a distinct gene expression profile also known as DAM ²⁴ or MgnD ³¹, were so far considered as the only pathophysiologically relevant microglial population in neurodegeneration ³². Therefore, this specific pathology-linked microglia population has been analyzed in more detail in previous studies for its potential to restrict neurodegeneration, which would have important implications for the treatment of AD, though recent studies targeting PAM-specific molecules and signaling pathways, including Trem2 and ApoE, revealed differing results on the disease progression and outcome ^{33, 35, 43}.

Our data, however, shifts this simplified DAM/PAM-centered view. In fact, we found that both, the transcriptomic and the epigenetic signature, clearly indicate that PAM represent a microglial population with a limited capacity to react upon peripheral stimulation. In contrast, non-PAM retained their homeostatic signature even in AD transgenic mice, with high levels of *Tmem119, P2ry12* and *Cx3cr1,* but absence of previously reported disease-associated signature such as *Itgax.* Furthermore, we extensively tested which homeostatic and DAM signature genes showed a dichotomy in protein expression levels, allowing us to perform marker-based discrimination between PAM and non-PAM. Besides CD11c as a marker for PAM, we identified Tmem119 expression to be highly restricted to non-PAM. In contrast, other markers tested showed less specificity for any of the microglia populations, including Clec7a, which was recently reported to be highly specific for disease-associated microglia in demyelination and proliferative microglia during development ⁴⁴. In line with our findings, this shows that microglia can share certain disease-associated microglia signature genes, but that there are subtle differences between microglia states across diseases, which need to be carefully addressed in the future.

Taking the relative longevity of microglia in both mouse and human into consideration ^{7, 9, 45}, our findings of two spatially and functionally distinct microglial cell populations during neurodegeneration is of considerable therapeutic relevance. Subset-specific targeting is highly desirable in a disease setting to ensure that the homeostatic functions of microglia in non-affected brain regions are maintained, as seen for example after depletion of microglia by CSF1R inhibition ^{46, 47}. However, only limited information are currently available on the specific lifetime of PAM and non-PAM during amyloid pathology and the dynamics between these two. The clonal expansion of PAM from individual non-PAM adjacent to the developing amyloid plaque site, and the proliferative transition of non-PAM to PAM described here are supported by recent studies demonstrating that prolonged and enhanced proliferation of microglia due to amyloid deposition results in a replicative senescence in microglia and the development of senescent DAM ^{48, 49}.

Our findings on the effects of peripheral stimuli on clonal expansion of PAM are further in line with studies in patients showing that chronic inflammation in humans is strongly associated with an increased risk of developing AD ⁵⁰. Similarly, previous studies in transgenic AD mouse models revealed that a single dose or short-term application of LPS alone induced an increase in plaque load and a decrease in Aβ uptake ^{37, 51, 52}. However, the effects on both microglia populations, with enhanced clonal expansion of PAM and priming of non-PAM had not been described in previous studies, showing that a single injection of LPS might induce different alterations compared to a chronic administration of LPS, the latter being a better model to study chronic inflammation in humans and its effects on AD.

Previous studies reported reduced amyloid pathology and differential effects on microglia by antibiotic-induced depletion of microbiota ^{38, 53, 54}. Most likely, the differences between our study and other reported findings is due to different transgenic lines, sexes, ages and distinct brain regions analyzed. While we found that depletion of endogenous microbiota mostly affects the non-PAM population and clonal PAM expansion at early stage of disease, our data support the assumption that peripheral stimuli such as LPS or depletion of endogenous microbiota affect microglia dynamics and clonality in an age-dependent manner during pathology.

One important characteristic of the non-PAM compartment is the higher gene expression and accessible gene locus for *Csflr,* which were both clearly reduced in the PAM population. This downregulation of Csflr gene expression was previously reported for DAM and MgnD microglia states ^{24, 31}. CSF1 R is an essential survival factor for microglial cell development, expansion and homeostasis *in vivo* ^{15, 55-57}. The two known ligands for CSF1Rare IL-34 and Csf1, which are derived from different cellular sources in the CNS ⁵⁸⁻⁶¹. IL-34 in the brain is mainly produced by neurons, whereas Csf1 is mostly secreted by astrocytes ⁵⁸⁻⁶⁰. Regulation of both ligands has been reported in the brains of AD patients: whereas *Csf1* gene expression was increased in hippocampal regions of AD patients, *Il34* expression was downregulated ⁶².

The therapeutic potential of modulating CSF1R signalling in brain diseases has been proposed on several levels ⁶³. CSF1R inhibition or depletion was achieved before by genetic modifications or pharmacological inhibition in models of neurodegeneration such as prion disease or AD ⁶⁴⁻⁶⁷. Of note, one recent study suggested that pharmacological CSF1R inhibition can be used to potentially modulate development of PAMs in AD and improve extracellular amyloid deposition by blocking senescent proliferation ⁴⁸. Of note, depleting effects on other macrophage populations and microglia in unaffected brain regions after CSF1R inhibition can hamper its potential therapeutic use ⁴⁷. Furthermore, genetic mutations reducing CSF1R signalling capacity are associated with development of hereditary diffuse leukoencephalopathy with spheroids (HDLS) ⁶⁸⁻⁷⁰. In contrast, we described here beneficial effects of peripheral Csf1 application to induce CSF1R signalling in non-PAM in an animal model for AD. Earlier studies suggested disease improvement in other neurodegenerative and neuroinflammatory diseases by application of IL-34 or Csf1 ⁷¹⁻⁷⁵. A recent study highlighted the protective effects on IL-34 application during autoimmune neuroinflammation in aged mice by expanding autophagy-dependent neuroprotective microglia ⁷¹. However, the specific modulation of microglia and their dynamics during amyloid pathology were not addressed. Our data supports the hypothesis that CSF1R engagement by Csf1, but not IL-34, critically modulates microglial subsets. Interestingly, we did not find evidence that the development of PAM was blocked but rather that Csf1 shapes the functionality of the non-PAM-PAM axis by reducing clonal expansion around the amyloid plaque and enhancing phagocytic activity. These results are in line with a recent report that expansion of microglia in neurodegeneration after LPS treatment is driven by CSF1R signalling ⁵². Our data point to a beneficial effect of Csf1 on controlling non-PAM to PAM differentiation and modulating PAM effector functions towards an amyloid-competent phenotype. Single-cell transcriptomic profiling further revealed an anti-inflammatory effect within both populations by Csf1 treatment and an enhancement of oxidative phosphorylation and reduced glycolysis, pointing to a metabolic switch in microglia, which was recently reported to be beneficial during amyloid pathology ⁷⁶.

In summary, our data point to the non-PAM population as a so far neglected key modulator of amyloid pathology and disease-associated microglial dynamics. We revealed that non-PAM represent an amyloid-responsive microglia population that continuously integrate and react to environmental cues with implications for disease progression. Individual non-PAM respond immediately to amyloid pathology by clonal expansion and differentiation to PAM at the plaque site. This transition can be modulated during early stages of amyloid pathology, providing a potential window of microglia-subset specific therapeutic intervention by adapting the transition from non-PAM to PAM.

### REFERENCES

1. Prinz, M., Jung, S. & Priller, J. Microglia Biology: One Century of Evolving Concepts. Cell 179, 292-311 (2019).
2. Kierdorf, K. & Prinz, M. Microglia in steady state. J Clin Invest 127, 3201-3209 (2017).
3. Colonna, M. & Butovsky, O. Microglia Function in the Central Nervous System During Health and Neurodegeneration. Annu Rev Immunol 35, 441-468 (2017).
4. Prinz, M., Masuda, T., Wheeler, M.A. & Quintana, F.J. Microglia and Central Nervous System-Associated Macrophages-From Origin to Disease Modulation. Annu Rev Immunol 39, 251-277 (2021).
5. Norris, G.T. & Kipnis, J. Immune cells and CNS physiology: Microglia and beyond. J Exp Med 216, 60-70 (2019).
6. Salter, M.W. & Stevens, B. Microglia emerge as central players in brain disease. Nat Med 23, 1018-1027 (2017).
7. Tay, T.L., et al. A new fate mapping system reveals context-dependent random or clonal expansion of microglia. Nat Neurosci 20, 793-803 (2017).
8. Askew, K., et al. Coupled Proliferation and Apoptosis Maintain the Rapid Turnover of Microglia in the Adult Brain. Cell Rep 18, 391-405 (2017).
9. Fuger, P., et al. Microglia turnover with aging and in an Alzheimer's model via long-term in vivo single-cell imaging. Nat Neurosci 20, 1371-1376 (2017).
10. Ajami, B., Bennett, J.L., Krieger, C., Tetzlaff, W. & Rossi, F.M. Local self-renewal can sustain CNS microglia maintenance and function throughout adult life. Nat Neurosci 10, 1538-1543 (2007).
11. Goldmann, T., et al. A new type of microglia gene targeting shows TAK1 to be pivotal in CNS autoimmune inflammation. Nat Neurosci 16, 1618-1626 (2013).
12. Mildner, A., et al. Microglia in the adult brain arise from Ly-6ChiCCR2+ monocytes only under defined host conditions. Nat Neurosci 10, 1544-1553 (2007).
13. Kierdorf, K., et al. Microglia emerge from erythromyeloid precursors via Pu.1- and Irf8-dependent pathways. Nat Neurosci 16, 273-280 (2013).
14. Schulz, C., et al. A lineage of myeloid cells independent of Myb and hematopoietic stem cells. Science 336, 86-90 (2012).
15. Hagemeyer, N., et al. Transcriptome-based profiling of yolk sac-derived macrophages reveals a role for Irf8 in macrophage maturation. EMBO J 35, 1730-1744 (2016).
16. Masuda, T., et al. Specification of CNS macrophage subsets occurs postnatally in defined niches. Nature 604, 740-748 (2022).
17. Grabert, K., et al. Microglial brain region-dependent diversity and selective regional sensitivities to aging. Nat Neurosci 19, 504-516 (2016).
18. Hammond, T.R., et al. Single-Cell RNA Sequencing of Microglia throughout the Mouse Lifespan and in the Injured Brain Reveals Complex Cell-State Changes. Immunity 50, 253-271 e256 (2019).
19. Kracht, L., et al. Human fetal microglia acquire homeostatic immune-sensing properties early in development. Science 369, 530-537 (2020).
20. Li, Q., et al. Developmental Heterogeneity of Microglia and Brain Myeloid Cells Revealed by Deep Single-Cell RNA Sequencing. Neuron 101, 207-223 e210 (2019).
21. Masuda, T., et al. Spatial and temporal heterogeneity of mouse and human microglia at single-cell resolution. Nature 566, 388-392 (2019).
22. Masuda, T., Sankowski, R., Staszewski, O. & Prinz, M. Microglia Heterogeneity in the Single-Cell Era. Cell Rep 30, 1271-1281 (2020).
23. Tejera, D. & Heneka, M.T. Microglia in Neurodegenerative Disorders. Methods Mol Biol 2034, 57-67 (2019).
24. Keren-Shaul, H., et al. A Unique Microglia Type Associated with Restricting Development of Alzheimer's Disease. Cell 169, 1276-1290 e1217 (2017).
25. Mathys, H., et al. Temporal Tracking of Microglia Activation in Neurodegeneration at Single-Cell Resolution. Cell Rep 21, 366-380 (2017).
26. Ellwanger, D.C., et al. Prior activation state shapes the microglia response to antihuman TREM2 in a mouse model of Alzheimer's disease. Proc Natl Acad Sci U S A 118 (2021).
27. Gerrits, E., et al. Distinct amyloid-beta and tau-associated microglia profiles in Alzheimer's disease. Acta Neuropathol 141, 681-696 (2021).
28. Mathys, H., et al. Single-cell transcriptomic analysis of Alzheimer's disease. Nature 570, 332-337 (2019).
29. Olah, M., et al. Single cell RNA sequencing of human microglia uncovers a subset associated with Alzheimer's disease. Nat Commun 11, 6129 (2020).
30. Prater, K.E., et al. Human microglia show unique transcriptional changes in Alzheimer's disease. Nat Aging 3, 894-907 (2023).
31. Krasemann, S., et al. The TREM2-APOE Pathway Drives the Transcriptional Phenotype of Dysfunctional Microglia in Neurodegenerative Diseases. Immunity 47, 566-581 e569 (2017).
32. Deczkowska, A., et al. Disease-Associated Microglia: A Universal Immune Sensor of Neurodegeneration. Cell 173, 1073-1081 (2018).
33. Wang, Y., et al. TREM2-mediated early microglial response limits diffusion and toxicity of amyloid plaques. J Exp Med 213, 667-675 (2016).
34. Ennerfelt, H., et al. SYK coordinates neuroprotective microglial responses in neurodegenerative disease. Cell 185, 4135-4152 e4122 (2022).
35. Wood, J.I., et al. Plaque contact and unimpaired Trem2 is required for the microglial response to amyloid pathology. Cell Rep 41, 111686 (2022).
36. Barry-Carroll, L., et al. Microglia colonize the developing brain by clonal expansion of highly proliferative progenitors, following allometric scaling. Cell Rep 42, 112425 (2023).
37. Wendeln, A.C., et al. Innate immune memory in the brain shapes neurological disease hallmarks. Nature 556, 332-338 (2018).
38. Mezo, C., et al. Different effects of constitutive and induced microbiota modulation on microglia in a mouse model of Alzheimer's disease. Acta Neuropathol Commun 8, 119 (2020).
39. Harach, T., et al. Reduction of Abeta amyloid pathology in APPPS1 transgenic mice in the absence of gut microbiota. Sci Rep 7, 41802 (2017).
40. Zhao, R., Hu, W., Tsai, J., Li, W. & Gan, W.B. Microglia limit the expansion of beta-amyloid plaques in a mouse model of Alzheimer's disease. Mol Neurodegener 12, 47 (2017).
41. Condello, C., Yuan, P., Schain, A. & Grutzendler, J. Microglia constitute a barrier that prevents neurotoxic protofibrillar Abeta42 hotspots around plaques. Nat Commun 6, 6176 (2015).
42. Hu, J., et al. Microglial Piezo1 senses Abeta fibril stiffness to restrict Alzheimer's disease. Neuron 111, 15-29 e18 (2023).
43. Meilandt, W.J., et al. Trem2 Deletion Reduces Late-Stage Amyloid Plaque Accumulation, Elevates the Abeta42:Abeta40 Ratio, and Exacerbates Axonal Dystrophy and Dendritic Spine Loss in the PS2APP Alzheimer's Mouse Model. J Neurosci 40, 1956-1974 (2020).
44. Barclay, K.M., et al. An inducible genetic tool to track and manipulate specific microglial states reveals their plasticity and roles in remyelination. Immunity 57, 1394-1412 e1398 (2024).
45. Reu, P., et al. The Lifespan and Turnover of Microglia in the Human Brain. Cell Rep 20, 779-784 (2017).
46. Priller, J. & Prinz, M. Targeting microglia in brain disorders. Science 365, 32-33 (2019).
47. Martin-Estebane, M. & Gomez-Nicola, D. Targeting Microglial Population Dynamics in Alzheimer's Disease: Are We Ready for a Potential Impact on Immune Function? Front Cell Neurosci 14, 149 (2020).
48. Hu, Y., et al. Replicative senescence dictates the emergence of disease-associated microglia and contributes to Abeta pathology. Cell Rep 35, 109228 (2021).
49. Rachmian, N., et al. Identification of senescent, TREM2-expressing microglia in aging and Alzheimer's disease model mouse brain. Nat Neurosci 27, 1116-1124 (2024).
50. Heneka, M.T., Golenbock, D.T. & Latz, E. Innate immunity in Alzheimer's disease. Nat Immunol 16, 229-236 (2015).
51. Tejera, D., et al. Systemic inflammation impairs microglial Abeta clearance through NLRP3 inflammasome. EMBO J 38, e101064 (2019).
52. Guerrero-Carrasco, M., Targett, I., Olmos-Alonso, A., Vargas-Caballero, M. & Gomez-Nicola, D. Low-grade systemic inflammation stimulates microglial turnover and accelerates the onset of Alzheimer's-like pathology. Glia 72, 1340-1355 (2024).
53. Dodiya, H.B., et al. Sex-specific effects of microbiome perturbations on cerebral Abeta amyloidosis and microglia phenotypes. J Exp Med 216, 1542-1560 (2019).
54. Dodiya, H.B., et al. Gut microbiota-driven brain Abeta amyloidosis in mice requires microglia. J Exp Med 219 (2022).
55. Elmore, M.R., et al. Colony-stimulating factor 1 receptor signaling is necessary for microglia viability, unmasking a microglia progenitor cell in the adult brain. Neuron 82, 380-397 (2014).
56. Erblich, B., Zhu, L., Etgen, A.M., Dobrenis, K. & Pollard, J.W. Absence of colony stimulation factor-1 receptor results in loss of microglia, disrupted brain development and olfactory deficits. PLoS One 6, e26317 (2011).
57. Hagemeyer, N., et al. Microglia contribute to normal myelinogenesis and to oligodendrocyte progenitor maintenance during adulthood. Acta Neuropathol 134, 441-458 (2017).
58. Kana, V., et al. CSF-1 controls cerebellar microglia and is required for motor function and social interaction. J Exp Med 216, 2265-2281 (2019).
59. Wang, Y., et al. IL-34 is a tissue-restricted ligand of CSF1R required for the development of Langerhans cells and microglia. Nat Immunol 13, 753-760 (2012).
60. Greter, M., et al. Stroma-derived interleukin-34 controls the development and maintenance of langerhans cells and the maintenance of microglia. Immunity 37, 1050-1060 (2012).
61. Easley-Neal, C., Foreman, O., Sharma, N., Zarrin, A.A. & Weimer, R.M. CSF1R Ligands IL-34 and CSF1 Are Differentially Required for Microglia Development and Maintenance in White and Gray Matter Brain Regions. Front Immunol 10, 2199 (2019).
62. Walker, D.G., Tang, T.M. & Lue, L.F. Studies on Colony Stimulating Factor Receptor-1 and Ligands Colony Stimulating Factor-1 and Interleukin-34 in Alzheimer's Disease Brains and Human Microglia. Front Aging Neurosci 9, 244 (2017).
63. Pons, V. & Rivest, S. New Therapeutic Avenues of mCSF for Brain Diseases and Injuries. Front Cell Neurosci 12, 499 (2018).
64. Mancuso, R., et al. CSF1R inhibitor JNJ-40346527 attenuates microglial proliferation and neurodegeneration in P301S mice. Brain 142, 3243-3264 (2019).
65. Olmos-Alonso, A., et al. Pharmacological targeting of CSF1R inhibits microglial proliferation and prevents the progression of Alzheimer's-like pathology. Brain 139, 891-907 (2016).
66. Pons, V., Levesque, P., Plante, M.M. & Rivest, S. Conditional genetic deletion of CSF1 receptor in microglia ameliorates the physiopathology of Alzheimer's disease. Alzheimers Res Ther 13, 8 (2021**).**
67. Spangenberg, E.E., et al. Eliminating microglia in Alzheimer's mice prevents neuronal loss without modulating amyloid-beta pathology. Brain 139, 1265-1281 (2016).
68. Rademakers, R., Neumann, M. & Mackenzie, I.R. Advances in understanding the molecular basis of frontotemporal dementia. Nat Rev Neurol 8, 423-434 (2012).
69. Oosterhof, N., et al. Homozygous Mutations in CSF1R Cause a Pediatric-Onset Leukoencephalopathy and Can Result in Congenital Absence of Microglia. Am J Hum Genet 104, 936-947 (2019).
70. Kempthorne, L., et al. Loss of homeostatic microglial phenotype in CSF1R-related Leukoencephalopathy. Acta Neuropathol Commun 8, 72 (2020).
71. Berglund, R., et al. The aging mouse CNS is protected by an autophagy-dependent microglia population promoted by IL-34. Nat Commun 15, 383 (2024).
72. Boissonneault, V., et al. Powerful beneficial effects of macrophage colony-stimulating factor on beta-amyloid deposition and cognitive impairment in Alzheimer's disease. Brain 132, 1078-1092 (2009).
73. Laflamme, N., et al. mCSF-lnduced Microglial Activation Prevents Myelin Loss and Promotes Its Repair in a Mouse Model of Multiple Sclerosis. Front Cell Neurosci 12, 178 (2018).
74. Mizuno, T., et al. Interleukin-34 selectively enhances the neuroprotective effects of microglia to attenuate oligomeric amyloid-beta neurotoxicity. Am J Pathol 179, 2016-2027 (2011).
75. Obst, J., et al. Inhibition of IL-34 Unveils Tissue-Selectivity and Is Sufficient to Reduce Microglial Proliferation in a Model of Chronic Neurodegeneration. Front Immunol 11, 579000 (2020).
76. Baik, S.H., et al. A Breakdown in Metabolic Reprogramming Causes Microglia Dysfunction in Alzheimer's Disease. Cell Metab 30, 493-507 e496 (2019).
77. Oakley, H., et al. Intraneuronal beta-amyloid aggregates, neurodegeneration, and neuron loss in transgenic mice with five familial Alzheimer's disease mutations: potential factors in amyloid plaque formation. J Neurosci 26, 10129-10140 (2006).
78. Yona, S., et al. Fate mapping reveals origins and dynamics of monocytes and tissue macrophages under homeostasis. Immunity 38, 79-91 (2013).
79. Snippert, H.J., et al. Intestinal crypt homeostasis results from neutral competition between symmetrically dividing Lgr5 stem cells. Cell 143, 134-144 (2010).
80. Schwabenland, M., et al. Analyzing microglial phenotypes across neuropathologies: a practical guide. Acta Neuropathol 142, 923-936 (2021).
81. Herman, J.S., Sagar & Grun, D. FatelD infers cell fate bias in multipotent progenitors from single-cell RNA-seq data. Nat Methods 15, 379-386 (2018).
82. Hashimshony, T., et al. CEL-Seq2: sensitive highly-multiplexed single-cell RNA-Seq. Genome Biol 17, 77 (2016).
83. Jordao, M.J.C., et al. Single-cell profiling identifies myeloid cell subsets with distinct fates during neuroinflammation. Science 363 (2019).
84. Subramanian, A., et al. Gene set enrichment analysis: a knowledge-based approach for interpreting genome-wide expression profiles. Proc Natl Acad Sci U S A 102, 15545-15550 (2005).
85. Mootha, V.K., et al. PGC-1alpha-responsive genes involved in oxidative phosphorylation are coordinately downregulated in human diabetes. Nat Genet 34, 267-273 (2003).
86. Finak, G., et al. MAST: a flexible statistical framework for assessing transcriptional changes and characterizing heterogeneity in single-cell RNA sequencing data. Genome Biol 16, 278 (2015).
87. Afgan, E., et al. The Galaxy platform for accessible, reproducible and collaborative biomedical analyses: 2018 update. Nucleic Acids Res 46, W537-W544 (2018).
88. Langmead, B. & Salzberg, S.L. Fast gapped-read alignment with Bowtie 2. Nat Methods 9, 357-359 (2012).
89. Barnett, D.W., Garrison, E.K., Quinlan, A.R., Stromberg, M.P. & Marth, G.T. BamTools: a C++ API and toolkit for analyzing and managing BAM files. Bioinformatics 27, 1691-1692 (2011).
90. Amemiya, H.M., Kundaje, A. & Boyle, A.P. The ENCODE Blacklist: Identification of Problematic Regions of the Genome. Sci Rep 9, 9354 (2019).
91. Quinlan, A.R. & Hall, I.M. BEDTools: a flexible suite of utilities for comparing genomic features. Bioinformatics 26, 841-842 (2010).
92. Zhang, Y., et al. Model-based analysis of ChlP-Seq (MACS). Genome Biol 9, R137 (2008).
93. Love, M.I., Huber, W. & Anders, S. Moderated estimation of fold change and dispersion for RNA-seq data with DESeq2. Genome Biol 15, 550 (2014).
94. Ramirez, F., et al. deepTools2: a next generation web server for deep-sequencing data analysis. Nucleic Acids Res 44, W160-165 (2016).
95. Chudnovskiy, A., et al. Host-Protozoan Interactions Protect from Mucosal Infections through Activation of the Inflammasome. Cell 167, 444-456 e414 (2016).
96. Gosselin, D., et al. Environment drives selection and function of enhancers controlling tissue-specific macrophage identities. Cell 159, 1327-1340 (2014).
97. Heinz, S., et al. Simple combinations of lineage-determining transcription factors prime cis-regulatory elements required for macrophage and B cell identities. Mol Cell 38, 576-589 (2010).
98. McLean, C.Y., et al. GREAT improves functional interpretation of cis-regulatory regions. Nat Biotechnol 28, 495-501 (2010).

## Claims

1. A colony stimulating factor 1 receptor (CSF1R) ligand for use in the treatment or prevention of a neurodegenerative disease in a subject.

2. The CSF1R ligand for use according to the preceding claim, wherein the CSF1R ligand is recombinant CSF1.

3. The CSF1R ligand for use according to claim 1, wherein the CSF1R ligand is a small molecule or antibody activating CSF1R.

4. The CSF1R ligand for use according to any one of the preceding claims, wherein the CSF1R ligand is able to cross the blood-brain barrier (BBB).

5. The CSF1R ligand for use according to any one of the preceding claims, wherein the CSF1R ligand modulates the differentiation of non-plaque associated microglia (nonPAM) to plaque-associated microglia (PAM) or directly the function of PAM or non PAM, preferably within the CNS.

6. The CSF1R ligand for use according to any one of the preceding claims, wherein the differentiation of nonPAM to PAM comprises the clonal expansion of PAM.

7. The CSF1R ligand for use according to any one of the preceding claims, wherein PAM are accumulating microglia at the extracellular amyloid deposits and express one or more of the genes CLEC7A, AXL, TREM2, APOE, and/or ITAGX.

8. The CSF1R ligand for use according to any one of the preceding claims, wherein the CSF1R ligand enhances phagocytic capacity, preferably for amyloid deposits, metabolic programming, migratory capacity, cell survival, inhibition so cellular senescent-associated secretory programs of PAM, induces the release of neurotrophic factors, and/or ameliorates PAM expansion.

9. The CSF1R ligand for use according to any one of the preceding claims, wherein the subject is experiencing or suffering from one or more of (neuro-) cognitive impairment, impaired motoric skills, loss of smell, difficulties with language, clinical symptoms of dementia, attention deficits, and/or impaired memory.

10. The CSF1R ligand for use according to any one of the preceding claims, wherein the subject is suspected or diagnosed to exhibit abnormal deposits of amyloid beta (Aβ) within their brain and/or central nervous system (CNS), preferably comprising amyloid plaques, tau proteins, and/or neurofibrillary tangles.

11. The CSF1R ligand for use according to any one of the preceding claims, wherein the neurodegenerative disease is Alzheimer's disease (AD), Lewy body dementia, Parkinson's disease (PD), multiple sclerosis (MS), motor neuron disease (MND), Huntington disease (HD), progressive supranuclear palsy (PSP), corticobasal degeneration (CBD), multiple system atrophy (MSA), amyotrophic lateral sclerosis (ALS), schizophrenia, depression, and/or an autism-spectrum disorder, preferably Alzheimer's disease (AD), multiple sclerosis (MS), and autism-spectrum disorders.

12. The CSF1R ligand for use according to any one of the preceding claims, wherein the treatment reduces inflammation, such as type I or type II interferon-mediated inflammation and/or NFkB activation within the central nervous system (CNS) of the subject.

13. A pharmaceutical composition comprising the CSF1R ligand according to any one of the preceding claims for use in the treatment or prevention of a neurodegenerative disease, additionally comprising a pharmaceutically acceptable carrier.

14. The pharmaceutical composition for use according to the preceding claim, wherein the composition is administered orally, intracerebrally, intrathecally, intravenously, or subcutaneously.

15. A colony stimulating factor 1 receptor (CSF1R) ligand for use in the modulation of the differentiation of non-plaque associated microglia (nonPAM) to plaque-associated microglia (PAM).
